# EUROPÄISCHE PATENTSCHRIFT

(11) **EP 4 442 792 B1**
(45) Veröffentlichungstag und Bekanntmachung des Hinweises auf die Patenterteilung: **30.09.2026**
(21) Anmeldenummer: 24183459.7
(22) Anmeldetag: 06.06.2014
(51) Int. Cl.: C10L 1/222, C10L 10/04, C10L 1/188

(54) **VERWENDUNG MIT ALKYLENOXID UND HYDROCARBYL-SUBSTITUIERTER POLYCARBONSÄURE QUATERNISIERTER STICKSTOFFVERBINDUNGEN ALS ADDITIVE IN KRAFT- UND SCHMIERSTOFFEN**
USE WITH ALKYLENE OXIDE AND HYDROCARBYL-SUBSTITUTED POLYCARBOXYLIC ACID OF QUATERNISED NITROGEN COMPOUNDS AS ADDITIVES IN FUELS AND LUBRICANTS
UTILISATION AVEC DE L'OXYDE D'ALKYLÈNE ET DE L'ACIDE POLYCARBOXYLIQUE SUBSTITUÉ PAR UN HYDROCARBYLE DE COMPOSÉS AZOTÉS QUATERNISÉS COMME ADDITIFS DANS LES CARBURANTS ET LUBRIFIANTS

(30) Priorität: 07.06.2013 EP 13171057; 16.01.2014 EP 14151379
(43) Veröffentlichungstag der Anmeldung: 09.10.2024
(62) Dokumentnummer(n) der früheren Anmeldung(en) nach Art. 76 EPÜ: 22215806.5 / 4 190 882; 19213293.4 / 3 653 689; 17159412.0 / 3 205 705; 14729297.3 / 3 004 294
(73) Patentinhaber: BASF SE, 67056 Ludwigshafen am Rhein (DE)
(72) Erfinder: HANSCH, Markus, 67346 Speyer (DE); BÖHNKE, Harald, 68163 Mannheim (DE); GRABARSE, Wolfgang, 68163 Mannheim (DE); VÖLKEL, Ludwig, 67117 Limburgerhof (DE); PERETOLCHIN, Maxim, 67466 Lambrecht (DE)
(74) Vertreter: BASF IP Association

(56) Entgegenhaltungen:
- WO-A1-2012/004300
- WO-A1-2013/043332
- WO-A2-2006/135881

## Beschreibung

Die vorliegende Erfindung betrifft die Verwendung in spezieller Weise quaternisierter Stickstoffverbindungen als Kraft- und Schmierstoffadditiv oder Kerosinadditiv, wie insbesondere als Detergensadditiv; zur Verringerung oder Verhinderung von Ablagerungen in den Einspritzsystemen von direkteinspritzenden Dieselmotoren, insbesondere in Common-Rail-Einspritzsystemen, zur Verringerung des Kraftstoffverbrauches von direkteinspritzenden Dieselmotoren, insbesondere von Dieselmotoren mit Common-Rail-Einspritzsystemen, und zur Minimierung des Leistungsverlustes (power loss) in direkteinspritzenden Dieselmotoren, insbesondere in Dieselmotoren mit Common-Rail-Einspritzsystemen; sowie als Additiv für Ottokraftstoffe, insbesondere zum Betrieb von DISI Motoren.

### Stand der Technik:

Bei direkteinspritzenden Dieselmotoren wird der Kraftstoff durch eine direkt in den Brennraum des Motors reichende Mehrloch-Einspritzdüse eingespritzt und feinst verteilt (vernebelt), anstatt wie beim klassischen (Kammer-) Dieselmotor in eine Vor- oder Wirbelkammer eingeführt zu werden. Der Vorteil der direkteinspritzenden Dieselmotoren liegt in ihrer für Dieselmotoren hohen Leistung und einem dennoch geringen Verbrauch. Außerdem erreichen diese Motoren ein sehr hohes Drehmoment schon bei niedrigen Drehzahlen.

Zurzeit werden im Wesentlichen drei Verfahren eingesetzt, um den Kraftstoff direkt in den Brennraum des Dieselmotors einzuspritzen: die konventionelle Verteilereinspritzpumpe, das Pumpe-Düse-System (Unit-Injector-System bzw. Unit-Pump-System) und das Common-Rail-System.

Beim Common-Rail-System wird der Dieselkraftstoff von einer Pumpe mit Drücken bis zu 2000 bar in eine Hochdruckleitung, die Common-Rail gefördert. Ausgehend von der Common-Rail laufen Stichleitungen zu den verschiedenen Injektoren, die den Kraftstoff direkt in den Brennraum injizieren. Dabei liegt auf der Common-Rail stets der volle Druck an, was eine Mehrfacheinspritzung oder eine spezielle Einspritzform ermöglicht. Bei den anderen Injektionssystemen ist dagegen nur eine geringere Variation der Einspritzung möglich. Die Einspritzung beim Common-Rail wird im Wesentlichen in drei Gruppen unterteilt: (1.) Voreinspritzung, durch die im Wesentlichen eine weichere Verbrennung erreicht wird, so dass harte Verbrennungsgeräusche ("Nageln") vermindert werden und der Motorlauf ruhig erscheint; (2.) Haupteinspritzung, die insbesondere für einen guten Drehmomentverlauf verantwortlich ist; und (3.) Nacheinspritzung, die insbesondere für einen geringen NOₓ-Wert sorgt. Bei dieser Nacheinspritzung wird der Kraftstoff in der Regel nicht verbrannt, sondern durch Restwärme im Zylinder verdampft. Das dabei gebildete Abgas-/Kraftstoffgemisch wird zur Abgasanlage transportiert, wo der Kraftstoff in Gegenwart geeigneter Katalysatoren als Reduktionsmittel für die Stickoxide NOₓ wirkt.

Durch die variable, zylinderindividuelle Einspritzung kann beim Common-Rail-Einspritzsystem der Schadstoffausstoß des Motors, z.B. der Ausstoß von Stickoxiden (NOₓ), Kohlenmonoxid (CO) und insbesondere von Partikeln (Ruß), positiv beeinflusst werden. Dies ermöglicht beispielsweise, dass mit Common-Rail-Einspritzsystemen ausgerüstete Motoren der Euro 4-Norm theoretisch auch ohne zusätzlichen Partikelfilter genügen können.

In modernen Common-Rail-Dieselmotoren können sich unter bestimmten Bedingungen, beispielsweise bei Verwendung von biodieselhaltigen Kraftstoffen oder von Kraftstoffen mit Metall-Verunreinigungen wie Zink-Verbindungen, Kupfer-Verbindungen, Bleiverbindungen und weiteren Metallverbindungen, an den Injektoröffnungen Ablagerungen bilden, die das Einspritzverhalten des Kraftstoffs negativ beeinflussen und dadurch die Performance des Motors beeinträchtigen, d.h. insbesondere die Leistung verringern, aber zum Teil auch die Verbrennung verschlechtern. Die Bildung von Ablagerungen wird durch bauliche Weiterentwicklungen der Injektoren, insbesondere durch die Veränderung der Geometrie der Düsen (engere, konische Öffnungen mit abgerundetem Auslass) noch verstärkt. Für eine dauerhaft optimale Funktionsweise von Motor und Injektoren müssen solche Ablagerungen in den Düsenöffnungen durch geeignete Kraftstoffadditive verhindert oder reduziert werden

In den Einspritzsystemen modernen Dieselmotoren verursachen Ablagerungen signifikante Performance-Probleme. Weit verbreitet ist die Erkenntnis, dass derartige Ablagerungen in den Sprühkanälen zu einer Verringerung des Kraftstoffflusses und damit zu Leistungsverlusten (power loss) führen können. Ablagerungen an der Injektorspitze beeinträchtigen dagegen die optimale Ausbildung von Kraftstoff-Sprühnebel und bedingen dadurch eine verschlechterte Verbrennung und damit verbunden höhere Emissionen und vermehrten Kraftstoffverbrauch. Im Gegensatz zu diesen herkömmlichen, "äußeren" Ablagerungsphänomenen bereiten auch "interne" Ablagerungen (zusammengefasst als innere Diesel-Injektor-Ablagerungen (IDID)) in bestimmten Teilen der Injektoren, wie an der Düsennadel, am Steuerkolben, am Ventilkolben, am Ventilsitz, an der Ansteuereinheit und an den Führungen dieser Komponenten zunehmend Performance-Probleme. Herkömmliche Additive zeigen eine unzureichende Wirkung gegen diese IDIDs.

Aus der US 4,248,719 sind quaternisierte Ammoniumsalze beschrieben, welche durch Umsetzung eines Alkenylsuccinimids mit einem Monocarbonsäureester hergestellt werden und als Dispergiermittel in Schmierölen zur Verhinderung von Schlammbildung Anwendung finden. Insbesondere ist beispielsweise die Umsetzung von Polyisobutylbernsteinsäureanhydrid (PIBSA) mit N,N-Dimethylaminopropylamin (DMAPA) und Quaternisierung mit Methylsalicylat beschrieben. Eine Anwendung in Kraftstoffen, insbesondere Dieselkraftstoffen, wird darin jedoch nicht vorgeschlagen. Die Verwendung von PIBSA mit niedrigen Bismaleinierungsgraden < 20 % wird darin nicht beschrieben.

Aus der US 4,171,959 sind quaternisierte Ammoniumsalze von hydrocarbylsubstituierten Succinimiden beschrieben, welche als Detergenzadditive für Ottokraftstoffzusammensetzungen geeignet sind. Zur Quaternisierung werden bevorzugt Alkylhalogenide eingesetzt. Erwähnt sind außerdem organische C₂-C₈-Hydrocarbyl-Carboxylate und -Sulfonate. Folglich weisen die gemäß dortiger Lehre bereitgestellten quaternisierten Ammoniumsalze als Gegenion entweder ein Halogenid oder ein C₂-C₈-Hydrocarbyl-Carboxylat oder ein C₂-C₈-Hydrocarbyl-Sulfonat-Gruppe auf. Die Verwendung von PIBSA mit niedrigen Bismaleinierungsgraden <20 % wird darin ebenfalls nicht beschrieben.

Aus der EP-A-2 033 945 sind Kaltfließverbesserer bekannt, welche durch Quaternisierung spezieller tertiärer Monoamine, die wenigstens einen C₈-C₄₀-Alkylrest tragen, mit einem C₁-C₄-Alkylester spezieller Carbonsäuren hergestellt werden. Beispiele solcher Carbonsäureester sind Dimethyloxalat, Dimethylmaleat, Dimethylphthalat und Dimethylfumarat. Andere Anwendungen als zur Verbesserung des CFPP Werts von Mitteldestillaten sind in der EP-A-2 033 945 nicht belegt.

Die WO 2006/135881 beschreibt quaternisierte Ammoniumsalze, hergestellt durch Kondensation eines Hydrocarbyl-substituierten Acylierungsmittels und einer Sauerstoff-oder Stickstoffatom-haltigen Verbindung mit tertiärer Aminogruppe, und anschließender Quaternisierung mittels Hydrocarbylepoxid in Kombination mit stöchiometrischen Mengen einer Säure, wie insbesondere Essigsäure. Weitere in der WO 2006/135881 beanspruchte Quaternisierungsmittel sind Dialkylsulfate, Benzylhalogenide und hydrocarbylsubstituierte Carbonate, wobei Dimethylsulfat, Benzylchlorid und Dimethylcarbonat experimentell untersucht wurden.

Die in der WO 2006/135881 bevorzugt verwendeten Quaternisierungsmittel weisen jedoch gravierende Nachteile auf, wie: Toxizität bzw. Carcinogenität (z.B. bei Dimethylsulfat und Benzylhalogeniden), keine rückstandsfreie Verbrennung (z.B. bei Dimethylsulfat und Alkylhalogeniden), sowie unzureichende Reaktivität, welche zu unvollständiger Quaternierung oder nicht-ökonomischen Reaktionsbedingungen (lange Reaktionszeiten, hohe Reaktionstemperaturen, Überschuss an Quaternierungsmittel; z.B. bei Dimethylcarbonat) führt.

Die EP-A-2 033 945 beschreibt die Herstellung halogen- und schwefelfreier quaternärer Ammoniumsalze von organischen Carbonsäuren (wie z.B. Oxalsäure, Phthalsäure, Salicylsäure, Malonsäure und Maleinsäure sowie deren Alkylester) und deren Verwendung zur Verbesserung des CFPP-Werts von Dieselkraftstoffen.

Quaternäre Ammoniumsalze von alpha-Hydroxycarbonsäuren werden in der EP-A-1 254 889 als Reinigungsmittel für elektronische Bauteile vorgeschlagen.

Weiterhin beschreibt die Japanische Patentanmeldung, Anmeldenummer 61-012197, die Verwendung quaternärer Ammoniumsalze von organischen Carbonsäuren als Oberflächenaktives mittel oder Rohmaterial für Medikamente oder Kosmetika.

Die WO2012004300 beschreibt ein quaternisiertes Hydrocarbyl-substituiertes Bernsteinsäureanhydrid, das mit einem Hydrocarbyl-Epoxid quaternisierten wurde und seine Verwendung zur Vermeidung von Ablagerungen in Dieseleinspritzsystemen.

Es bestand daher die Aufgabe, weitere Kraftstoffadditive bereitzustellen, welche Ablagerungen in der Injektorspitze und interne Injektorablagerungen beim Betrieb von Common-Rail-Dieselmotoren verhindern.

### Kurze Beschreibung der Erfindung:

Die Erfindung ist in den angefügten Ansprüchen 1 bis 14 definiert.

Es wurde nun überraschenderweise gefunden, dass obige Aufgabe durch die Verwendung quaternisierter Stickstoffverbindungen, wie z.B. Hydrocarbylaminverbindungen gelöst wird.

Überraschenderweise sind die verwendeten Additive wie insbesondere durch die beiliegenden Anwendungsbeispiele veranschaulicht, überraschend wirksam in Common-Rail-Dieselmotoren und zeichnen sich durch ihre besondere Eignung als Additiv zur Verringerung von Powerloss durch externe und Kaltstartprobleme durch interne Ablagerungen aus.

### Figurenbeschreibung:

Figur 1 zeigt den Ablauf eines einstündigen Motorentestzyklus gemäß CEC F-098-08.
Figur 2A bis D zeigt photographische Aufnahmen von Injektoren eines DISI Benzinmotors, betrieben mit Kraftstoff, entweder unadditiviert (A) oder additiviert mit verschiedenen erfindungsgemäßen Additiven (B, C, D)

### Detaillierte Beschreibung der Erfindung:

### A1) Spezielle Ausführungsformen

Die vorliegende Erfindung betrifft die Verwendung quaternisierten Stickstoffverbindungen als Additiv in Kraftstoffen, wie in den erteilten Patentansprüchen und in der folgenden Beschreibung näher definiert.

Die vorliegende Erfindung betrifft insbesondere folgende spezielle Ausführungsformen:
Die Erfindung betrifft insbesonere Verwendung einer quaternisierten Stickstoffverbindung erhältlich durch
Quaternisierung einer quaternisierbaren Stickstoffverbindung, wie z.B. eines quaternisierbaren Alkylamins, enthaltend wenigstens eine quaternisierbare, insbesondere tertiäre Aminogruppe mit einem Quaternierungsmittel, das die wenigstens eine quaternisierbare, insbesondere tertiäre Aminogruppe in eine quaternäre Ammoniumgruppe überführt,
wobei das Quaternierungsmittel ein Hydrocarbylepoxid in Kombination mit einer freien Hydrocarbyl-substituierten Polycarbonsäure ist,
als Detergensadditiv in Kraftstoffen; oder
als Dieselkraftstoffadditiv zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen; oder
als Ottokraftstoffadditiv zur Verringerung von Ablagerungen im Einlasssystem eines Ottomotors, wie insbesondere DISI und PFI (Port Fuel Injector) -Motoren;
   oder
als Dieselkraftstoffadditiv zur Verringerung und/oder Vermeidung von Ablagerungen in den Einspritzsystemen, bevorzugt bestimmt in einem XUD 9 Test, nach CEC-F-23-1-01 oder in einem DW10 Test in Anlehnung an CEC F-98-08, wie insbesondere der Internal Diesel Injector Deposits (IDID) und / oder von Ventilkleben in direkteinspritzenden Dieselmotoren, insbesondere in Common-Rail-Einspritzsystemen.

Insbesondere ist dabei die quaternisierbare Stickstoffverbindung, ausgewählt ist unter:
a) wenigstens einem Alkylamin, das wenigstens eine Verbindung der folgenden allgemeinen Formel 3 umfasst,

   RₐR_{b}R_{c}N (3)

   worin
   wenigstens einer der Reste Rₐ ,R_{b} und R_{c}, wie z.B. einer oder zwei, für einen geradkettigen oder verzweigte, gesättigten oder ungesättigten C₈-C₄₀-Hydrocarbylrest (insbesondere geradkettigen oder verzweigte C₈ - C₄₀-Alkyl) steht und die übrigen Reste für gleiche oder verschiedene, geradkettige oder verzweigte, gesättigte oder ungesättigten C₁-C₆-Hydrocarbylreste (insbesondere C₁-C₆-Alkyl) stehen; oder
   worin alle Reste Rₐ ,R_{b} und R_{c} für gleiche oder verschiedene geradkettige oder verzweigte, gesättigten oder ungesättigten C₈-C₄₀-Hydrocarbylreste, insbesondere geradkettige oder verzweigte C₈ - C₄₀-Alkyl-Reste stehen; oder
   worin die Reste Rₐ, R_{b} und R_{c} gleiche oder verschiedene kurzkettige Alkylreste, insbesondere geradkettige oder verzweigte Alkylgruppen mit 1 bis 7 oder insbesondere 1 bis 4 Kohlenstoffatomen aufweisen;
b) wenigstens einem Polyalken-substituierten Amin, enthaltend wenigstens eine quaternisierbare, insbesondere tertiäre, Aminogruppe;
c) wenigstens einem Polyether-substituierten Amin, enthaltend wenigstens eine quaternisierbare, insbesondere tertiäre, Aminogruppe; und
d) wenigstens einem Reaktionsprodukt eines hydrocarbylsubstituierten Acylierungsmittels und einer Verbindung, enthaltend ein Stickstoff- oder Sauerstoffatom und zusätzlich enthaltend wenigstens eine quaternisierbare, insbesondere tertiäre Aminogruppe; und
e) Mischungen davon;

Insbesondere ist das Quaternierungsmittel ein Epoxid der allgemeinen Formel 4 umfasst 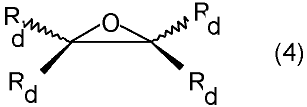 wobei
die darin enthaltenen Reste R_{d} gleich oder verschieden sind und für H oder für einen Hydrocarbylrest stehen, wobei der Hydrocarbylrest für einen aliphatischen oder aromatischen Rest mit wenigstens 1 bis 10 Kohlenstoffatomen steht

Insbesondere ist die freie Säure des Quaternierungsmittels eine hydrocarbyl-substituierte C₃-C₂₈- Dicarbonsäure.

Insbesondere weist der Hydrocarbyl-Substituent der Carbonsäure ein zahlengemitteltes Molekulargewicht (Mₙ) von 85 bis 20.000 aufweist; oder ist ein Polyalkylenrest mit einem Polymerisationsgrad von 2 bis 100, oder 3 bis 50 oder 4 bis 25 ist.

Insbesondere ist das quaternisierbare tertiäre Amin eine Verbindung der Formel 3, , worin wenigstens zwei der Reste Rₐ ,R_{b} und R_{c} gleich oder verschieden sind und für einen geradkettigen oder verzweigte, C₁₀-C₂₀-Alkylrest steht und der übrigen Rest für C₁-C₄-Alkyl steht.

Insbesondere ist das Quaternierungsmittel ausgewählt unter Hydrocarbylepoxiden der obigen allgemeinen Formel 4 in Kombination mit einer hydrocarbyl-substituierten C₃-C₂₈-Dicarbonsäure.

Insbesondere ist die Carbonsäure ausgewählt ist unter Hydrocarbyl-substituierter Malon-, Bernstein-, Glutar-, Adipin- und Pimelinsäure.

Insbesondere ist das quaternisierbare Amin ausgewählt aus der Gruppe bestehend aus Hydroxyalkyl-substituierten primären, sekundären, und insbesondere tertiären Monoaminen und Hydroxyalkyl-substituierten primären, sekundären und insbesondere tertiären Diaminen.

Insbesondere handelt es sich bei dem quaternisierbaren Amin um ein hydroxyalkylsubstituiertes Monoamin handelt, ausgewählt ist aus der Gruppe bestehend aus N-Hydroxyalkyl-monoaminen, N,N-Dihydroxyalkyl-monoaminen und N,N,N-Trihydroxyalkyl-monoaminen.

Insbesondere ist das quaternisierbare Amin ausgewählt aus der Gruppe bestehend aus Ethanolamin, 3-Hydroxy-1-propylamin, Diethanolamin, Diisopropanolamin, N-(2-Hydroxyethyl)ethylendiamin, Triethanolamin, 1-(3-Hydroxypropyl)imidazol, Tris(hydroxymethyl)amin, 3-Dimethylamino-1-propanol, 3-Diethylamino-1-propanol, 2-Dimethylamino-1-ethanol und 4-Diethylamino-1-butanol.

Insbesondere ist der Kraftstoff ausgewählt unter Dieselkraftstoffen, Biodieselkraftstoffen, Ottokraftstoffen, und Alkanol-haltigen Ottokraftstoffen., wie Bioethanol-haltige Kraftstoffe, insbesondere Dieselkraftstoffe.

Insbesondere handelt es sich bei dem Kraftstoff um Diesel- oder eine Biodiesel-Kraftstoff-Zusammensetzung, umfassend Biobrennstofföl, in Mengen von 1 bis 30 Gew.-%, insbesondere von 3 bis 10 Gew.-%, bezogen auf die Gesamtmenge aus Mitteldestillat fossilen, pflanzlichem oder tierischen Ursprungs und Biobrennstofföl; insbesondere basierend auf Alkylestern von Fettsäuren; vor allem Niedrigalkylestern, insbesondere C₁- bis C₄-Alkylestern, die durch Umesterung der in pflanzlichen und/oder tierischen Ölen und/oder Fetten vorkommenden Glyceride, insbesondere Triglyceride, mittels Niedrigalkoholen, erhältlich sind. Insbesondere ist dabei der Alkylester von Fettsäure ausgewählt ist unter Sonnenblumenmethylester, Palmölmethylester ("PME"), Sojaölmethylester ("SME"), hydrierte Pflanzenöle ("HVO"), used kitchen oil ("UKO") und insbesondere Rapsölmethylester ("RME").

Hierin beschrieben, aber nicht Gegenstand der Erdfindung sind quaternisierte Stickstoffverbindung gemäß der obigen Definition.

Hierin beschrieben, aber nicht Gegenstand der Erdfindung ist ein Verfahren zur Herstellung solcher quaternisierten Stickstoffverbindungen,
umfassend die Umsetzung eines quaternisierbaren Alkylamins, enthaltend wenigstens eine quaternisierbare, tertiäre Aminogruppe mit einem Quaternierungsmittel, das die wenigstens eine tertiäre Aminogruppe in eine quaternäre Ammoniumgruppe überführt,
wobei das Quaternierungsmittel ein Hydrocarbylepoxid in Kombination mit einer Hydrocarbyl-substituierten Polycarbonsäure ist.

Hierin beschrieben, aber nicht Gegenstand der Erdfindung sind Additivkonzentrate, enthaltend in Kombination mit weiteren Diesel- oder Ottokraftstoffadditiven oder Schmierstoffadditiven wenigstens eine quaternisierte Stickstoffverbindung gemäß obiger Definition oder hergestellt wie oben beschrieben.

Jeweils geeignete Testmethoden zur Überprüfung der oben bezeichneten Anwendungen sind den Fachmann bekannt, bzw. in folgenden experimentellen Teil, worauf hiermit ausdrücklich allgemein Bezug genommen wird, beschrieben.

### A2) Allgemeine Definitionen

Werden keine gegenteiligen Angaben gemacht, so gelten folgende allgemeine Bedeutungen:
"Quaternisierbare" Stickstoffgruppen oder Aminogruppen umfassen insbesondere primäre, sekundäre und , vor allem, tertiäre Aminogruppen.
"Hydrocarbyl" ist breit auszulegen und umfasst sowohl langkettige als auch kurzkettige, gerade oder verzweigte Kohlenwasserstoffreste mit 1 bis 50 Kohlenstoffatomen, welche ggf. zusätzlich Heteroatome, wie z.B. O, N, NH,S, in ihrer Kette enthalten können. Eine spezielle Gruppe von Hydrocarbyl Resten umfasst sowohl langkettige als auch kurzkettige, geradkettige oder verzweigte Alkylreste mit 1 bis 1000, 3 bis 500 4 bis 400 Kohlenstoffatomen.
"Langkettige" oder "Hochmolekulare" Hydrocarbylreste stehen für geradkettige oder verzweigte Kohlenwasserstoffreste und weisen 7 bis 50 oder 8 bis 50 oder 8 bis 40 oder 10 bis 20 Kohlenstoffatomen, welche ggf. zusätzlich Heteroatome, wie z.B. O, N, NH,S, in ihrer Kette enthalten können. Weiterhin können die Reste ein- oder mehrfach ungesättigt sein und ein oder mehrere nicht-kumulierte, wie z.B. 1 bis 5, wie 1, 2 oder 3 C-C Doppelbindungen oder C-C- Dreifachbindungen, insbesondere 1, 2 oder 3 Doppelbindungen, aufweisen. Sie können natürlichen oder synthetischen Ursprungs sein.

Sie können auch ein zahlengemitteltes Molekulargewicht (Mₙ) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500 aufweisen Sie sind dann insbesondere im Wesentlichen aus C₂₋₆-, insbesondere C₂₋₄-Monomerbausteinen, wie Ethylen, Propylen, n- oder iso-Butylen oder Mischungen davon aufgebaut, wobei die verschiedenen Monomere statistisch verteilt oder als Blöcke einpolymerisiert enthalten sein können. Derartige langkettige Hydrocarbylreste werden auch als Polyalkylenreste oder Poly-C₂₋₆- oder Poly-C₂₋₄-alkylenreste bezeichnet. Geeignete langkettige Hydrocarbylreste und deren Herstellung sind beispielsweise auch beschreiben in der WO2006/135881 und der dort zitierten Literatur.

Beispiele für besonders brauchbare Polyalkylen-Reste sind Polyisobutenyl-Reste, abgeleitet von sogenannten "hochreaktiven" Polyisobutenen, die sich durch einen hohen Gehalt an terminal angeordneten Doppelbindungen auszeichnen. Terminal angeordnete Doppelbindungen sind dabei alpha-olefinische Doppelbindungen des Typs 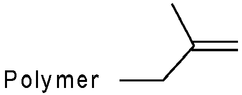 welche zusammen auch als Vinyliden-Doppelbindungen bezeichnet werden. Geeignete hochreaktive Polyisobutene sind beispielsweise Polyisobutene, die einen Anteil an Vinyliden-Doppelbindungen von größer 70 Mol-%, insbesondere größer 80 Mol-% oder größer 85 Mol-% aufweisen. Bevorzugt sind insbesondere Polyisobutene, die einheitliche Polymergerüste aufweisen. Einheitliche Polymergerüste weisen insbesondere solche Polyisobutene auf, die zu wenigstens 85 Gew.-%, vorzugsweise zu wenigstens 90 Gew.-% und besonders bevorzugt zu wenigstens 95 Gew.-% aus Isobuteneinheiten aufgebaut sind. Vorzugsweise weisen solche hochreaktiven Polyisobutene ein zahlenmittleres Molekulargewicht in dem oben genannten Bereich auf. Darüber hinaus können die hochreaktiven Polyisobutene eine Polydispersität im Bereich von 1,05 bis 7, insbesondere von etwa 1,1 bis 2,5, wie z.B. von kleiner 1,9 oder kleiner 1,5, aufweisen. Unter Polydispersität versteht man den Quotienten aus gewichtsmittlerem Molekulargewicht Mw geteilt durch das zahlenmittlere Molekulargewicht Mn.

Besonders geeignete hochreaktive Polyisobutene sind z.B. die Glissopal-Marken der BASF SE, insbesondere Glissopal 1000 (Mn = 1000), Glissopal V 33 (Mn = 550) und Glissopal 2300 (Mn = 2300) und deren Mischungen. Andere zahlenmittlere Molekulargewichte können nach im Prinzip bekannter Weise durch Mischen von Polyisobutenen unterschiedlicher zahlenmittlerer Molekulargewichte oder durch extraktive Anreicherung von Polyisobutenen bestimmter Molekulargewichtsbereiche eingestellt werden.

Eine spezielle Gruppe von langkettigen Hydrocarbyl Resten umfasst geradkettige oder verzweigte Alkylresten ("langkettige" Alkylreste") mit 8 bis 50, wie z.B. 8 bis 40 oder 8 bis 30 oder 10 bis 20 Kohlenstoffatomen.

Eine weitere Gruppe spezieller langkettiger Hydrocarbylreste umfasst Polyalkylenreste, die insbesondere im Wesentlichen aus C₂₋₆-, insbesondere C₂₋₄-Monomerbausteinen, wie Ethylen, Propylen, n- oder iso-Butylen oder Mischungen davon aufgebaut ssind und einen Polymerisationsgrad von 2 bis 100, oder 3 bis 50 oder 4 bis 25 aufweisen.,

"Kurzkettiges Hydrocarbyl" oder "niedermolekulares Hydrocarbyl" steht insbesondere für geradkettiges oder verzweigtes Alkyl oder Alkenyl, gegebenenfalls unterbrochen durch eine oder mehrere, wie z.B. 2, 3 oder 4 Heteroatomgruppen, wie -O- oder -NH-. oder gegebenenfalls ein- oder mehrfach, wie z.B. 2, 3 oder 4-fach substituiert.

"Hydrocarbylen" steht für geradkettige oder ein- oder mehrfach verzweigte Brückengruppen mit 1 bis 10 Kohlenstoffatomen, gegebenenfalls unterbrochen durch eine oder mehrere, wie z.B. 2, 3 oder 4 Heteroatomgruppen, wie -O- oder -NH-. oder gegebenenfalls ein- oder mehrfach, wie z.B. 2, 3 oder 4-fach substituiert.

"Alkyl" oder "Niedrigalkyl" steht insbesondere für gesättigte, geradkettige oder verzweigte Kohlenwasserstoffreste mit 1 bis 4, 1 bis 5, 1 bis 6, oder 1 bis 7, Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, sowie die ein- oder mehrfach verzweigten Analoga davon.

"Langkettiges Alkyl" steht beispielsweise für gesättigte, geradkettige oder verzweigte Kohlenwasserstoffreste mit 8 bis 50, wie z.B. 8 bis 40 oder 8 bis 30 oder 10 bis 20 Kohlenstoffatomen, wie Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Hencosyl, Docosyl, Tricosyl, Tetracosyl, Pentacosyl, Hexacosyl, Heptacosyl, Octacosyl, Nonacosyl, Squalyl, Konstitutionsisomere, insbesondere ein oder mehrfach verzweigte Isomere und höhere Homologe davon.

"Hydroxyalkyl" steht insbesondere für die ein- oder mehrfach, insbesondere einfach hydroxylierten Analoga obiger Alkylreste, wie z.B. die monohydroxylierten Analoga obiger geradkettiger oder verzweigter Alkylreste, wie z.B. die linearen Hydroxyalkylgruppen, wie z.B. solchen mit primärer (endständigen) Hydroxylgruppe, wie Hydroxymethyl, 2-Hydroxyethyl, 3-Hydroxypropyl, 4-Hydroxybutyl, oder solchen mit nichtendständigen Hydroxylgruppen, wie 1-Hydroxyethyl, 1- oder 2-Hydroxypropyl, 1- oder 2-Hydroxybutyl oder 1-, 2- oder 3-Hydroxybutyl.

"Alkenyl" steht für ein- oder mehrfach, insbesondere einfach ungesättigte, geradkettige oder verzweigte Kohlenwasserstoffreste mit 2 bis 4, 2 bis 6, oder 2 bis 7 Kohlenstoffatomen und einer Doppelbindung in einer beliebigen Position, z. B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

"Hydroxyalkenyl" steht insbesondere für die ein- oder mehrfach, insbesondere einfach hydroxylierten Analoga obiger Alkenylreste

"Aminoalkyl" und "Aminoalkenyl" steht insbesondere für die ein- oder mehrfach, insbesondere einfach aminierten Analoga obiger Alkyl- bzw. Alkenylreste, oder Analoga obiger Hydroxyalkyl, wobei die OH-Gruppe durch eine Aminogruppe ersetzt ist.

"Alkylen" steht für geradkettige oder ein- oder mehrfach verzweigte Kohlenwasserstoff-Brückengruppen mit 1 bis 10 Kohlenstoffatomen, wie z.B. C₁-C₇-Alkylengruppen ausgewählt unter -CH₂-, -(CH₂)₂-, -(CH₂)₃-,-(CH₂)₄-, -(CH₂)₂-CH(CH₃)-, -CH₂-CH(CH₃)-CH₂-, (CH₂)₄-, -(CH₂)₅-, -(CH₂)₆, -(CH₂)₇-, -CH(CH₃)-CH₂-CH₂-CH(CH₃)- oder -CH(CH₃)-CH₂-CH₂-CH₂-CH(CH₃)- oder C₁-C₄-Alkylengruppen ausgewählt unter -CH₂-, -(CH₂)₂-, - (CH₂)₃-,-(CH₂)₄-, -(CH₂)₂-CH(CH₃)-, -CH₂-CH(CH₃)-CH₂-
oder für C₂-C₆-Alkylengruppen, wie z.B.

-CH₂-CH(CH₃)-, -CH(CH₃)-CH₂-, -CH(CH₃)-CH(CH₃)-, -C(CH₃)₂-CH₂-, -CH₂-C(CH₃)₂-,

-C(CH₃)₂-CH(CH₃)-, -CH(CH₃)-C(CH₃)₂-, -CH₂-CH(Et)-, -CH(CH₂CH₃)-CH₂-,

-CH(CH₂CH₃)-CH(CH₂CH₃)-, -C(CH₂CH₃)₂-CH₂-, -CH₂-C(CH₂CH₃)₂-,

-CH₂-CH(n-Propyl)-, -CH(n-Propyl)-CH₂-, -CH(n-Propyl)-CH(CH₃)-, -CH₂-CH(n-Butyl)-,

-CH(n-Butyl)-CH₂-, -CH(CH₃)-CH(CH₂CH₃)-, -CH(CH₃)-CH(n-Propyl)-, -CH(CH₂CH₃)-

CH(CH₃)-, -CH(CH₃)-CH(CH₂CH₃)-, oder für C₂-C₄-Alkylengruppen, wie z.B. ausgewählt unter -(CH₂)₂-, -CH₂-CH(CH₃)-, -CH(CH₃)-CH₂-, -CH(CH₃)-CH(CH₃)-, -C(CH₃)₂-CH₂-, -CH₂-C(CH₃)₂-, -CH₂-CH(CH₂CH₃)-, -CH(CH₂CH₃)-CH₂-.

"Oxyalkylen-Reste entsprechen der Definition obiger geradkettiger oder ein- oder mehrfach verzweigter Alkylenreste mit 2 bis 10 Kohlenstoffatomen, wobei die Kohlenkette durch ein Sauerstoff-Heteroatom 1- oder mehrfach, insbesondere 1-fach unterbrochen ist. Als nichtlimitierende Beispiele sind zu nennen: -CH₂-O-CH₂-, -(CH₂)₂-O-(CH₂)₂-, -(CH₂)₃-O-(CH₂)₃-, oder -CH₂-O-(CH₂)₂-, -(CH₂)₂-O-(CH₂)₃-, -CH₂-O-(CH₂)₃

"Aminoalkylen"entsprechen der Definition obiger geradkettiger oder ein- oder mehrfach verzweigter Alkylenreste mit 2 bis 10 Kohlenstoffatomen, wobei die Kohlenkette durch eine Stickstoffgruppe (insbesondere -NH-Gruppe) 1- oder mehrfach, insbesondere 1-fach unterbrochen ist. Als nichtlimitierende Beispiele sind zu nennen: -CH₂-NH-CH₂-, - (CH₂)₂-NH-(CH₂)₂-, -(CH₂)₃-NH-(CH₂)₃-, oder -CH₂-NH-(CH₂)₂-, -(CH₂)₂-NH-(CH₂)₃-, - CH₂-NH-(CH₂)₃.

"Alkenylen" steht für die ein- oder mehrfach, insbesondere einfach ungesättigten Analoga obiger Alkylengruppen mit 2 bis 10 Kohlenstoffatomen, insbesondere für C₂-C₇-Alkenylene oder C₂-C₄-Alkenylen, wie -CH=CH-, -CH=CH-CH₂-, - CH₂-CH=CH-, -CH=CH-CH₂-CH₂-, -CH₂-CH=CH-CH₂-, -CH₂-CH₂-CH=CH-, -CH(CH₃)-CH=CH-, -CH₂-C(CH₃)=CH-.

-"Cycloalkyl" steht für carbocyclische Reste mit 3 bis 20 Kohlenstoffatomen, wie z.B. C₃-C₁₂-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder C₃-C₇-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann.

"Cycloalkenyl" oder ein- oder mehrfach ungesättigtes Cycloalkyl" steht insbesondere für monocyclische, ein oder mehrfach ungesättigte Kohlenwasserstoffgruppen mit 5 bis 8, vorzugsweise bis 6 Kohlenstoffringgliedern, wie z.B. die einfach ungesättigten Rests Cyclopenten-1-yl, Cyclopenten-3-yl, Cyclohexen-1-yl, Cyclohexen-3-yl und Cyclohexen-4-yl;

"Aryl" steht für ein- oder mehrkernige, vorzugsweise ein- oder zweikernige, gegebenenfalls substituierte aromatische Reste mit 6 bis 20 wie z.B. 6 bis 10 RingKohlenstoffatomen, wie z.B. Phenyl, Biphenyl, Naphthyl wie 1- oder 2-Naphthyl, Tetrahydronaphthyl, Fluorenyl, Indenyl und Phenanthrenyl. Diese Arylreste können gegebenenfalls 1, 2, 3, 4, 5 oder 6 gleiche oder verschiedene Substituenten tragen.

"Alkylaryl" steh für die in beliebiger Ringposition ein- oder mehrfach, insbesondere 1-oder 2-fach, Alkyl-substituierten Analoga obiger Arylreste, wobei Aryl ebenfalls die oben angegebenen Bedeutungen besitzt, wie z.B. C₁-C₄-Alkyl-Phenyl-, wobei der C₁-C₄-Alkylreste in beliebiger Ringposition liegen können..

"Substituenten" für hierin angegebene Reste, sind insbesondere, wenn keine anderen Angaben gemacht werde, ausgewählt sind unter Ketogruppen, - COOH, -COO-Alkyl, - OH, -SH, -CN, Amino, -NO₂, Alkyl, oder Alkenylgruppen.

"Mn" steht für das zahlenmittleres Molekulargewicht und wird in herkömmlicher Weise bestimmt; insbesondere beziehen sich solche Angaben auf Mn-Werte, bestimmt durch Relativmethoden, wie der Gelpermeationschromatographie mit THF als Eluent und Polystyrol-Standards oder Absolutmethoden, wie der Dampfphasenosmometrie unter Verwendung von Toluol als Lösungsmittel.

"Mw" steht für das gewichtsmittlere Molekulargewicht und wird in herkömmlicher Weise bestimmt; insbesondere beziehen sich solche Angaben auf Mw-Werte, bestimmt durch Relativmethoden, wie der Gelpermeationschromatographie mit THF als Eluent und Polystyrol-Standards oder Absolutmethoden, wie der Lichtstreuung.

Der "Polymerisationsgrad"bezeichnet gewöhnich den zahlenmäßigen mittleren Polymerisationsgrad (Bestimmungsmethode Gelpermeationschromatographie mit THF als Eluent und Polystyrol-Standards; oder GC-MS Kopplung).

### A3) Quaternisierbare Stickstoffverbindungen

Quaternisierbare Stickstoffverbindungen sind insbesondere:

### A3.1) Tertiäre Amine

Tertiäre Amine sind insbesondere Verbindungen der obigen Formel (3) und sind an sich bekannte Verbindungen, wie z.B. beschrieben in der EP-A-2 033 945.

Das tertiäre Amin-Edukt (3) trägt vorzugsweise ein Segment der Formel NRₐ R_{b} wobei einer der Reste eine Alkylgruppe mit 8 bis 40 Kohlenstoffatomen und der andere eine Alkylgruppe mit bis zu 40, besonders bevorzugt 8 bis 40 Kohlenstoffatomen aufweist. Der Rest R_{c} ist dabei insbesondere ein kurzkettiger C₁-C₆-Alkylrest, wie eine Methyl-, Ethyl- oder Propyl-Gruppe. Rₐ und R_{b} können geradkettig oder verzweigt sein, und / oder können gleich oder verschieden sein. Beispielsweise können Rₐ und R_{b} eine geradkettige C₁₂-C₂₄-Alkylgruppe sein. Alternativ kann nur einer der beiden Reste langkettig (z.B. mit mit 8 bis 40 Kohlenstoffatomen) sein und der andere eine Methyl-, Ethyl- oder Propyl-Gruppe darstellen.

Zweckmäßig ist das Segment NRₐR_{b} von einem sekundären Amin abgeleitet, wie Dioctadecylamin, Di-Kokosamin, di-hydriertes Talgamin und Methylbehenylamin. Amingemische, wie sie aus natürlichen Materialien erhältlich sind, eignen sich ebenfalls. Beispielsweise ist zu nennen ein sekundäres hydriertes Talg-Amin, wobei die Alkylgruppen von hydriertem Talgfett abgeleitet sind, und etwa 4 Gew.-% C₁₄, 31 Gew.-% C₁₆ und 59 Gew.-% C₁₈-Alkylgruppen aufweisen. Entsprechende tertiäre Amine der Formel (3) werden beispielsweise von der Firma Akzo Nobel unter der Bezeichnung Armeen^{®} M2HT oder Armeen^{®} M2C vertrieben.

Das tertiäre Amin-Edukt (3) kann aber auch so ausgebildet sein, dass die Reste Rₐ, R_{b} und R_{c} gleich oder verschiedene langkettige Alkylreste insbesondere geradkettige oder verzweigte Alkylgruppen mit 8 bis 40 Kohlenstoffatomen aufweisen.

Das tertiäre Amin-Edukt (3) kann aber auch so ausgebildet sein, dass die Reste Rₐ, R_{b} und R_{c} gleich oder verschiedene kurzkettige Alkylreste, insbesondere geradkettige oder verzweigte Alkylgruppen mit 1 bis 7 oder insbesondere 1 bis 4 Kohlenstoffatomen aufweisen.

Weitere nichtlimitierende Beispiele für geeignete Amine sind:
N,N-Dimethyl-N-(2-ethylhexyl)amin, N,N-Dimethyl-N-(2-propylheptyl)amin, Dodecyl-dimethylamin, Hexadecyldimethylamin, Oleyldimethylamin, Stearyldimethylamin, Hep-tadecyldimethylamin, Cocoyldimethylamin, Dicocoylmethylamin, Talgfettdimethylamin, Ditalgfettmethylamin, Tridodecylamin, Trihexadecylamin, Trioctadecylamin, Sojadimethylamin, Tris(2-ethylhexyl)amin, sowie Alamine 336 (Tri-n-octylamin).

Nichtlimitierende Beispiele für kurzkettige tertiäre Amine sind: Trimethylamin, Triethylamin, Tri-n-propylamin, Tri-n-butylamin, Tri-n-pentylamin, Tri-n-hexylamin, Tri-n-heptylamin, Ethyldimethylamin, Dimethylethylamin, n-Proplydimethylamin, Isopropyldimethylamin, n-Proplydiethylamin, Isopropyldiethylamin, n-Butyldimethylamin. n-Butyldiethylamin, . n-Butyldipropylamin.

Kurzkettige Triamine sind insbesondere auch dann zweckmäßig, wenn das Quaternierungsmittel (s. unten) einen oder mehrere Alkylreste R_{d} mit mehr als einem C-Atom oder einen oder mehrere aromatische Reste R_{d} trägt

### A3.2) Quaternisierbare, Polyether-substituierte Amin, enthaltend wenigstens eine quaternisierbare, insbesondere tertiäre, Aminogruppe;

Derartige Verbindungen sind z.B. beschrieben in der WO2013/064689 der Anmelderin, worauf hiermit ausdrücklich Bezug genommen wird.

Derartige substituierte Amine besitzen insbesondere wenigstens einen, insbesondere einen Polyether-Substituenten mit Monomereinheiten der allgemeinen Formel Ic

-[-CH(R₃)-CH(R₄)-O-]- (Ic)

worin
R₃ und R₄ gleich oder verschieden sind und für H, Alkyl, Alkylaryl oder Aryl stehen.

Das Polyether-substituierte Amin kann dabei ein zahlenmittleres Molekulargewicht im Bereich von 500 bis 5000, insbesondere 800 bis 3000 oder 900 bis 1500 aufweisen.

Die quaternisierbaren, Polyether-substituierten Amine sind insbesondere Stickstoffverbindung der allgemeinen Formel la-1 oder Ib-2 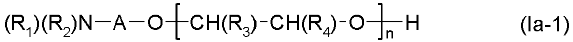 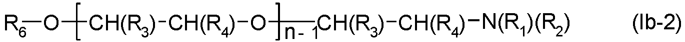 worin
R₁ und R₂ gleich oder verschieden sind und für Alkyl, Alkenyl, Hydroxyalkyl, Hydroxyalkenyl, Aminoalkyl oder Aminoalkenyl stehen, oder R₁ und R₂ zusammen für Alkylen, Oxyalkylen oder Aminoalkylen stehen;
R₃ und R₄ gleich oder verschieden sind und für H, Alkyl, Alkylaryl oder Aryl stehen;
R₆ für Alkyl, Alkenyl, ggf ein oder mehrfach ungesättigtes Cycloalkyl, Aryl, jeweils gegebenenfalls substituiert, wie z.B. mit wenigstens einem Hydroxylrest oder Alkylrest, oder unterbrochen durch wenigstens ein Heteroatom, steht;
A für einen geradkettigen oder verzweigten Alkylenrest steht, der gegebenenfalls durch ein oder mehrere Heteroatome, wie N, O und S, unterbrochen ist; und
n für einen ganzzahligen Wert von 1 bis 50 steht;

Insbesondere sind solche Stickstoffverbindungen der Formeln la-1 und Ib-2 zu nennen, worin
R₁ und R₂ gleich oder verschieden sind und für C₁-C₆-Alkyl, Hydroxy-C₁-C₆-alkyl, Hydroxy-C₁-C₆-alkenyl, oder Amino-C₁-C₆-alkyl stehen, oder R₁ und R₂ zusammen eine C₂-C₆-Alkylen-, C₂-C₆-Oxyalkylen- oder C₂-C₆-Aminoalkylen-Rest bilden;
R₃ und R₄ gleich oder verschieden sind und für H, C₁-C₆-Alkyl oder Phenyl stehen;
R₆ für C₁-C₂₀-Alkyl, wie z.B. C₁₀-C₂₀- ,C₁₁-C₂₀- oder C₁₂-C₂₀-Alkyl oder Aryl oder Alkylaryl, wobei Alkyl insbesondere für C₁-C₂₀- bedeutet, steht;
A für einen geradkettigen oder verzweigten C₂-C₆-Alkylenrest steht, der gegebenenfalls durch ein oder mehrere Heteroatome, wie N, O und S, unterbrochen ist; und
n für einen ganzzahligen Wert von 1 bis 30.

Weiterhin besonders zu nennen sind Umsetzungsprodukte von N,N-Dimethylethanolamin und Propylenoxid wie beschrieben in Synthesebeispiel 1 der WO 2013/064689. Diese Umsetzung kann auch unkatalysiert oder mit einem Amin (beispielsweise Imidazol) als Katalysator durchgeführt werden, wie z.B. beschrieben in M. lonescu, Chemistry and Technology of Polyols for Polyurethanes, 2005, ISBN 978-85957-501-7.

Stickstoffverbindungen der allgemeinen Formel la-1, sind herstellbar wobei man ein Aminoalkanol der allgemeinen Formel II

(R₁)(R₂)N-A-OH (II)

worin
R₁, R₂ und A die oben angegebenen Bedeutungen besitzen,
mit einem Epoxid der allgemeinen Formel III 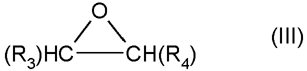
worin
R₃ und R₄ die oben angegebenen Bedeutungen besitzen,
alkoxyliert, wobei man ein alkoxyliertes Amin der Formel 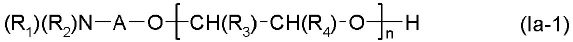
erhält, worin R₁ bis R₄, A und n die oben angegebenen Bedeutungen besitzen.

Stickstoffverbindungen der allgemeinen Formel la-2, sind herstellbar wobei man wobei man
einen Alkohol der allgemeinen Formel V

   R₆-OH (V)
worin
R₆ die oben angegebenen Bedeutungen besitzt, mit einem Epoxid der allgemeinen Formel III 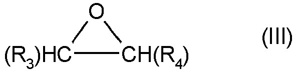
worin
R₃ und R₄ die oben angegebenen Bedeutungen besitzen, alkoxyliert, wobei man einen Polyether der Formel Ib-1 erhält; 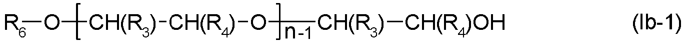
erhält; worin R₃, R₄ und R₆, A und n die oben angegebenen Bedeutungen besitzen und
b) anschließend den so erhaltenen Polyether der Formel Ib-1 mit einem Amin der allgemeinen Formel

   NH(R₁)(R₂) (VII)
worin R₁ und R₂ die oben angegebenen Bedeutungen besitzen,
aminiert, wobei man ein Amin der Formel Ib-2 erhält.

Ausgangsverbindungen zur Herstellung obiger Polyether-substituierter, quaternisierbarer Stickstoffverbindungen sind somit:
1) Alkohole
   wie z.B. der allgemeinen Formel V

      R₆-OH (V)
   worin R₆ für Alkyl, Alkenyl, ggf ein oder mehrfach ungesättigtes Cycloalkyl, Aryl, jeweils gegebenenfalls substituiert, wie z.B. mit wenigstens einem Hydroxylrest oder Alkylrest, oder unterbrochen durch wenigstens ein Heteroatom, steht;
   und
2) Aminoalkanole
   wie z.B. der allgemeinen Formel II

      (R₁)(R₂)N-A-O_{H} (II)
   worin
   R₁ und R₂ gleich oder verschieden sind und für Alkyl, Alkenyl, Hydroxyalkyl, Hydroxyalkenyl, Aminoalkyl oder Aminoalkenyl stehen, oder R₁ und R₂ zusammen für Alkylen, Oxyalkylen oder Aminoalkylen stehen; und
   A für einen geradkettigen oder verzweigten Alkylen- oder Alkenylenrest steht, der gegebenenfalls durch ein oder mehrere Heteroatome, wie N, O und S, unterbrochen ist;

Als weitere geeignete Gruppe von quaternisierbaren Aminoalkoholen sind zu nennen Verbindungen, ausgewählt unter Hydroxyalkyl-substituierten Mono- oder Polyaminen mit wenigstens einer quaternisierbaren, primären, sekundären oder tertiären Aminogruppe und wenigstens einer Hydroxylgruppe, welche mit einem Polyetherrest verknüpfbar ist.

Insbesondere ausgewählt sind die quaternisierbaren Stickstoff-Verbindung unter Hydroxyalkyl-substituierten primären, sekundären, und insbesondere tertiären Monoaminen und Hydroxyalkyl-substituierten primären, sekundären und insbesondere tertiären Diaminen.

Beispiele für geeignete "hydroxyalkyl-substituierte Mono- oder Polyamine" sind solche, die mit wenigstens einem, wie z.B. 1, 2, 3, 4, 5 oder 6, Hydroxyalkyl-Substituierten ausgestattet sind.

Ala Beispiele für "Hydroxyalkyl-substituierte Monoamine" können genannt werden: N-Hydroxyalkyl-monoamine, N,N-Dihydroxyalkyl-monoamine und N,N,N-Trihydroxyalkyl-monoamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl.

Beispielsweise können folgende "Hydroxyalkyl-substituierte Polyamine" und insbesondere "Hydroxyalkyl-substituierte Diamine" genannt werden: (N-Hydroxyalkyl)-alkylendiamine, N,N-Dihydroxyalkyl-alkylendiamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl; Alkylen steht dabei insbesondere für Ethylen, Propylen oder Butylen.

Folgende quaternisierbare Stickstoffverbindungen seien insbesondere genannt:

| **NAME** | **FORMEL** |
|---|---|
| ***Alkohole mit primärem und sekundärem Amin*** | |
| Ethanolamin | 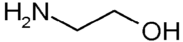 |
| 3-Hydroxy-1-propylamin | 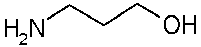 |
| Diethanolamin | 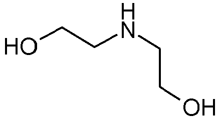 |
| Diisopropanolamin | 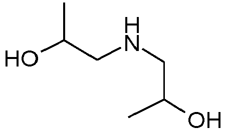 |
| N-(2-Hydroxyethyl)ethylendiamin | 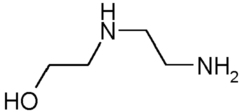 |

| ***Alkohole mit tertiärem Amin*** | |
|---|---|
| Triethanolamin, (2,2',2"-Nitrilotriethanol) | 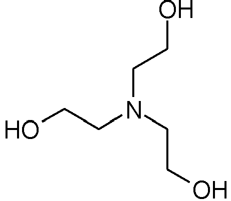 |
| 1-(3-Hydroxypropyl)imidazol | 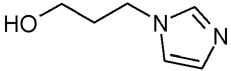 |
| Tris(hydroxymethyl)amin | 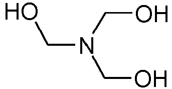 |
| 3-Dimethylamino-1-propanol | 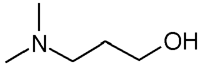 |
| 3-Diethylamino-1-propanol | 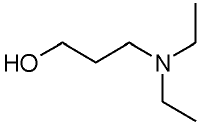 |
| 2-Dimethylamino-1-ethanol | 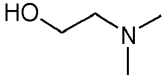 |
| 4-Diethylamino-1-butanol | 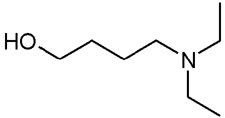 |

Zur Herstellung der Polyethersubstituierten quaternisierbaren Verbindungen (la-1 und Ib-1) kann wie folgt verfahren werden:

### a1) Ausgehend von Aminoalkoholen der Formel II:

Die Aminoalkohole der allgemeinen Formel II können in prinzipiell bekannter Art und Weise alkoxyliert werden, wobei alkoxylierte Amine der allgemeinen Formel la-1 erhalten werden.

Die Durchführung von Alkoxylierungen ist dem Fachmann prinzipiell bekannt. Es ist dem Fachmann ebenfalls bekannt, dass man durch die Reaktionsbedingungen, insbesondere die Wahl des Katalysators, die Molekulargewichtsverteilung der Alkoxylate beeinflussen kann.

Zur Alkoxylierung werden C₂- C₁₆-Alkylenoxide eingesetzt, beispielsweise Ethylenoxid, Propylenoxid, oder Butylenoxid. Bevorzugt sind jeweils die 1,2-Alkylenoxide.

Bei der Alkoxylierung kann es sich um eine basenkatalysierte Alkoxylierung handeln. Dazu können die Aminoalkohole (II) in einem Druckreaktor mit Alkalimetallhydroxiden, bevorzugt Kaliumhydroxid oder mit Alkalialkoholaten wie beispielsweise Natriummethylat versetzt werden. Durch verminderten Druck (bspw. <100 mbar) und/oder Erhöhung der Temperatur (30 bis 150°C) kann noch in der Mischung vorhandenes Wasser abgezogen werden. Der Alkohol liegt danach als das entsprechende Alkoholat vor. Anschließend wird mit Inertgas (z.B. Stickstoff) inertisiert und das(die) Alkylenoxid(e) bei Temperaturen von 60 bis 180°C bis zu einem Druck von max. 10 bar schrittweise zugegeben. Am Ende der Reaktion kann der Katalysator durch Zugabe von Säure (z.B. Essigsäure oder Phosphorsäure) neutralisiert und kann bei Bedarf abfiltriert werden. Der basische Katalysator kann aber auch durch Zugabe handelsüblicher Mg-Silikate neutralisiert werden, welche anschließend abfiltriert werden. Optional kann die Alkoxylierung auch in Gegenwart eines Lösungsmittels durchgeführt werden. Dies kann z.B. Toluol, Xylol, Dimethylformamid oder Ethylencarbonat sein.

Die Alkoxylierung der Aminoalkohole kann aber auch mittels anderer Methoden vorgenommen werden, beispielsweise durch säurekatalysierte Alkoxylierung. Weiterhin können beispielsweise Doppelhydroxidtone wie in DE 43 25 237 A1 beschrieben eingesetzt werden, oder es können Doppelmetallcyanid-Katalysatoren (DMC-Katalysatoren) verwendet werden. Geeignete DMC-Katalysatoren sind beispielsweise in der DE 102 43 361 A1, insbesondere den Abschnitten [0029] bis [0041] sowie der darin zitierten Literatur offenbart. Beispielsweise können Katalysatoren vom Zn-Co-Typ eingesetzt werden. Zur Durchführung der Reaktion kann der Aminoalkohol mit dem Katalysator versetzt, die Mischung wie oben beschrieben entwässert und mit den Alkylenoxiden wie beschrieben umgesetzt werden. Es werden üblicherweise nicht mehr als 1000 ppm Katalysator bezüglich der Mischung eingesetzt, und der Katalysator kann aufgrund dieser geringen Menge im Produkt verbleiben. Die Katalysatormenge kann in der Regel geringer sein als 1000 ppm, beispielsweise 250 ppm und weniger.

Die Alkoxylierung kann alternativ auch durch Reaktion der Verbindungen (IV) und (V) mit cyclischen Carbonaten wie beispielsweise Ethylencarbonat vorgenommen werden.

### a2) Ausgehend von Alkanolen der Formel V:

Wie unter dem vorherigen Abschnitt a1) für Aminoalkohole (II) beschrieben können analog auch Alkanole R₆OH in prinzipiell bekannter Weise zu Polyethern (Ib-1) alkoxyliert werden. Die so erhaltenen Polyether können anschließend durch reduktive Aminierung mit Ammoniak, primären Aminen oder sekundären Aminen (VII) nach üblichen Methoden in kontinuierlichen oder diskontinuierlichen Verfahren unter Verwendung hierfür üblicher Hydrier- bzw. Aminierungskatalysatoren wie beispielsweise solchen, die katalytisch aktive Bestandteile auf Basis der Elemente Ni, Co, Cu, Fe, Pd, Pt, Ru, Rh, Re, Al, Si, Ti, Zr, Nb, Mg, Zn, Ag, Au, Os, Ir, Cr, Mo,, W oder Kombinationen dieser Elemente untereinander enthalten, in üblichen Mengen zu den entsprechenden Polyetheraminen (Ib-2) umgesetzt werden. Die Umsetzung kann ohne Lösungsmittel oder bei hohen Polyetherviskositäten in Gegenwart eines Lösungsmittels, vorzugsweise in Gegenwart verzweigter Aliphaten wie beispielsweise Isododekan durchgeführt werden. Die Aminkomponente (VII) wird dabei im Allgemeinen im Überschuss, z.B. im 2- bis 100-fachen Überschuss, vorzugsweise 10- bis 80-fachem Überschuss, eingesetzt. Die Reaktion wird bei Drücken von 10 bis 600 bar durchgeführt über einen Zeitraum von 10 Minuten bis 10 Stunden. Nach dem Abkühlen trennt man den Katalysator durch Filtrieren ab, verdampft überschüssige Aminkomponente (VII) und destilliert das Reaktionswasser azeotrop oder unter einem leichten Stickstoffstrom ab.

Sollte das resultierende Polyetheramin (Ib-2) primäre oder sekundäre Aminfunktionalitäten aufweisen (R₁ und/oder R₂ gleich H), kann dieses nachfolgend in ein Polyetheramin mit tertiärer Aminfunktion überführt werden (R₁ und R₂ ungleich H). Die Alkylierung kann in prinzipiell bekannter Weise durch Umsetzung mit Alkylierungsmitteln erfolgen. Geeignet sind prinzipiell alle Alkylierungsmittel wie beispielsweise Alkylhalogenide, Alkylarylhalogenide, Dialkylsulfate, Alkylenoxide ggf. in Kombination mit Säure; aliphatische oder aromatische Carbonsäureester, wie insbesondere Dialkylcarboxylate; Alkanoate; cyclische nichtaromatische oder aromatische Carbonsäureester; Dialkylcarbonate; und Mischungen davon. Die Umsetzungen zum tertiären Polyetheramin können auch durch reduktive Aminierung durch Umsetzung mit einer Carbonylverbindung wie beispielsweise Formaldehyd in Gegenwart eines Reduktionsmittels stattfinden. Geeignete Reduktionsmittels sind Ameisensäure oder Wasserstoff in Gegenwart eines geeigneten heterogenen oder homogenen Hydrierkatalysators. Die Reaktionen können ohne Lösungsmittel oder in Gegenwart von Lösungsmitteln durchgeführt werden. Geeignete Lösungsmittel sind beispielsweise H₂O, Alkanole wie Methanol oder Ethanol, oder 2-Ethylhexanol, aromatische Lösungsmitteln wie Toluol, Xylol oder Lösungsmittelgemischen der Solvesso-Serie, oder aliphatische Lösungsmittel, insbesondere Gemische verzweigter aliphatischer Lösungsmittel. Die Reaktionen werden bei Temperaturen von 10°C bis 300°C bei Drücken von 1 bis 600 bar über einen Zeitraum von 10 Minuten bis 10 h durchgeführt. Das Reduktionsmittel wird dabei mindestens stöchiometrisch, vorzugsweise im Überschuss eingesetzt, insbesondere im 2- bis 10-fachen Überschuss.

Das so gebildete Reaktionsprodukt (Polyetheramin Ib-1 oder Ib-2) kann theoretisch weiter aufgereinigt oder das Lösungsmittel entfernt werden. Gewöhnlich ist dies aber nicht zwingend notwendig, so dass das Reaktionsprodukt ohne weitere Aufreinigung in den nächsten Syntheseschritt, der Quaternisierung, überführt werden kann.

### A3.3) Polyalkensubstituierte Amine mit wenigstens einer tertiären, quaternisierbaren Stickstoffgruppe

Weiterhin als quaternisierbare Stickstoffverbindungen geeignet sind polyalkensubstituierte Amine mit wenigstens einer tertiären Stickstoffgruppe. Diese Verbindungsgruppe ist ebenfalls bekannt und z.B. beschrieben in der WO 2008/060888 oder US 2008/0113890 und dem darin genannten weiteren Stand der Technik, worauf hiermit ausdrücklich Bezug genommen wird.

Derartige Polyalken-substituierten Amine mit mindestens einer tertiären Aminogruppe sind aus einem Olefin-Polymer und einem Amin wie Ammoniak, Monoaminen, Polyaminen oder Gemischen davon, ableitbar. Sie können durch eine Vielzahl von Verfahren hergestellt werden, wie z.B. die folgenden beispielhaft aufgeführten Verfahren:
Ein Verfahren zur Herstellung eines Polyalken-substituierten Amins umfasst die Umsetzung eines halogenierten Olefin-Polymers mit einem Amin, wie in den US-Patenten 3,275,554, 3,438,757, 3,454,555, 3,565,804, 3,755,433 und 3,822,289 beschrieben.

Ein weiteres Verfahren zur Herstellung eines Polyalken-substituierten Amins umfasst die Umsetzung eines hydroformylierten Olefins mit einem Polyamin und Hydrierung des Reaktionsprodukts, wie in US 5,567,845 und 5,496,383 beschrieben

Ein weiteres Verfahren zur Herstellung eines Polyalken-substituierten Amins umfasst die Umwandlung eines Polyalkens mit Hilfe eines herkömmlichen Epoxidierungsreagenz mit oder ohne Katalysator, in das entsprechende Epoxid und die Umsetzung des Epoxids zum Polyalken-substituierten Amin durch Umsetzung mit Ammoniak oder einem Amin unter den Bedingungen der reduktiven Animation, wie in US 5,350,429 beschrieben.

Ein weiteres Verfahren zur Herstellung von Polyalken-substituierten Amins umfasst die Hydrierung eines ß-Aminonitrils, das durch Umsetzung eines Amins mit einem Nitril hergestellt wurde, wie in US 5,492,641, beschrieben.

Ein weiteres Verfahren zur Herstellung eines Polyalken-substituierten Amins umfasst Hydroformylierung eines Polybutens oder Polyisobutylens mit einem Katalysator, wie Rhodium oder Kobalt, in Gegenwart von CO und und Wasserstoff bei erhöhten Drücken und Temperaturen, wie in US 4,832,702 beschrieben.

In einer Ausführungsform der Erfindung sind die zur Herstellung verwendeten Polyalkene von Olefin-Polymeren abgeleitet. Die Olefin-Polymere können Homopolymere und Copolymere von polymerisierbaren Olefinmonomeren mit 2 bis etwa 16 Kohlenstoffatomen, 2 bis etwa 6 Kohlenstoffatomen oder 2 bis etwa 4 Kohlenstoffatomen umfassen.

Interpolymere sind solche, in denen zwei oder mehr Olefinmonomere nach bekannten herkömmlichen Methoden interpolymerisiert werden, wobei Polyalkene anfallen mit Einheiten innerhalb ihrer Struktur, die von jedem der der zwei oder mehr Olefinmonomeren abgeleitet sind.

Somit umfassen "Interpolymere" Copolymere, Terpolymere und Tetrapolymere.

"Polyalkene", von denen die Polyalken-substituierten Amine abgeleitet sind, werden herkömmlich häufig auch als "Polyolefine" bezeichnet.

Die Olefinmonomeren, von denen die Olefinpolymere abgeleitet sind, sind polymerisierbare Olefinmonomere, die eine oder mehrere ethylenisch ungesättigte Gruppen (d.h. >C=C< ) aufweisen., das heißt, sie sind monoolefinische Monomeren, wie Ethylen, Propylen , 1-Buten, Isobuten (2-methyl-1-buten), 1-Octen oder polyolefinische Monomere (gewöhnlich diolefinische Monomere) wie 1,3-Butadien und Isopren.

Die Olefinmonomere sind gewöhnlich polymerisierbare endständige Olefine, das heißt, Olefine, die in ihrer Struktur die Gruppe > C=CH₂ aufweisen. Es können aber auch polymerisierbare innenständige Olefinmonomere eingesetzt werden, die durch Gruppen der Formel >C-C=C-C< gekennzeichnet sind

Spezifische Beispiele für endständige und innenständige Olefinmonomere, die verwendet werden können, um die Polyalkene nach herkömmlichen Methoden herzustellen, sind: Ethylen, Propylen, die Butene (Butylen), insbesondere 1-Buten, 2-Buten und Isobutylen, 1-Penten, 1-Hexen, 1-Hepten, 1-Octen, 1-Nonen, 1-Decen, 2-Penten, Propylen-Tetramer, Diisobutylen, Isobutylen-Trimer, 1,2-Butadien, 1,3-Butadien , 1,2-Pentadien, 1,3-Pentadien, 1,4-Pentadien, Isopren, 1,5-Hexadien, 2-Methyl-5-propyl-1-hexen, 3-Penten, 4-Octen und 3, 3-Dimethyl-1-penten.

In einer anderen Ausführungsform ist das Olefin-Polymer durch Polymerisation eines C₄-Raffineriestroms mit einem Butengehalt von etwa 35 bis etwa 75 Gewichtsprozent und einem Isobutengehalt von etwa 30 bis etwa 60 Gewichtsprozent in Gegenwart einer Lewis-Säure Katalysators wie Aluminiumtrichlorid oder Bortrifluorid herstellbar. Diese Polybutene enthalten üblicherweise vorwiegend (mehr als etwa 80% der gesamten Wiederholungseinheiten) Isobuten-Wiederholungseinheiten des Typs (-CH₂-C(CH₃)₂-)

In einer weiteren Ausführungsform ist der Polyalken-Substituent des Polyalken-substituierte Amin von einem Polyisobutylen abgeleitet.

In einer anderen Ausführungsform umfassen die Amine, die verwendet werden können, um das Polyalken-substituierte Amin zu bilden, Ammoniak, Monoaminen, Polyaminen oder Gemischen davon, einschließlich Mischungen von verschiedenen Monoamine, Mischungen verschiedener Polyamine und Mischungen von Monomaminen und Polyaminen (die Diamine). Die Amine umfassen aliphatische, aromatische, heterocyclische und carbocyclische Amine. Monoamine und Polyamine sind durch die Anwesenheit in ihrer Struktur von mindestens einer HN <-Gruppe gekennzeichnet. Die Amine können aliphatisch, cycloaliphatisch, aromatisch oder heterocyclisch sein.

Die Monoamine sind im allgemeinen durch eine Kohlenwasserstoffgruppe mit 1 bis 50 Kohlenstoffatomen substituiert. Diese Kohlenwasserstoffgruppen können insbesondere aliphatisch und frei von acetylenisch ungesättigten Gruppen sein und 1 bis etwa 30 Kohlenstoffatome aufweisen. Insbesondere sind zu nennen gesättigte aliphatische Kohlenwasserstoffreste mit 1 bis 30 Kohlenstoffatomen.

In einer weiteren Ausführungsform können die Monoamine die Formel HNR₁R₂ aufweisen, wobei R₁ eine Kohlenwasserstoffgruppe mit bis zu 30 Kohlenstoffatomen und R₂ Wasserstoff oder eine Hydrocarbylgruppe von bis zu etwa 30 Kohlenstoffatomen ist. Beispiele für geeignete Monoamine sind Methylamin, Ethylamin, Diethylamin, 2-Ethylhexylamin, Di-(2-ethylhexyl)-amin, n-Butylamin, Di-n-butylamin, Allylamin, Isobutylamin, Kokosamin, Stearylamin, Laurylamin, Methyllaurylamine und Oleylamin.

Aromatische Monoamine sind jene Monoamine, bei denen ein Kohlenstoffatom der aromatischen Ringstruktur direkt an das Aminstickstoffatom gebunden ist. Der aromatische Ring wird gewöhnlich ein einkerniger aromatischer Ring (d.h. von Benzol abgeleitet) sein, kann aber kondensierte aromatische Ringe aufweisen, und insbesondere solche von Naphthalin abgeleitet sein. Beispiele für aromatische Monoamine sind Anilin, Di (para-methylphenyl) amin, Naphthylamin, N-(n-Butyl) anilin. Beispiele für aliphatisch-substituierte, cycloaliphatisch-substituierte und heterocyclisch-substituierte aromatische Monoamine sind: para-Dodecylanilin, Cyclohexyl-substituiertes Naphthylamin und Thienyl-substituiertes Anilin.

Hydroxyamine sind ebenfalls geeigneter Monoamine. Derartige Verbindungen sind die Hydroxyhydrocarbyl-substituierten Analoga der vorstehend erwähnten Monoamine.

In einer Ausführungsform kann die Hydroxy-Monoamine der Formel HNR₃R₄, wobei R₃ eine hydroxysubstituierte Alkylgruppe mit bis zu etwa 30 Kohlenstoffatomen darstellt, und in einer Ausführungsform bis zu etwa 10 Kohlenstoffatome aufweist; und R₄ eine hydroxysubstituierte Alkylgruppe mit bis zu etwa 30 Kohlenstoffatomen, Wasserstoff oder eine Hydrocarbylgruppe mit bis zu etwa 10 Kohlenstoffatomen darstellt. Beispiele für Hydroxy-substituierte Monoamine umfassen: Ethanolamin, Di-3-Propanolamin, 4-Hydroxybutylamin, Diethanolamin und N-Methyl-2-hydroxypropylamin.

In einer anderen Ausführungsform kann das Amin der Polyalken-substituierten Amine ein Polyamin sein. Das Polyamin kann aliphatisch, cycloaliphatisch, heterocyclisch oder aromatisch sein. Beispiele für die Polyamine umfassen: Alkylenpolyamine, Hydroxygruppen-enthaltende Polyamine, Arylpolyamine und heterocyclische Polyamine.

Die Alkylenpolyamine umfassen solche der folgenden Formel

HN(R⁵)-(Alkylen-N(R⁵))ₙ-(R⁵)

worin n im Bereich von 1 bis etwa 10 und z.B. im Bereich von 2 bis etwa 7, oder von 2 bis etwa 5 liegt, und die "Alkylen"-Gruppe 1 bis etwa 10 Kohlenstoffatome , wie z.B. 2 bis etwa 6, oder 2 bis etwa 4 Kohlenstoffatome aufweist;
die Reste R⁵ unabhängig voneinander für Wasserstoff, eine aliphatische Gruppe, eine Hydroxyl- oder Amin-substituierte aliphatische Gruppe von jeweils bis zu etwa 30 Kohlenstoffatome stehen. Typischerweise bedeutet R⁵ H oder Niedrigalkyl (eine Alkylgruppe mit 1 bis etwa 5 Kohlenstoffatome), insbesondere H. Solche Alkylenpolyamine umfassen: Methylenpolyamine, Ethylenpolyamine, Butylenpolyamine, Propylenpolyamine, Pentylenpolyamine, Hexylenpolyamine und Heptylenpolyamine. Die höheren Homologen solcher Amine und verwandte Aminoalkyl-substituierte Piperazine sind ebenfalls eingeschlossen.

Spezifische Alkylenpolyamine zur Herstellung der Polyalken-substituierte Amine sind die folgenden: Ethylendiamin, Diethylentriamin, Triethylentetramin, Tetraethylenpentamin, Propylendiamin, 3-Dimethylaminopropylamin, Trimethylendiamin, Hexamethylendiamin, Decamethylendiamin, Octamethylendiamin, Di (Heptamethylen) triamin, Tripropylentetramin, Pentaethylenhexamin, Di (trimethylen-triamin), N-(2-Aminoethyl) piperazin und 1,4-Bis-(2-Aminoethyl) piperazin.

Ethylenpolyamine, wie die vorstehend genannten, sind besonders aus Gründen der Kosten und Effektivität geeignet. Solche Polyamine sind im Detail im Kapitel "Diamine und höhere Amine" in Encyclopedia of Chemical Technology, zweite Ausgabe, Kirk - Othemer, Band 7, Seiten 27-39, Interscience Publishers, Abteilung von John Wiley & Sons, 1965 beschrieben. Solche Verbindungen werden am bequemsten durch die Umsetzung eines Alkylenchlorids mit Ammoniak oder durch Umsetzung eines Ethylenimins mit einem ringöffnenden Reagenz wie Ammoniak, hergestellt. Diese Reaktionen führen zur Herstellung von komplexen Gemischen von Alkylenpolyaminen, einschließlich cyclischer Kondensationsprodukte wie Piperazinen.

Andere geeignete Typen von Polyamingemischen sind die durch Stripping der vorstehend beschriebenen Polyamingemische als Rückstand gebildeten und häufig als "Polyamin-Sumpf" bezeichneten Produkte. Im allgemeinen Alkylenpolyamin-Sumpfprodukte solche, die weniger als zwei, gewöhnlich weniger als 1 Gew.% Material enthalten das unter etwa 200 °C siedet. Eine typisches Beispiel für solche Ethylenpolyaminsümpfe sind mit "E-100" bezeichneten Produkte von Dow Chemical Company in Freeport, Texas. Diese Alkylenpolyamin-Sümpfe umfassen cyclische Kondensationsprodukte wie Piperazin und höhere Analoga von Diethylentriamin, Triethylenetetriamine und dergleichen.

Hydroxylgruppen-enthaltende Polyamine umfassen: Hydroxyalkylalkylenpolyamine mit einem oder mehreren Hydroxyalkyl-Substituenten an den Stickstoffatomen. Solche Polyamine können durch Umsetzen der vorstehend beschriebenen Alkylenpolyamine mit einem oder mehreren Alkylenoxiden (z. B. Ethylenoxid, Propylenoxid und Butylenoxid) hergestellt werden. Ähnliche Alkylenoxid-Alkanolamin-Reaktionsprodukte können auch beispielsweise die Produkte der Umsetzung von primären, sekundären oder tertiären Alkanolamine mit Ethylen, Propylen oder höheren Epoxiden in einem MolVerhältnis von 1:1 bis 1:2 sein. Reaktantenverhältnisse und Temperaturen zur Durchführung solcher Umsetzungen sind dem Fachmann bekannt.

In einer anderen Ausführungsform kann das Hydroxyalkyl-substituierte Alkylenpolyamin, eine Verbindung sein in der die Hydroxyalkylgruppe eine Hydroxy-Niedrigalkylgruppe ist, d.h. weniger als acht Kohlenstoffatomen aufweist. Beispiele für solche Hydroxyalkyl-substituierten Polyamine umfassen N-(2-Hydroxyethyl) ethylendiamin (auch bekannt als 2 - (2-Aminoethylamino) ethanol), N, N-Bis (2-hydroxyethyl) ethylendiamin, 1 - (2 - Hydroxyethyl) piperazin, monohydroxypropyl substituiertes Diethylentriamin, Dihydroxypropyl-substituiertes Tetraethylenpentamin und N-(3-Hydroxybutyl) tetramethylendiamin.

Arylpolyamine sind Analoga zu den oben genannten aromatischen Monoamine. Beispiele für Arylpolyamine umfassen: N, N'-Di-n-butyl-para-phenylendiamin und Bis-(para-aminophenyl) methan.

Heterocyclische Mono-und Polyamine können, umfassen: Aziridine, Azetidine, Azolidine, Pyridine, Pyrrole, Indole, Piperidine, Imidazole, Piperazine, Isoindole, Purine, Morpholine, Thiomorpholine, N-Aminoalkylmorpholine, N-aminoalkylthiomorpholines, N-Aminoalkylpiperazine, N, N'-Diamino-Alkylpiperazine, Azepine, Azocine, Azonine, Azecine und Tetra-, Di-und Perhydroderivate von jeder der vorstehenden Verbindungen und Gemische von zwei oder mehreren dieser heterocyclischen Amine. Typische heterocyclische Amine sind gesättigte 5 - und 6-gliedrige heterocyclische Amine, die nur Stickstoff, Sauerstoff und / oder Schwefel im Heterocyclus aufweisen, insbesondere Piperidine, Piperazine, Thiomorpholine, Morpholine, Pyrrolidine und dergleichen. Piperidin, Aminoalkyl-substituierte Piperidine, Piperazin, Aminoalkyl-substituierte Piperazine, Morpholin, Aminoalkyl-substituierte Morpholine, Pyrrolidin und Aminoalkyl-substituierte Pyrrolidine sind besonders bevorzugt. Gewöhnlich sind die Aminoalkylsubstituenten an einem Stickstoffatom gebunden , das Teil des Heterocyclus ist

Spezielle Beispiele solcher heterocyclischer Amine umfassen N-Aminopropylmorpholin, N-Aminoethylpiperazin und N, N'-Diaminoethylpiperazin. Hydroxy-heterocyclische Polyamine sind auch geeignet, Beispiele umfassen: N-(2-Hydroxyethyl) cyclohexylamin, 3-Hydroxycyclopentylamin, parahydroxy-anilin und N-Hydroxyethylpiperazin.

Beispiele von Polyalken-substituierten Amine sind die folgenden: Poly (propylen) amin, Poly (buten)-amin, N, N-dimethylpolyisobutyleneamine; polybutenemorpholine N-, N-Poly (buten)-ethylendiamin, N-Poly (propylen) trimethylendiamin, N-Poly (buten), Diethylentriamin, N ', N'-Poly (buten) Tetraethylenpentamin und N, N-Dimethyl-N'poly (propylen) -1,3-propylendiamin.

Das zahlenmittlere Molekulargewichts solcher Polyalken-substituierten Amine beträgt etwa 500 bis etwa 5000, wie z.B. 1000 bis etwa 1500 oder etwa 500 bis etwa 3000.

Jedes der oben genannten Polyalken-substituierten Amine, die sekundäre oder primäre Amine sind, können zu tertiären Aminen mit Alkylierungsmitteln alkyliert werden, welche auch als Quaternisierungsmittel bekannt sind, wie Dialkylsulfate, Alkylhalogenide, Hydrocarbyl-substituierte Carbonate; Hydrocarbylepoxiden in Kombination mit einer Säure und Mischungen davon. Wenn bestimmte Quaternisierungsmittel, wie Alkylhalogenide oder Dialkylsulfate verwendet werden, so kann es erforderlich sein, eine Base oder basische Mittel, wie Natriumcarbonat oder Natriumhydroxid, bereitzustellen, um die freie tertiäre Aminform zu ergeben. Primäre Amine erfordern zwei Äquivalenten Alkylierungsmittel und zwei Äquivalente Base, um ein tertiäres Amin zu erhalten. In einer anderen Ausführungsform kann die Alkylierung von primären Aminen häufig in vier aufeinanderfolgenden Schritten durchgeführt werden, zunächst eine Behandlung mit dem Alkylierungsmittel und zweiten Behandlung mit einer Base und dann Wiederholen der beiden Schritte. In einer anderen Ausführungsform wird die Alkylierung eines primären Amins in einem Schritt erfolgen, beispielsweise unter Verwendung von zwei Mol Alkylhalogenid in Gegenwart eines Überschusses von heterogenen Base, wie Natriumcarbonat. Das Polyamin kann in an sich bekannter Weise erschöpfend oder partiell alkyliert sein.

In einer anderen Ausführungsform kann die Alkylierung primärer Amine und sekundärer Amine zu tertiären Aminen mit Epoxiden erfolgen. Anders als mit den Alkylhalogeniden, ist bei Verwendung eines Epoxids keine Behandlung mit Base erforderlich, um das freie Amin zu erhalten. Typischerweise wird bei Alkylierung von Aminen mit Epoxiden mindestens ein Mol Epoxid für jedes Wasserstoffatom am Amin eingesetzt. Bei der Alkylierung zum tertiären Amin mit einem Epoxid sind weder zusätzliche Säure noch Base erforderlich.

Weiterhin besonders bevorzugt ist Polyisobutendimethylamin erhältlich durch Hydroformylierung von Polyisobuten (Mn 1000) und anschließende reduktive Aminierung mit Dimethylamin, siehe Example B der WO 2008/060888.

### A3.4) Reaktionsprodukte eines hydrocarbylsubstituierten Acylierungsmittels und einer Verbindung enthaltend ein Stickstoff- oder Sauerstoffatom und zusätzlich enthaltend wenigstens eine quaternisierbare Aminogruppe

Derartige Verbindungen sind z.B. beschrieben in der WO2013/000997 der Anmelderin, worauf hiermit ausdrücklich Bezug genommen wird.

Als geeignete Hydrocarbyl-substituierte Polycarbonsäure-Verbindungen, bzw. hydrocarbylsubstituierten Acylierungsmittels sind zu nennen:
Die eingesetzten Polycarbonsäure-Verbindungen sind aliphatische zwei- oder mehrwertige (wie z.B. 3- oder 4-wertig), insbesondere von Di-, Tri- oder Tetracarbonsäuren, sowie Analoga davon, wie Anhydride oder Niedrigalkylester (teilweise oder vollständig verestert), und gegebenenfalls durch einen oder mehrere (wie z.B. 2 oder 3), insbesondere einem langkettigen Alkylrest und/oder einem hochmolekularen Hydrocarbylrest, insbesondere einem Polyalkylenrest substituiert. Beispiele sind C₃ -C₁₀ Polycarbonsäuren, wie die Dicarbonsäuren Malonsäure, Bernsteinsäure, Glutarsäure, Adipinsäure, Pimelinsäure, Suberinsäure, Azelainsäure und Sebacinsäure, und deren verzweigte Analoga; sowie die Tricarbonsäure Citronensäure; sowie Anhydride oder Niedrigalkylester davon von. Die Polycarbonsäure-Verbindungen können auch aus den entsprechenden einfach ungesättigten Säuren und Addition wenigstens eines langkettigen Alkylrests und/oder hochmolekularen Hydrocarbylrests erzeugt werden. Beispiele geeigneter einfach ungesättigter Säuren sind Fumarsäure, Maleinsäure, Itaconsäure.

Der hydrophobe "langkettige" oder "hochmolekulare" Hydrocarbylrest, welcher für die ausreichende Löslichkeit des quaternisierten Produkts im Kraftstoff sorgt, hat ein zahlengemitteltes Molekulargewicht (Mₙ) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500. Als typische hydrophobe Hydrocarbylreste sind zu nennen Polypropenyl-, Polybutenyl- und Polyisobutenylreste, z.B. mit einem zahlenmittleren Molekulargewicht Mₙ von 3.500 bis 5.000, 350 bis 3.000, 500 bis 2.500, 700 bis 2.500 und 800 bis 1.500.

Geeignete Hydrocarbyl substituierte Verbindungen sind z.B. beschrieben in der DE 43 19 672 und der WO2008/138836.

Geeignete Hydrocarbyl-substituierte Polycarbonsäure-Verbindungen umfassen auch polymere, insbesondere dimere Formen solcher Hydrocarbyl-substituierten Polycarbonsäure-Verbindungen. Dimere Formen enthalten z.B. zwei Säureanhydridgruppen welche unabhängig voneinander im erfindungsgemäßen Herstellungsverfahren mit der quaternisierbaren Stickstoffverbindung umgesetzt werden können.

Die mit obiger Polycarbonsäure-Verbindung reaktiven, quaternisierbaren Stickstoff-Verbindungen sind ausgewählt unter
a. Hydroxyalkyl-substituierten Mono- oder Polyaminen mit wenigstens einer quarternisierten (z.B. Cholin) oder quaternisierbaren, primären, sekundären oder tertiären Aminogruppe,
b. geradkettigen oder verzweigtem, cyclischen, heterocyclischen, aromatischen oder nichtaromatischen Polyaminen mit wenigstens einer primären oder sekundären (anhydridreaktiven) Aminogruppe und mit wenigstens einer quarternisierten oder quaternisierbaren, primären, sekundären oder tertiären Aminogruppe;
c. Piperazinen.

Insbesondere ausgewählt sind die quaternisierbaren Stickstoff-Verbindungen unter
d. Hydroxyalkyl-substituierten primären, sekundären, tertiären oder quartären Monoaminen und Hydroxyalkyl-substituierten primären, sekundären, tertiären oder quartären Diaminen.
e. geradkettigen oder verzweigten aliphatischen Diaminen mit zwei primären Aminogruppen; Di- oder Polyaminen mit wenigstens einer primären und wenigstens einer sekundären Aminogruppe; Di- oder Polyaminen mit wenigstens einer primären und wenigstens einer tertiären Aminogruppe; Di- oder Polyaminen mit wenigstens einer primären und wenigstens einer quartären Aminogruppe; aromatischen carbo-cyclischen Diaminen mit zwei primären Aminogruppen; aromatischen heterocyclischen Polyaminen mit zwei primären Aminogruppen; aromatischen oder nichtaromatischen Heterozyklen mit einer primären und einer tertiären Aminogruppe;

Beispiele für geeignete "hydroxyalkyl-substituierte Mono- oder Polyamine"sind solche die mit wenigstens einem, wie z.B. 1, 2, 3, 4, 5 oder 6, Hydroxyalkyl-Substituierten ausgestattet sind.

Als Beispiele für "Hydroxyalkyl-substituierte Monoamine" können genannt werden: N-Hydroxyalkyl-monoamine, N,N-Dihydroxyalkyl-monoamine und N,N,N-Trihydroxyalkyl-monoamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl.

Beispielsweise können folgende "Hydroxyalkyl-substituierte Polyamine" und insbesondere "Hydroxyalkyl-substituierte Diamine" genannt werden: (N-Hydroxyalkyl)-alkylendiamine, N,N-Dihydroxyalkyl-alkylendiamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl; Alkylen steht dabei insbesondere für Ethylen, Propylen oder Butylen.

Geeignete "Diamine" sind Alkylendiamine, sowie die N-alkylsubstituierten Analoga davon, wie N-monoalkylierten Alkylendiamine und die N,N- oder N, N'-dialkylierten Alkylendiamine. Alkylen steht insbesondere für geradkettiges oder verzweigtes C₁₋₇oder C₁₋₄-Alkylen, wie oben definiert. Alkyl steht insbesondere für C₁₋₄-Alkyl gemäß obiger Definition. Beispiele sind insbesondere Ethylendiamin, 1,2-Propylendiamin, 1,3-Propylendiamin, 1,4-Butylendiamin und Isomere davon, Pentandiamin und Isomere davon, Hexandiamin und Isomere davon, Heptandiamin und Isomere davon, sowie ein-oder mehrfach, wie z.B. ein - oder zweifach C₁-C₄-alkylierte, wie z.B. methylierte, Derivate der vorher genannten Diamin-Verbindungen, wie 3-Dimethylamino-1-propylamin (DMAPA), N,N-Diethylaminopropylamin, und N,N-Dimethylaminoethylamin.

Geeignete geradkettige "Polyamine" sind beispielsweise Dialkylentriamin, Trialkylentetramin, Tetraalkylenpentamin, Pentaalkylenhexamin, sowie die N-alkylsubstituierten Analoga davon, wie N-monoalkylierten und die N,N- oder N, N'-dialkylierten Alkylenpolyamine. Alkylen steht insbesondere für geradkettiges oder verzweigtes C₁₋₇ oder C₁₋₄₋ Alkylen, wie oben definiert. Alkyl steht insbesondere für C₁₋₄-Alkyl gemäß obiger Definition.

Beispiele sind insbesondere Diethylentriamin, Triethylentetramin, Tetraethylenpentamin, Pentaethylenhexamin, Dipropylentriamin, Tripropylenetetramin, Tetrapropylenpentamin, Pentapropylenhexamin, Dibutylentriamin, Tributylentetramin, Tetrabutylenpentamin, Pentabutylenhexamin; sowie die N,N-Dialkylderivate davon, insbesondere die N,N-Di-C₁₋₄-alkylderivate davon. Als Beispiele können genannt werden: N,N-Dimethyldimethylentriamin, N,N-Diethyldimethylentriamin, N,N-Dipropyldimethylentriamin, N,N-Dimethyldiethylen-1,2-triamin, N,N-Diethyldiethylen-1,2-triamin, N,N-Dipropyldiethylen-1,2-triamin, N,N-Dimethyldipropylen-1,3-triamin (i.e. DMAPAPA), N,N-Diethyldipropylen-1,3-triamin, N,N-Dipropyldipropylen-1,3-triamin, N,N-Dimethyldibutylen-1,4-triamin, N,N-Diethyldibutylen-1,4-triamin, N,N-Dipropyldibutylen-1,4-triamin, N,N-Dimethyldipentylen-1,5-triamin, N,N-Diethyldipentylen-1,5-triamin, N,N-Dipropyldipentylen-1,5-triamin, N,N-Dimethyldihexylen-1,6-triamin, N,N-Diethyldihexylen-1,6-triamin und N,N-Dipropyldihexylen-1,6-triamin,

"Aromatische carbocyclischen Diamine" mit zwei primären Aminogruppen sind die diaminosubstituierten Derivate von Benzol, Biphenyl, Naphthalin, Tetrahydronaphthalin, Fluoren, Inden und Phenanthren.

"Aromatische oder nicht-aromatische heterocyclische Polyamine" mit zwei primären Aminogruppen sind die mit zwei Aminogruppen substituierten Derivate von folgenden Heterocyclen:
- 5- oder 6-gliedriges gesättigtes oder einfach ungesättigtes Heterocyclen, enthaltend ein bis zwei Stickstoffatome und/oder ein Sauerstoff- oder Schwefelatom oder ein oder zwei Sauerstoff- und/oder Schwefelatome als Ringglieder, z. B. Tetrahydrofuran, Pyrrolidin, Isoxazolidin, Isothiazolidin, Pyrazolidin, Oxazolidin, Thiazolidin, Imidazolidin, Pyrrolin, Piperidin, Piperidinyl, 1,3-Dioxan, Tetrahydropyran, Hexahydropyridazin, Hexahydropyrimidin, Piperazin;
- 5-gliedrige aromatische Heterocyclen, enthaltend neben Kohlenstoffatomen ein, zwei oder drei Stickstoffatome oder ein oder zwei Stickstoffatome und ein Schwefel-oder Sauerstoffatom als Ringglieder, z. B. Furan, Thian, Pyrrol, Pyrazol, Oxazol, Thiazol, Imidazol und 1,3,4-Triazol; Isoxazol, Isothiazol, Thiadiazol, Oxadiazol
- 6-gliedrige Heterocyclen enthaltend neben Kohlenstoffatomen ein oder zwei bzw. ein, zwei oder drei Stickstoffatome als Ringglieder, z. B. Pyridinyl, Pyridazin, Pyrimidin, Pyrazinyl, 1,2,4-Triazin , 1,3,5-Triazin-2-yl;

"Aromatische oder nichtaromatische Heterozyklen mit einer primären und einer tertiären Aminogruppe" sind beispielsweise die oben genannten N-Heterocyclen, welche an wenigstens einem Ring-N-Atom aminoalkyliert sind, und insbesondere eine Amino- C₁₋₄⁻alkylgruppe tragen.

"Aromatischen oder nichtaromatischen Heterozyklen mit einer tertiären Aminogruppe und einer Hydroxyalkylgruppe" sind beispielsweise die oben genannten N-Heterocyclen, welche an wenigstens einem Ring-N-Atom hydroxyalkyliert sind, und insbesondere eine Hydroxy-C₁₋₄-alkylgruppe tragen.

Folgende Gruppen einzelner Verbindungsklassen quaternisierbarer Stickstoffverbindungen seien insbesondere genannt:

### Gruppe 1:

| **NAME** | **FORMEL** |
|---|---|
| ***Diamine mit primärem zweiten N-Atom*** | |
| Ethylendiamin | 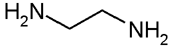 |
| 1,2-Propylendiamin | 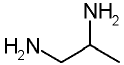 |
| 1,3-Propylendiamin | 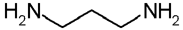 |
| Isomere Butylendiamine, wie z.B. | 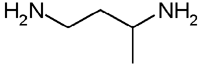 |
| 1,5-Pentylendiamin | 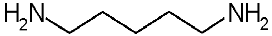 |
| Isomere Pentandiamine, wie z.B. | 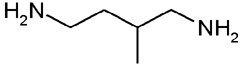 |
| Isomere Hexandiamine, wie z.B. | 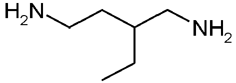 |
| Isomere Heptandiamine, wie z.B. | 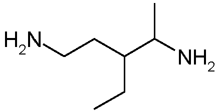 |

| ***Di- und Polyamine mit sekundärem zweiten N-Atom*** | |
|---|---|
| Diethylentriamin (DETA) | 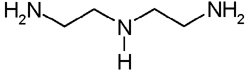 |
| Dipropylentriamin (DPTA), 3,3'-Iminobis(N,N-dimethylpropylamin) | 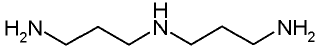 |
| Triethylentetramin (TETA) | 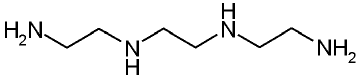 |
| Tetraethylenpentamin (TEPA) | 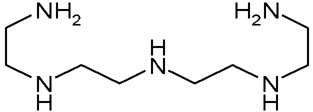 |
| Pentaethylenhexamin | 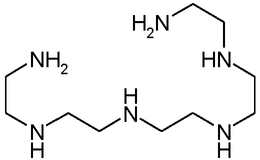 |
| N-Methyl-3-amino-1-propylamin | 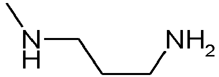 |
| Bishexamethylentriamin | 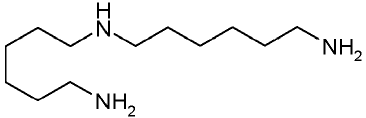 |

| ***Aromaten*** | |
|---|---|
| Diaminobenzole, wie z.B. | 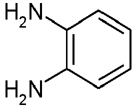 |
| Diaminopyridine, wie z.B. | 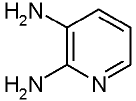 |

### Gruppe 2:

| **NAME** | **FORMEL** |
|---|---|
| ***Heterozyklen*** | |
| 1-(3-Aminopropyl)imidazol | 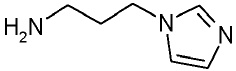 |
| 4-(3-Aminopropyl)-morpholin | 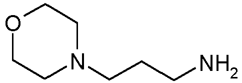 |
| 1-(2-Aminoethylpiperidin) | 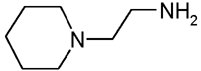 |
| 2-(1-Piperazinyl)ethylamin (AEP) | 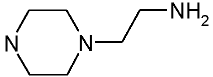 |
| N-Methylpiperazin | 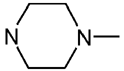 |

| ***Amine mit tertiärem zweiten N-Atom*** | |
|---|---|
| 3,3-Diamino-N-methyldipropylamin | 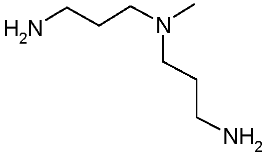 |
| 3-Dimethylamino-1-propylamin (DMAPA) | 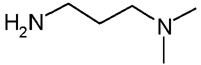 |
| N,N-Diethylaminopropylamin | 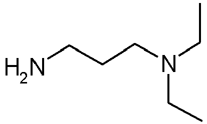 |
| N,N-Dimethylaminoethylamin | 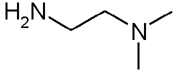 |

### Gruppe 3:

| **NAME** | **FORMEL** |
|---|---|
| ***Alkohole mit primärem und sekundärem Amin*** | |
| Ethanolamin | 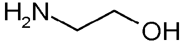 |
| 3-Hydroxy-1-propylamin | 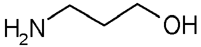 |
| Diethanolamin | 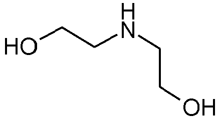 |
| Diisopropanolamin | 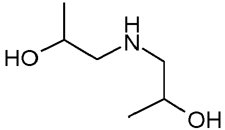 |
| N-(2-Hydroxyethyl)ethylendiamin | 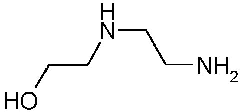 |

| ***Alkohole mit tertiärem Amin*** | |
|---|---|
| Triethanolamin, (2,2',2"-Nitrilotriethanol) | 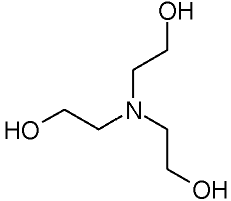 |
| 1-(3-Hydroxypropyl)imidazol | 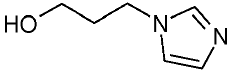 |
| Tris(hydroxymethyl)amin | 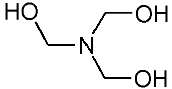 |
| 3-Dimethylamino-1-propanol | 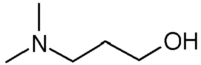 |
| 3-Diethylamino-1-propanol | 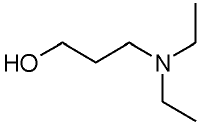 |
| 2-Dimethylamino-1-ethanol | 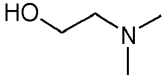 |
| 4-Diethylamino-1-butanol | 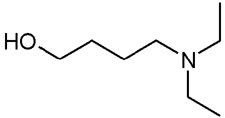 |

Die Umsetzung der Hydrocarbyl-substituierten Polycarbonsäure-Verbindung mit der quaternisierbaren Stickstoffverbindung kann unter thermisch kontrollierten Bedingungen erfolgen, so dass im Wesentlichen keine Kondensationsreaktion erfolgt. Insbesondere ist dann keine Bildung von Reaktionswasser zu beobachten. Insbesondere erfolgt eine derartige Reaktion bei einer Temperatur im Bereich von 10 bis 80, insbesondere 20 bis 60 oder 30 bis 50 °C. Die Reaktionsdauer kann dabei im Bereich von wenigen Minuten oder einigen Stunden, wie z.B. etwa 1 Minute bis zu etwa 10 Stunden liegen. Der Umsetzung kann dabei bei etwa 0,1 bis 2 atm Druck, insbesondere aber etwa bei Normaldruck erfolgen. Beispielsweise ist eine Inertgas-Atmosphäre, wie z.B. Stickstoff, zweckmäßig

Insbesondere kann die Reaktion auch unter erhöhten, eine Kondensation begünstigenden Temperaturen, z. B. im Bereich von oder 90 bis 100 °C oder 100 bis 170 °C erfolgen. Die Reaktionsdauer kann dabei im Bereich von wenigen Minuten oder einigen Stunden, wie z.B. etwa 1 Minute bis zu etwa 10 Stunden liegen. Der Umsetzung kann dabei bei etwa 0,1 bis 2 atm Druck, insbesondere aber etwa bei Normaldruck erfolgen.

Die Reaktanden werden insbesondere in etwa äquimolaren Mengen vorgelegt, gegebenenfalls ist ein geringer, z. B. 0,05 bis 0,5-facher, wie z.B. 0,1 bis 0,3 facher, molarer Überschuss der Polycarbonsäureverbindung wünschenswert. Falls erforderlich können die Reaktanden in einem geeigneten inerten organischen aliphatischen oder aromatischen Lösungsmittel oder einem Gemisch davon, vorgelegt werden. Typischen Beispiele sind z.B. Lösungsmittel der Solvesso Serie, Toluol oder Xylol. Das Lösungsmittel kann beispielsweise auch dazu dienen, Kondensationswasser azeotrop aus dem Reaktionsgemisch zu entfernen. Insbesondere werden die Reaktionen aber ohne Lösungsmittel durchgeführt.

Das so gebildete Reaktionsprodukt kann theoretisch weiter aufgereinigt oder das Lösungsmittel entfernt werden. Gewöhnlich ist dies aber nicht zwingend notwendig, so dass das Reaktionsprodukt ohne weitere Aufreinigung in den nächsten Syntheseschritt, der Quaternisierung, überführt werden kann.

Besonders zu nennen ist das Kondensationprodukt von Polyisobutylenbernsteinsäureanhydrid (Glissopal^{®} SA der Fa. BASF hergestellt aus Polyisobuten (Mn 1000) und Maleinsäureanhydrid in bekannter Weise) und N,N-Dimethyl-1,3-diaminopropan (CAS 109-55-7), siehe Herstellungsbeispiel 1 der WO 2013/000997.

### A4) Quaternisierungsmittel:

)In einer weiteren besonderen Ausführungsform erfolgt die Quaternisierung des mindestens einen quaternisierbaren tertiären Stickstoffatoms mit mindestens einem Quaternisierungsmittel ausgewählt aus Epoxiden, insbesondere Hydrocarbyl-Epoxiden. 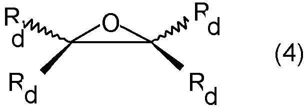 wobei die darin enthaltenen R_{d} Reste gleich oder verschieden sind und für H oder für einen Hydrocarbylrest stehen, wobei der Hydrocarbylrest wenigstens 1 bis 10 Kohlenstoffatome aufweist. Insbesondere sind dies aliphatische oder aromatische Reste, wie z.B. lineare oder verzweigte C₁₋₁₀-Alkylreste oder aromatische Reste, wie Phenyl oder C₁₋₄-Alkylphenyl.

Als Hydrocarbyl-Epoxide eignen sich beispielsweise aliphatische und aromatische Alkylenoxide, wie insbesondere C₂₋₁₂-Alkylenoxide, wie Ethylenoxid, Propylenoxid, 1,2-Butylenoxid, 2,3-Butylenoxid, 2-Methyl-1,2-propenoxid (Isobutenoxid), 1,2-Pentenoxid, 2,3-Pentenoxid, 2-Methyl-1,2-butenoxid, 3-Methyl-1,2-butenoxid, 1,2-Hexenoxid, 2,3-Hexenoxid, 3,4-Hexenoxid, 2-Methyl-1,2-pentenoxid, 2-Ethyl-1,2-butenoxid, 3-Methyl-1,2-pentenoxid, 1,2-Decenoxid, 1,2-Dodecenoxid oder 4-Methyl-1,2-pentenoxid; sowie aromatensubstituierte Ethylenoxide, wie gegebenenfalls substituiertes Styroloxid, insbesondere Styroloxid oder 4-Methyl-styroloxid.

Im Falle der Verwendung von Epoxiden als Quaternisierungsmittel werden diese in Gegenwart von freien Säuren, insbesondere in Gegenwart von freien Hydrocarbyl-substituierten ungesättigten, insbesondere gesättigten, gegebenenfalls substituierten, insbesondere unsubstituierten Protonensäuren, wie vor allem mit Hydrocarbyl-substituierten Dicarbonsäuren, insbesondere Hydrocarbyl-substituierten C₃-C₂₈ oder C₃-C₁₂-Dicarbonsäuren, insbesondere unsubstituierten, gesättigten C₃-C₆ Dicarbonsäure eingesetzt..

Geeignete Dicarbonsäuren sind dabei gesättigte Säuren, wie Malon-, Bernstein-, Glutar-, Adipin, Pimelin-, Suberin,- Azelain-, Sebacinsäure, Undecandisäure und Dodecandisäure oder höhermolekulare Säuren, wie Terta- ,Hexa- oder Octadecandisäure substituierte Säuren, wie Äpfelsäure, α-Ketoglutarsäure, Oxalessigsäure; Glutaminsäure; Asparaginsäure; und ungesättigte Säuren, wie Maleinsäure und Fumarsäure; wie insbesondere Malon-, Bernstein-, Glutar-, Adipin und Pimelinsäure.

Weiterhin geeignet sind aromatische Dicarbonsäure, wie z.B. Phthalsäure.

Falls erforderlich oder gewünscht können auch hydrocarbylsubstituierte Dicarbonsäuren in ihrer Anhydridform eingesetzt werden. Für die Quaternisierung wird die Ringöffnung des Anhydrids dann durch Zugabe von Wasser bewirkt.

Weitere Ausgestaltungen zu Hydrocarbyl-substituierten Dicarbonsäuren:
Die Hydrocarbyl-substituierten Dicarbonsäuren können durch Hydrolyse der entsprechenden Hydrocarbyl-substituierten Dicarbonsäureanhydride in prinzipiell bekannter Weise hergestellt werden, wie beispielsweise in DE 2443537 beschrieben. Die Hydrolyse wird vorzugsweise mit stöchiometrischen Mengen Wasser bei Temperaturen von 50 bis 150°C durchgeführt, es kann aber auch ein Überschuss an Wasser eingesetzt werden. Die Hydrolyse kann ohne Lösungsmittel oder in Gegenwart eines inerten Lösungsmittels durchgeführt werden. Typische Beispiele sind z.B. Lösungsmittel der Solvesso Serie, Toluol, Xylol oder geradkettige und verzweigte gesättigte Kohlenwasserstoffe wie Paraffine oder Naphthene. Das Lösungsmittel kann nach der Hydrolyse entfernt werden, vorzugsweise verbleibt es aber und wird als Lösungsmittel oder Co-Solvent für die anschließende Quaternierung verwendet.

Bevorzugte Hydrocarbyl-substituierten Dicarbonsäureanhydride sind Hydrocarbyl-substituierte Bernsteinsäureanhydride, wie sie beispielsweise von der Fa. Pentagon vertrieben werden: n-Dodecenylbernsteinsäureanhydrid CAS 19780-11-1, n-Octadecenylbernsteinsäureanhydrid CAS. 28777-98-2, i-Octadecenylbernsteinsäureanhydrid CAS. 28777-98-2, i-Hexadecenylbernsteinsäureanhydrid/i-Octadecenylbernsteinsäureanhydrid CAS 32072-96-1 & 28777-98-2, n-Octenylbernsteinsäureanhydrid CAS 26680-54-6. Tetrapropenylbernsteinsäureanhydrid CAS. 26544-38-7.

Weiterhin bevorzugt ist Polyisobutenbernsteinsäureahnydrid (PIBSA). Die Herstellung von PIBSA aus Polyisobuten (PIB) und Maleinsäureanhydrid (MSA) ist prinzipiell bekannt und führt zu einem Gemisch von PIBSA und bismaleiniertem PIBSA (BM PIBSA, siehe bitte Schema 1 unten), welches generell nicht aufgereinigt, sondern als solches weiterverarbeitet wird. Das Verhältnis der beiden Komponenten zueinander kann als Bismaleinierungsgrad (BMG) angegeben werden. Der BMG ist prinzipiell bekannt (siehe US 5,883,196) und wird bestimmt wie in US 5,883,196 beschrieben.

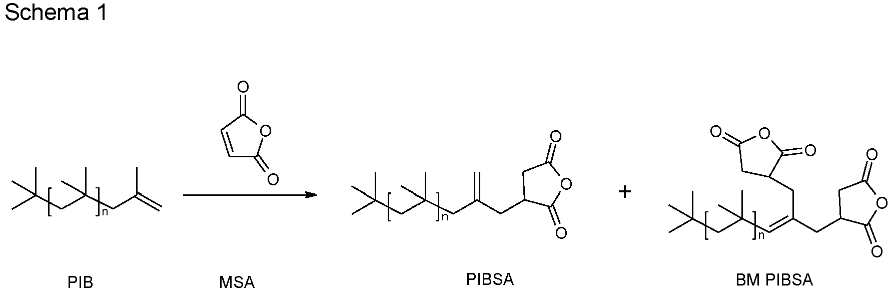

Insbesondere bevorzugt ist PIBSA mit einem Bismaleinierungsgrad bis zu 30, bevorzugt bis zu 25 und besonders bevorzugt bis zu 20%. In der Regel beträgt der Bismaleinierungsgrad mindestens 2, bevorzugt mindestens 5 und besonders bevorzugt mindestens 10%. Die gezielte Herstellung ist beispielsweise in US 5,883,196 beschrieben. Für die Herstellung ist insbesondere hochreaktives PIB (HR-PIB) mit Mn im Bereich von 500 bis 3000, beispielsweise 550 bis 2500, 800 bis 1200 oder 900 bis 1100 geeignet.

Mn wird dabei mittels GPC bestimmt wie in US 5,883,196 beschrieben. Besonders bevorzugtes PIBSA hergestellt aus HR-PIB (Mn = 1000) hat Verseifungszahlen von 85-95 mg KOH/g.

Ein nicht-limitierendes Beispiel für ein besonders geeignetes PIBSA ist Glissopal^{®} SA F der Fa. BASF hergestellt aus HR-PIB (Mn = 1000) mit einem Bismaleinierungsgrad von 15% und einer Verseifungszahl von 90 mg KOH/g.

Es ist auch denkbar, wenn auch weniger bevorzugt, die oben genannten Hydrocarbyl-substituierten Dicarbonsäureanhydride statt mit Wasser mit einem Alkohol, bevorzugt einem Monoalkohol, besonders bevorzugt einem Alkanol, oder einem Amin zum entsprechenden Monoester bzw. Monoamid der Hydrocarbyl-substituierten Dicarbonsäuren umzusetzen. Wichtig ist dabei, dass bei einer derartigen Umsetzung eine Säurefunktion im Molekül verbleibt.

Wir die Quaternisierung in Gegenwart eines Alkohols durchgeführt, so wird für eine derartige Umsetzung der Hydrocarbyl-substituierten Dicarbonsäureanhydride bevorzugt der gleiche Alkohol eingesetzt, wie als Lösungsmittel in der Quaternisierung, also bevorzugt 2-Ethylhexanol oder 2-Propylheptanol, sowie Butyldiglykol, Butylglykol, Methoxypropoxypropanol oder Butoxydipropanol.

Eine derartige Alkoholyse wird vorzugsweise mit stöchiometrischen Mengen Alkohol oder Amin bei Temperaturen von 50 bis 150°C durchgeführt, es kann aber auch ein Überschuss an Alkohol oder Amin, bevorzugt Alkohol eingesetzt werden. Dieser verbleibt dann zweckmäßigerweise im Reaktionsgemisch und dient in der nachfolgenden Quaternisierung als Lösungsmittel.

### A5) Herstellung erfindungsgemäßer Additive:

### a) Quaternisierung

Die Quaternierung mit einem Epoxid der Formel (4) erfolgt grundsätzlich in Anlehnung an bekannte Verfahren. Liegt die Siedetemperatur einer Komponente des Reaktionsgemisches, insbesondere des Epoxides, bei Normaldruck oberhalb der Reaktionstemperatur, wird die Reaktion zweckmäßigerweise in einem Autoklaven durchgeführt.

Beispielsweise wird in einem Autoklaven eine Lösung des tertiären Amins mit der organischen hydrocarbylsustituierten Dicarbonsäure (wie z.B. Polyisobutenbernsteinäure) in den erforderlichen in etwa stöchiometrischen Mengen versetzt. Pro Äquivalent an quaternisierbarem tertiären Stickstoffatom kann man z.B. 0,1 bis 2,0, 0,2 bis 1,5, oder 0,5 bis 1,25 Äquivalente an Dicarbonsäure einsetzen. Insbesondere werden aber etwa annähernd molare Anteile der Dicarbonsäure eingesetzt. Anschließend wird ausreichend mit N₂ gespült, sowie ein geeigneter Vordruck eingestellt und das Epoxid (z.B. Propylenoxid) wird in den erforderlichen stöchiometrischen Mengen bei einer Temperatur zwischen 20°C und 180°C zudosiert. Pro Äquivalent an quaternisierbarem tertiären Stickstoffatom kann man z.B. 0,1 bis 4,0 , 0,2 bis 3, oder 0,5 bis 2 Äquivalente an Epoxid einsetzen. Insbesondere werden aber etwa 1 bis 2 Äquivalente Epoxid in Relation zum tertiären Amin eingesetzt, um die tertiäre Amingruppe vollständig zu quaternisieren. Insbesondere kann auch ein Molarer Überschuss an Alkylenoxid gesetzt werden, wodurch die freie Carboxylgruppe der Dicarbonsäure teilweise oder vollständig verestert wird. Anschließend wird über eine geeignet langen Zeitraum von wenigen Minuten bis etwa 24 Stunden, wie z.B. etwa 10 h bei einer Temperatur zwischen 20°C und 180°C (z.B. 50°C) nachgerührt, abgekühlt, wie z.B. auf etwa 20 bis 50°C, mit N₂ gespült und der Reaktor entleert.

Die Umsetzung kann dabei bei etwa 0,1 bis 20 bar, wie z.B. 1 bis 10 oder 1,5 bis 5 bar Druck erfolgen. Die Umsetzung kann aber auch bei Normaldruck erfolgen. Insbesondere ist eine Inertgas-Atmosphäre, wie z.B. Stickstoff, zweckmäßig.

Falls erforderlich können die Reaktanden in einem geeigneten inerten organischen aliphatischen oder aromatischen Lösungsmittel oder einem Gemisch davon, für die Quaternisierung vorgelegt werden. Typische Beispiele sind z.B. Lösungsmittel der Solvesso Serie, Toluol oder Xylol oder 2-Ethylhexanol, oder 2-Propylheptanol, sowie Butyldiglykol, Butylglykol, Methoxypropoxypropanol, Butoxydipropanol oder geradkettige und verzweigte gesättigte Kohlenwasserstoffe, wie Paraffine oder Naphthene. Die Quaternisierung kann aber auch in Abwesenheit eines Lösungsmittels durchgeführt werden.

Die Quaternisierung kann in Gegenwart eines protischen Lösungsmittels durchgeführt werden, gegebenenfalls auch in Kombination mit einem aliphatischen oder aromatischen Lösungsmittel. Geeignete protische Lösungsmittel haben insbesondere eine Dielktrizitätskonstante (bei 20°C) von größer 7. Das protische Lösungsmittel kann eine oder mehrere OH-Gruppen enthalten und kann auch Wasser sein. Geeignete Lösungsmittel können auch Alkohole, Glykole und Glykolether sein. Insbesondere können geeignete protische Lösungsmittel solche sein, die in WO 2010132259 genannt sind. Insbesondere geeignete Lösungsmittel sind Methanol, Ethanol, n-Propanol, isoPropanol, alle Isomeren des Butanols, alle Isomeren des Pentanols, alle Isomeren des Hexanols, 2-Ethylhexanol, 2-Propylheptanol, sowie auch Gemische verschiedener Alkohole. Die Anwesenheit eines protischen Lösungsmittels kann den Umsatz und die Reaktionsgeschwindigkeit der Quaternierung positiv beeinflussen.

### b) Aufarbeitung des Reaktionsgemisches

Das so gebildete Reaktionsendprodukt kann theoretisch weiter aufgereinigt oder das Lösungsmittel kann entfernt werden. Gegebenenfalls kann überschüssiges Reagenz, wie beispielsweise überschüssiges Epoxid, entfernt werden. Dies kann beispielsweise durch Einleiten von Stickstoff bei Normaldruck oder unter vermindertem Druck geschehen. Um die weitere Prozessierbarkeit der Produkte zu verbessern kann aber auch nach der Reaktion Lösungsmittel zugesetzt werden, wie z.B. Lösungsmittel der Solvesso Reihe, 2-Ethylhexanol, oder im Wesentlichen aliphatische Lösungsmittel. Gewöhnlich ist dies aber nicht zwingend notwendig, so dass das Reaktionsprodukt ohne weitere Aufreinigung als Additiv, gegebenenfalls nach Abmischung mit weiteren Additivkomponenten (s. unten) einsetzbar ist.

Es stellt eine bevorzugte Ausführungsform der vorliegenden Erfindung dar, dass die quaternisierten Ammoniumverbindungen einen Gewichtsverlust in einer thermogravimetrischen Analyse (TGA) bei 350 °C von weniger als 50 Gew.-%, wie z.B. weniger als 40, weniger als 35, weniger als 30, weniger als 20 oder weniger als 15, wie z.B. bis zu 0 bis 5 Gew.-% Gewichtsverlust aufweisen.

Dazu wird eine thermogravimetrische Analyse (TGA) entsprechend der Norm ISO-4154 durchgeführt. Im Einzelnen wird im Test ein Lauf von 50° auf 900 °C bei einer Temperaturanstiegsgeschwindigkeit von 20 °C pro Minute unter einer Stickstoffatmosphäre bei einer Fließgeschwindigkeit von 60 mL pro Minute durchgeführt.

### B) Weitere Additivkomponenten

Der mit dem erfindungsgemäßen quaternisierten Additiv additivierte Kraftstoff ist ein Ottokraftstoff oder insbesondere ein Mitteldestillat-Kraftstoff, vor allem ein Dieselkraftstoff.

Der Kraftstoff kann weitere übliche Additive zur Wirksamkeitsverbesserung und/oder Verschleißunterdrückung enthalten.

Im Falle von Dieselkraftstoffen sind dies in erster Linie übliche Detergenz-Additive, Trägeröle, Kaltfließverbesserer, Schmierfähigkeitsverbesserer (Lubricity Improver), Korrosionsinhibitoren, Demulgatoren, Dehazer, Antischaummittel, Cetanzahlverbesserer, Verbrennungsverbesserer, Antioxidantien oder Stabilisatoren, Antistatika, Metallocene, Metalldeaktivatoren, Farbstoffe und/oder Lösungsmittel.

Im Falle von Ottokraftstoffen sind dies vor allem Schmierfähigkeitsverbesserer (Friction Modifier), Korrosionsinhibitoren, Demulgatoren, Dehazer, Antischaummittel, Verbrennungsverbesserer, Antioxidantien oder Stabilisatoren, Antistatika, Metallocene, Metalldeaktivatoren, Farbstoffe und/oder Lösungsmittel.

Typische Beispiele geeigneter Co-Additive sind im folgenden Abschnitt aufgeführt:

### B1) Detergenz-Additive

Vorzugsweise handelt es sich bei den üblichen Detergenz-Additiven um amphiphile Substanzen, die mindestens einen hydrophoben Kohlenwasserstoffrest mit einem zahlengemittelten Molekulargewicht (Mₙ) von 85 bis 20.000 und mindestens eine polare Gruppierung besitzen, die ausgewählt ist unter:
(Da) Mono- oder Polyaminogruppen mit bis zu 6 Stickstoffatomen, wobei mindestens ein Stickstoffatom basische Eigenschaften hat;
(Db) Nitrogruppen, gegebenenfalls in Kombination mit Hydroxylgruppen;
(Dc) Hydroxylgruppen in Kombination mit Mono- oder Polyaminogruppen, wobei mindestens ein Stickstoffatom basische Eigenschaften hat;
(Dd) Carboxylgruppen oder deren Alkalimetall- oder Erdalkalimetallsalzen;
(De) Sulfonsäuregruppen oder deren Alkalimetall- oder Erdalkalimetallsalzen;
(Df) Polyoxy-C₂- bis C₄-alkylengruppierungen, die durch Hydroxylgruppen, Mono-oder Polyaminogruppen, wobei mindestens ein Stickstoffatom basische Eigenschaften hat, oder durch Carbamatgruppen terminiert sind;
(Dg) Carbonsäureestergruppen;
(Dh) aus Bernsteinsäureanhydrid abgeleiteten Gruppierungen mit Hydroxy- und/oder Amino- und/oder Amido- und/oder Imidogruppen; und/oder
(Di) durch Mannich-Umsetzung von substituierten Phenolen mit Aldehyden und Mono- oder Polyaminen erzeugten Gruppierungen.

Der hydrophobe Kohlenwasserstoffrest in den obigen Detergenz-Additiven, welcher für die ausreichende Löslichkeit im Kraftstoff sorgt, hat ein zahlengemitteltes Molekulargewicht (Mₙ) von 85 bis 20.000, vorzugsweise von 113 bis 10.000, besonders bevorzugt von 300 bis 5.000, stärker bevorzugt von 300 bis 3.000, noch stärker bevorzugt von 500 bis 2.500 und insbesondere von 700 bis 2.500, vor allem von 800 bis 1500. Als typischer hydrophober Kohlenwasserstoffrest, insbesondere in Verbindung mit den polaren insbesondere Polypropenyl-, Polybutenyl- und Polyisobutenylreste mit einem zahlenmittleren Molekulargewicht Mₙ von vorzugsweise jeweils 300 bis 5.000, besonders bevorzugt 300 bis 3.000, stärker bevorzugt 500 bis 2.500 noch stärker bevorzugt 700 bis 2.500 und insbesondere 800 bis 1.500 in Betracht.

Als Beispiele für obige Gruppen von Detergenz-Additiven seien die folgenden genannt:
Mono- oder Polyaminogruppen (Da) enthaltende Additive sind vorzugsweise Polyalkenmono- oder Polyalkenpolyamine auf Basis von Polypropen oder von hochreaktivem (d.h. mit überwiegend endständigen Doppelbindungen) oder konventionellem (d.h. mit überwiegend mittenständigen Doppelbindungen) Polybuten oder Polyisobuten mit Mₙ = 300 bis 5000, besonders bevorzugt 500 bis 2500 und insbesondere 700 bis 2500. Derartige Additive auf Basis von hochreaktivem Polyisobuten, welche aus dem Polyisobuten, das bis zu 20 Gew.-% n-Buten-Einheiten enthalten kann, durch Hydroformylierung und reduktive Aminierung mit Ammoniak, Monoaminen oder Polyaminen wie Dimethyl-aminopropylamin, Ethylendiamin, Diethylentriamin, Triethylentetramin oder Tetraethylenpentamin hergestellt werden können, sind insbesondere aus der EP-A 244 616 bekannt. Geht man bei der Herstellung der Additive von Polybuten oder Polyisobuten mit überwiegend mittenständigen Doppelbindungen (meist in der β- und γ-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier Amine, wie z. B. Ammoniak, Monoamine oder die oben genannten Polyamine, eingesetzt werden. Entsprechende Additive auf Basis von Polypropen sind insbesondere in der WO-A 94/24231 beschrieben.

Weitere besondere Monoaminogruppen (Da) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in der WO-A 97/03946 beschrieben sind.

Weitere besondere Monoaminogruppen (Da) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in der DE-A 196 20 262 beschrieben sind.

Nitrogruppen (Db), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in der WO-A96/03367 und in der WO-A 96/03479 beschrieben sind. Diese Umsetzungsprodukte stellen in der Regel Mischungen aus reinen Nitropolyisobutenen (z. B. α,β-Dinitropolyisobuten) und gemischten Hydroxynitropolyisobutenen (z. B. α-Nitro-β-hydroxypolyisobuten) dar.

Hydroxylgruppen in Kombination mit Mono- oder Polyaminogruppen (Dc) enthaltende Additive sind insbesondere Umsetzungsprodukte von Polyisobutenepoxiden, erhältlich aus vorzugsweise überwiegend endständige Doppelbindungen aufweisendem Polyisobuten mit Mₙ = 300 bis 5000 mit Ammoniak, Mono- oder Polyaminen, wie sie insbeson-dere in der EP-A 476 485 beschrieben sind.

Carboxylgruppen oder deren Alkalimetall- oder Erdalkalimetallsalze (Dd) enthaltende Additive sind vorzugsweise Copolymere von C₂- bis C₄₀-Olefinen mit Maleinsäureanhydrid mit einer Gesamt-Molmasse von 500 bis 20.000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A 307 815 bekannt. Derartige Additive dienen hauptsächlich zur Verhinderung von Ventilsitzverschleiß und können, wie in der WO-A 87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (De) enthaltende Additive sind vorzugsweise Alkalimetall- oder Erdalkalimetallsalze eines Sulfobernsteinsäurealkylesters, wie er insbesondere in der EP-A 639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)buten-aminen oder Polyetheraminen eingesetzt werden.

Polyoxy-C₂-C₄-alkylengruppierungen (Df) enthaltende Additive sind vorzugsweise Polyether oder Polyetheramine, welche durch Umsetzung von C₂- bis C₆₀-Alkanolen, C₆-bis C₃₀-Alkandiolen, Mono- oder Di-C₂- bis C₃₀-alkylaminen, C₁- bis C₃₀-Alkylcyclo-hexanolen oder C₁- bis C₃₀-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und US-A 4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (Dg) enthaltende Additive sind vorzugsweise Ester aus Mono-, Di- oder Tricarbonsäuren mit langkettigen Alkanolen oder Polyolen, insbesondere solche mit einer Mindestviskosität von 2 mm²/s bei 100 °C, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder insbesondere Imidogruppen (Dh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit Mₙ = vorzugsweise 300 bis 5000, besonders bevorzugt 300 bis 3000, stärker bevorzugt 500 bis 2500, noch stärker bevorzugt 700 bis 2500 und insbesondere 800 bis 1500, mit Maleinsäureanhydrid auf thermischem Weg in einer En-Reaktion oder über das chlorierte Polyisobuten erhältlich sind. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Beim Vorliegen von Imidogruppierungen D(h) wird das weitere Detergenz-Additiv im Sinne der vorliegenden Erfindung jedoch nur bis maximal 100 % der Gewichtsmenge an Verbindungen mit Betainstruktur eingesetzt. Derartige Kraftstoffadditive sind allgemein bekannt und beispielsweise in den Dokumenten (1) und (2) beschrieben. Bevorzugt handelt es sich um die Umsetzungsprodukte von Alkyl- oder Alkenyl-substituierten Bernsteinsäuren oder Derivaten davon mit Aminen und besonders bevorzugt um die Umsetzungsprodukte von Polyisobutenyl-substituierten Bernsteinsäuren oder Derivaten davon mit Aminen. Von besonderem Interesse sind hierbei Umsetzungsprodukte mit aliphatischen Polyaminen (Polyalkylenimine) wie insbesondere Ethylendiamin, Diethylentriamin, Triethylentetramin, Tetraethylenpentamin, Pentaethylenhexamin und Hexaethylenheptamin, welche eine Imidstruktur aufweisen.

Durch Mannich-Umsetzung von substituierten Phenolen mit Aldehyden und Mono-oder Polyaminen erzeugte Gruppierungen (Di) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit Mₙ = 300 bis 5000 stammen. Derartige "Polyisobuten-Mannichbasen" sind insbesondere in der EP-A 831 141 beschrieben.

Dem Kraftstoff können ein oder mehrere der genannten Detergenz-Additive in solch einer Menge zugegeben werden, dass die Dosierrate an diesen Detergenz-Additiven vozugsweise 25 bis 2500 Gew.-ppm, insbesondere 75 bis 1500 Gew.-ppm, vor allem 150 bis 1000 Gew.-ppm, beträgt.

### B2) Trägeröle

Mitverwendete Trägeröle können mineralischer oder synthetischer Natur sein. Geeignete mineralische Trägeröle sind bei der Erdölverarbeitung anfallende Fraktionen, wie Brightstock oder Grundöle mit Viskositäten wie beispielsweise aus der Klasse SN 500 bis 2000, aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500 °C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind Polyolefine (Polyalphaolefine oder Polyinternalolefine), (Poly)ester, Poly)alkoxylate, Polyether, aliphatische Polyetheramine, alkylphenolgestartete Polyether, alkylphenolgestartete Polyetheramine und Carbonsäureester langkettiger Alkanole.

Beispiele für geeignete Polyolefine sind Olefinpolymerisate mit Mₙ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-C₂-bis C₄-alkylengruppierungen enthaltende Verbindungen, welche durch Umsetzung von C₂- bis C₆₀-Alkanolen, C₆- bis C₃₀-Alkandiolen, Mono- oder Di-C₂- bis C₃₀-alkylaminen, C₁- bis C₃₀-Alkyl-cyclohexanolen oder C₁- bis C₃₀-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und der US-A 4,877,416 beschrieben. Beispielsweise können als Polyetheramine Poly-C₂- bis C₆-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 Kohlenstoffatomen. Typische Vertreter der Ester sind Adipate, Phthalate, iso-Phthalate, Terephthalate und Trimellitate des Isooctanols, Isononanols, Isodecanols und des Iso-tridecanols, z. B. Di-(n- oder Isotridecyl)phthalat.

Weitere geeignete Trägerölsysteme sind beispielsweise in der DE-A 38 26 608, DE-A 41 42 241, DE-A 43 09 074, EP-A 452 328 und der EP-A 548 617 beschrieben.

Beispiele für besonders geeignete synthetische Trägeröle sind alkoholgestartete Polyether mit etwa 5 bis 35, vorzugsweise etwa 5 bis 30, besonders bevorzugt 10 bis 30 und insbesondere 15 bis 30 C₃- bis C₆-Alkylenoxideinheiten, z. B. Propylenoxid-, n-Butylenoxid- und Isobutylenoxid-Einheiten oder Gemischen davon, pro Alkoholmolekül. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten C₆- bis C₁₈-Alkylrest steht. Als besondere Beispiele sind zu nennen Tridecanol und Nonylphenol. Besonders bevorzugte alkoholgestartete Polyether sind die Umsetzungsprodukte (Polyveretherungsprodukte) von einwertigen aliphatischen C₆- bis C₁₈-Alkoholen mit C₃- bis C₆-Alkylenoxiden. Beispiele für einwertige aliphatische C₆-C₁₈-Alkohole sind Hexanol, Heptanol, Octanol, 2-Ethylhexanol, Nonylalkohol, Decanol, 3-Propylheptanol, Undecanol, Dodecanol, Tridecanol, Tetradecanol, Pentadecanol, Hexadecanol, Octadecanol und deren Konstitutions- und Stellungsisomere. Die Alkohole können sowohl in Form der reinen Isomere als auch in Form technischer Gemische eingesetzt werden. Ein besonders bevorzugter Alkohol ist Tridecanol. Beispiele für C₃- bis C₆-Alkylenoxide sind Propylenoxid, wie 1,2-Propylenoxid, Butylenoxid, wie 1,2-Butylenoxid, 2,3-Butylenoxid, Isobutylenoxid oder Tetrahydrofuran, Pentylenoxid und Hexylenoxid. Besonders bevorzugt sind hierunter C₃- bis C₄-Alkylenoxide, d.h. Propylenoxid wie 1,2-Propylenoxid und Butylenoxid wie 1,2-Butylenoxid, 2,3-Butylenoxid und Isobutylenoxid. Speziell verwendet man Butylenoxid.

Weitere geeignete synthetische Trägeröle sind alkoxylierte Alkylphenole, wie sie in der DE-A 10 102 913 beschrieben sind.

Besondere Trägeröle sind synthetische Trägeröle, wobei die zuvor beschriebenen alkoholgestarteten Polyether besonders bevorzugt sind.

Das Trägeröl bzw. das Gemisch verschiedener Trägeröle wird dem Kraftstoff in einer Menge von vorzugsweise 1 bis 1000 Gew.-ppm, besonders bevorzugt von 10 bis 500 Gew.-ppm und insbesondere von 20 bis 100 Gew.-ppm zugesetzt.

### B3) Kaltfließverbesserer

Geeignete Kaltfließverbesserer sind im Prinzip alle organischen Verbindungen, welche in der Lage sind, das Fließverhalten von Mitteldestillat-Kraftstoffen bzw. Dieselkraftstoffen in der Kälte zu verbessern. Zweckmäßigerweise müssen sie eine ausreichende Öllöslichkeit aufweisen. Insbesondere kommen hierfür die üblicherweise bei Mitteldestillaten aus fossilem Ursprung, also bei üblichen mineralischen Dieselkraftstoffen, eingesetzten Kaltfließverbesserer ("middle distillate flow improvers", "MDFI") in Betracht. Jedoch können auch organische Verbindungen verwendet werden, die beim Einsatz in üblichen Dieselkraftstoffen zum Teil oder überwiegend die Eigenschaften eines Wax Anti-Settling Additivs ("WASA") aufweisen. Auch können sie zum Teil oder überwiegend als Nukleatoren wirken. Es können aber auch Mischungen aus als MDFI wirksamen und/oder als WASA wirksamen und/oder als Nukleatoren wirksamen organischen Verbindungen eingesetzt werden.

Typischerweise wird der Kaltfließverbesserer ausgewählt aus:

| | |
|---|---|
| (K1) | Copolymeren eines C₂- bis C₄₀-Olefins mit wenigstens einem weiteren ethylenisch ungesättigten Monomer; |
| (K2) | Kammpolymeren; |
| (K3) | Polyoxyalkylenen; |
| (K4) | polaren Stickstoffverbindungen; |
| (K5) | Sulfocarbonsäuren oder Sulfonsäuren oder deren Derivaten; und |
| (K6) | Poly(meth)acrylsäureestern. |

Es können sowohl Mischungen verschiedener Vertreter aus einer der jeweiligen Klassen (K1) bis (K6) als auch Mischungen von Vertretern aus verschiedenen Klassen (K1) bis (K6) eingesetzt werden.

Geeignete C₂- bis C₄₀-Olefin-Monomere für die Copolymeren der Klasse (K1) sind beispielsweise solche mit 2 bis 20, insbesondere 2 bis10 Kohlenstoffatomen sowie mit 1 bis 3, vorzugsweise mit 1 oder 2, insbesondere mit einer Kohlenstoff-Kohlenstoff-Doppelbindung. Im zuletzt genannten Fall kann die Kohlenstoff-Kohlenstoff-Doppelbindung sowohl terminal (α-Olefine) als auch intern angeordnet sein kann. Bevorzugt sind jedoch α-Olefine, besonders bevorzugt α-Olefine mit 2 bis 6 Kohlenstoffatomen, beispielsweise Propen, 1-Buten, 1-Penten, 1-Hexen und vor allem Ethylen.

Bei den Copolymeren der Klasse (K1) ist das wenigstens eine weitere ethylenisch ungesättigte Monomer vorzugsweise ausgewählt unter Carbonsäurealkenylestern, (Meth)Acrylsäureestern und weiteren Olefinen.

Werden weitere Olefine mit einpolymerisiert, sind dies vorzugsweise höhermolekulare als das oben genannte C₂- bis C₄₀-Olefin-Basismonomere. Setzt man beispielsweise als Olefin-Basismonomer Ethylen oder Propen ein, eignen sich als weitere Olefine insbesondere C₁₀- bis C₄₀-α-Olefine. Weitere Olefine werden in den meisten Fällen nur dann mit einpolymerisiert, wenn auch Monomere mit Carbonsäureester-Funktionen eingesetzt werden.

Geeignete (Meth)Acrylsäureester sind beispielsweise Ester der (Meth)Acrylsäure mit C₁- bis C₂₀-Alkanolen, insbesondere C₁- bis C₁₀-Alkanolen, vor allem mit Methanol, Ethanol, Propanol, Isopropanol, n-Butanol, sec.-Butanol, Isobutanol, tert.-Butanol, Pentanol, Hexanol, Heptanol, Octanol, 2-Ethylhexanol, Nonanol und Decanol sowie Strukturisomeren hiervon.

Geeignete Carbonsäurealkenylester sind beispielsweise C₂- bis C₁₄-Alkenylester, z.B. die Vinyl- und Propenylester, von Carbonsäuren mit 2 bis 21 Kohlenstoffatomen, deren Kohlenwasserstoffrest linear oder verzweigt sein kann. Bevorzugt sind hierunter die Vinylester. Unter den Carbonsäuren mit verzweigtem Kohlenwasserstoffrest sind solche bevorzugt, deren Verzweigung sich in der α-Position zur Carboxylgruppe befindet, wobei das α-Kohlenstoffatom besonders bevorzugt tertiär ist, d. h. die Carbonsäure eine sogenannte Neocarbonsäure ist. Vorzugsweise ist der Kohlenwasserstoffrest der Carbonsäure jedoch linear.

Beispiele für geeignete Carbonsäurealkenylester sind Vinylacetat, Vinylpropionat, Vinylbutyrat, Vinyl-2-ethylhexanoat, Neopentansäurevinylester, Hexansäurevinylester, Neononansäurevinylester, Neodecansäurevinylester und die entsprechenden Propenyl-ester, wobei die Vinylester bevorzugt sind. Ein besonders bevorzugter Carbonsäurealkenylester ist Vinylacetat; typische hieraus resultierende Copolymere der Gruppe (K1) sind die mit am häufigsten eingesetzten Ethylen-Vinylacetat-Copolymere ("EVA"). Besonders vorteilhaft einsetzbare Ethylen-Vinylacetat-Copolymere und ihre Herstellung sind in der WO 99/29748 beschrieben.

Als Copolymere der Klasse (K1) sind auch solche geeignet, die zwei oder mehrere voneinander verschiedene Carbonsäurealkenylester einpolymerisiert enthalten, wobei diese sich in der Alkenylfunktion und/oder in der Carbonsäuregruppe unterscheiden. Ebenfalls geeignet sind Copolymere, die neben dem/den Carbonsäurealkenylester(n) wenigstens ein Olefin und/oder wenigstens ein (Meth)Acrylsäureester einpolymerisiert enthalten.

Auch Terpolymere aus einem C₂- bis C₄₀-α-Olefin, einem C₁- bis C₂₀-Alkylester einer ethylenisch ungesättigten Monocarbonsäure mit 3 bis 15 Kohlenstoffatomen und einem C₂- bis C₁₄-Alkenylester einer gesättigten Monocarbonsäure mit 2 bis 21 Kohlenstoffatomen sind als Copolymere der Klasse (K1) geeignet. Derartige Terpolymere sind in der WO 2005/054314 beschrieben. Ein typisches derartiges Terpolymer ist aus Ethylen, Acrylsäure-2-ethylhexylester und Vinylacetat aufgebaut.

Das wenigstens eine oder die weiteren ethylenisch ungesättigten Monomeren sind in den Copolymeren der Klasse (K1) in einer Menge von vorzugsweise 1 bis 50 Gew.-%, insbesondere von 10 bis 45 Gew.-% und vor allem von 20 bis 40 Gew.-%, bezogen auf das Gesamtcopolymer, einpolymerisiert. Der gewichtsmäßige Hauptanteil der Monomereinheiten in den Copolymeren der Klasse (K1) stammt somit in der Regel aus den C₂- bis C₄₀-Basis-Olefinen.

Die Copolymere der Klasse (K1) weisen vorzugsweise ein zahlenmittleres Molekulargewicht Mₙ von 1000 bis 20.000, besonders bevorzugt von 1000 bis 10.000 und insbesondere von 1000 bis 8000 auf.

Typische Kammpolymere der Komponente (K2) sind beispielsweise durch die Copolymerisation von Maleinsäureanhydrid oder Fumarsäure mit einem anderen ethylenisch ungesättigten Monomer, beispielsweise mit einem α-Olefin oder einem ungesättigten Ester wie Vinylacetat, und anschließende Veresterung der Anhydrid- bzw. Säurefunktion mit einem Alkohol mit wenigstens 10 Kohlenstoffatomen erhältlich. Weitere geeignete Kammpolymere sind Copolymere von α-Olefinen und veresterten Comonomeren, beispielsweise veresterte Copolymere von Styrol und Maleinsäureanhydrid oder veresterte Copolymere von Styrol und Fumarsäure. Geeignete Kammpolymere können auch Polyfumarate oder Polymaleinate sein. Außerdem sind Homo- und Copolymere von Vinylethern geeignete Kammpolymere. Als Komponente der Klasse (K2) geeignete Kammpolymere sind beispielsweise auch solche, die in der WO 2004/035715 und in "Comb-Like Polymers. Structure and Properties", N. A. Platé und V. P. Shibaev, J. Poly. Sci. Macromolecular Revs. 8, Seiten 117 bis 253 (1974)" beschrieben sind. Auch Gemische von Kammpolymeren sind geeignet.

Als Komponente der Klasse (K3) geeignete Polyoxyalkylene sind beispielsweise Polyoxyalkylenester, Polyoxyalkylenether, gemischte Polyoxyalkylenesterether und Gemische davon. Bevorzugt enthalten diese Polyoxyalkylenverbindungen wenigstens eine, vorzugsweise wenigstens zwei lineare Alkylgruppen mit jeweils 10 bis 30 Kohlenstoffatomen und eine Polyoxyalkylengruppe mit einem zahlenmittleren Molekulargewicht von bis zu 5000. Derartige Polyoxyalkylenverbindungen sind beispielsweise in der EP-A 061 895 sowie in der US 4 491 455 beschrieben. Besondere Polyoxyalkylenverbindungen basieren auf Polyethylenglykolen und Polypropylenglykolen mit einem zahlenmittleren Molekulargewicht von 100 bis 5000. Weiterhin sind Polyoxyalkylenmono- und -diester von Fettsäuren mit 10 bis 30 Kohlenstoffatomen wie Stearinsäure oder Behensäure geeignet.

Als Komponente der Klasse (K4) geeignete polare Stickstoffverbindungen können sowohl ionischer als auch nicht ionischer Natur sein und besitzen vorzugsweise wenigstens einen, insbesondere wenigstens zwei Substituenten in Form eines tertiären Stickstoffatoms der allgemeinen Formel >NR⁷, worin R⁷ für einen C₈- bis C₄₀-Kohlenwasserstoffrest steht. Die Stickstoffsubstituenten können auch quaternisiert, das heißt in kationischer Form, vorliegen. Beispiele für solche Stickstoffverbindungen sind Ammoniumsalze und/oder Amide, die durch die Umsetzung wenigstens eines mit wenigstens einem Kohlenwasserstoffrest substituierten Amins mit einer Carbonsäure mit 1 bis 4 Carboxylgruppen bzw. mit einem geeignetem Derivat davon erhältlich sind. Vorzugsweise enthalten die Amine wenigstens einen linearen C₈- bis C₄₀-Alkylrest. Zur Herstellung der genannten polaren Stickstoffverbindungen geeignete primäre Amine sind beispielsweise Octylamin, Nonylamin, Decylamin, Undecylamin, Dodecylamin, Tetradecylamin und die höheren linearen Homologen, hierzu geeignete sekundäre Amine sind beispielsweise Dioctadecylamin und Methylbehenylamin. Geeignet sind hierzu auch Amingemische, insbesondere großtechnisch zugängliche Amingemische wie Fettamine oder hydrierte Tallamine, wie sie beispielsweise in Ullmanns Encyclopedia of Industrial Chemistry, 6. Auflage, im Kapitel "Amines, aliphatic" beschrieben werden. Für die Umsetzung geeignete Säuren sind beispielsweise Cyclohexan-1,2-dicarbonsäure, Cyclohexen-1,2-dicarbonsäure, Cyclopentan-1,2-dicarbonsäure, Naphthalindicarbonsäure, Phthalsäure, Isophthalsäure, Terephthalsäure und mit langkettigen Kohlenwasserstoffresten substituierte Bernsteinsäuren.

Insbesondere ist die Komponente der Klasse (K4) ein öllösliches Umsetzungsprodukt aus mindestens eine tertiäre Aminogruppe aufweisenden Poly(C₂- bis C₂₀-Carbon-säuren) mit primären oder sekundären Aminen. Die diesem Umsetzungsprodukt zugrundeliegenden mindestens eine tertiäre Aminogruppe aufweisenden Poly(C₂- bis C₂₀-Carbonsäuren) enthalten vorzugsweise mindestens 3 Carboxylgruppen, insbesondere 3 bis 12, vor allem 3 bis 5 Carboxylgruppen. Die Carbonsäure-Einheiten in den Polycarbonsäuren weisen vorzugsweise 2 bis 10 Kohlenstoffatome auf, insbesondere sind es Essigsäure-Einheiten. Die Carbonsäure-Einheiten sind in geeigneter Weise zu den Polycarbonsäuren verknüpft, meist über ein oder mehrere Kohlenstoff- und/oder Stickstoffatome. Vorzugsweise sind sie an tertiäre Stickstoffatome angebunden, die im Falle mehrerer Stickstoffatome über Kohlenwasserstoffketten verbunden sind.

Vorzugsweise ist die Komponente der Klasse (K4) ein öllösliches Umsetzungsprodukt auf Basis von mindestens eine tertiäre Aminogruppe aufweisenden Poly(C₂- bis C₂₀-Carbonsäuren) der allgemeinen Formel IIa oder Ilb 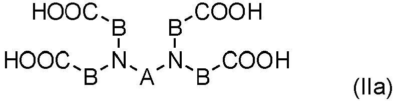 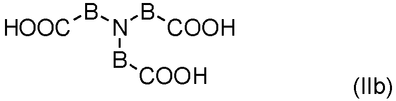 in denen die Variable A eine geradkettige oder verzweigte C₂- bis C₆-Alkylengruppe oder die Gruppierung der Formel III 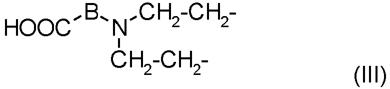 darstellt und die Variable B eine C₁- bis C₁₉-Alkylengruppe bezeichnet. Die Verbindungen der allgemeinen Formel IIa und Ilb weisen insbesondere die Eigenschaften eines WASA auf.

Weiterhin ist das bevorzugte öllösliche Umsetzungsprodukt der Komponente (K4), insbesondere das der allgemeinen Formel IIa oder IIb, ein Amid, ein Amidammoniumsalz oder ein Ammoniumsalz, in dem keine, eine oder mehrere Carbonsäuregruppen in Amidgruppen übergeführt sind.

Geradkettige oder verzweigte C₂- bis C₆-Alkylengruppen der Variablen A sind beispielsweise 1,1-Ethylen, 1,2-Propylen, 1,3-Propylen, 1,2-Butylen, 1,3-Butylen, 1,4-Butylen, 2-Methyl-1,3-propylen, 1,5-Pentylen, 2-Methyl-1,4-butylen, 2,2-Dimethyl-1,3-propylen, 1,6-Hexylen (Hexamethylen) und insbesondere 1,2-Ethylen. Vorzugsweise umfasst die Variable A 2 bis 4, insbesondere 2 oder 3 Kohlenstoffatome.

C₁- bis C₁₉-Alkylengruppen der Variablen B sind vor beispielsweise 1,2-Ethylen, 1,3-Propylen, 1,4-Butylen, Hexamethylen, Octamethylen, Decamethylen, Dodecamethylen, Tetradecamethylen, Hexadecamethylen, Octadecamethylen, Nonadecamethylen und insbesondere Methylen. Vorzugsweise umfasst die Variable B 1 bis 10, insbesondere 1 bis 4 Kohlenstoffatome.

Die primären und sekundären Amine als Umsetzungspartner für die Polycarbonsäuren zur Bildung der Komponente (K4) sind üblicherweise Monoamine, insbesondere aliphatische Monoamine. Diese primären und sekundären Amine können aus einer Vielzahl von Aminen ausgewählt sein, die - gegebenenfalls miteinander verbundene - Kohlenwasserstoffreste tragen.

Meist sind diese den öllöslichen Umsetzungsprodukten der Komponente (K4) zugrundeliegenden Amine sekundären Amine und weisen die allgemeine Formel HN(R⁸)₂ auf, in der die beiden Variablen R⁸ unabhängig voneinander jeweils geradkettige oder verzweigte C₁₀- bis C₃₀-Alkylreste, insbesondere C₁₄- bis C₂₄-Alkylreste bedeuten. Diese längerkettigen Alkylreste sind vorzugsweise geradkettig oder nur in geringem Grade verzweigt. In der Regel leiten sich die genannten sekundären Amine hinsichtlich ihrer längerkettigen Alkylreste von natürlich vorkommenden Fettsäuren bzw. von deren Derivaten ab. Vorzugsweise sind die beiden Reste R⁸ gleich.

Die genannten sekundären Amine können mittels Amidstrukturen oder in Form der Ammoniumsalze an die Polycarbonsäuren gebunden sein, auch kann nur ein Teil als Amidstrukturen und ein anderer Teil als Ammoniumsalze vorliegen. Vorzugsweise liegen nur wenige oder keine freien Säuregruppen vor. Vorzugsweise liegen die öllöslichen Umsetzungsprodukte der Komponente (K4) vollständig in Form der Amidstrukturen vor.

Typische Beispiele für derartige Komponenten (K4) sind Umsetzungsprodukte der Nitrilotriessigsäure, der Ethylendiamintetraessigsäure oder der Propylen-1,2-diamintetraessigsäure mit jeweils 0,5 bis 1,5 Mol pro Carboxylgruppe, insbesondere 0,8 bis 1,2 Mol pro Carboxylgruppe, Dioleylamin, Dipalmitinamin, Dikokosfettamin, Distearylamin, Dibehenylamin oder insbesondere Ditalgfettamin. Eine besonders bevorzugte Komponente (K4) ist das Umsetzungsprodukt aus 1 Mol Ethylendiamintetraessigsäure und 4 Mol hydriertem Ditalgfettamin.

Als weitere typische Beispiele für die Komponente (K4) seien die N,N-Dialkylammoniumsalze von 2-N',N'-Dialkylamidobenzoaten, beispielsweise das Reaktionsprodukt aus 1 Mol Phthalsäureanhydrid und 2 Mol Ditalgfettamin, wobei letzteres hydriert oder nicht hydriert sein kann, und das Reaktionsprodukt von 1 Mol eines Alkenylspirobislactons mit 2 Mol eines Dialkylamins, beispielsweise Ditalgfettamin und/oder Talgfettamin, wobei die beiden letzteren hydriert oder nicht hydriert sein können, genannt.

Weitere typische Strukturtypen für die Komponente der Klasse (K4) sind cyclische Verbindungen mit tertiären Aminogruppen oder Kondensate langkettiger primärer oder sekundärer Amine mit carbonsäurehaltigen Polymeren, wie sie in der WO 93/18115 beschrieben sind.

Als Kaltfließverbesserer der Komponente der Klasse (K5) geeignete Sulfocarbonsäuren, Sulfonsäuren oder deren Derivate sind beispielsweise die öllöslichen Carbonsäureamide und Carbonsäureester von ortho-Sulfobenzoesäure, in denen die Sulfonsäurefunktion als Sulfonat mit alkylsubstituierten Ammoniumkationen vorliegt, wie sie in der EP-A 261 957 beschrieben werden.

Als Kaltfließverbesserer der Komponente der Klasse (K6) geeignete Poly(meth)acrylsäureester sind sowohl Homo- als auch Copolymere von Acryl- und Methacrylsäureestern. Bevorzugt sind Copolymere von wenigstens zwei voneinander verschiedenen (Meth)Acrylsäureestern, die sich bezüglich des einkondensierten Alkohols unterscheiden. Gegebenenfalls enthält das Copolymer noch ein weiteres, davon verschiedenes olefinisch ungesättigtes Monomer einpolymerisiert. Das gewichtsmittlere Molekulargewicht des Polymers beträgt vorzugsweise 50.000 bis 500.000. Ein besonders bevorzugtes Polymer ist ein Copolymer von Methacrylsäure und Methacrylsäureestern von gesättigten C₁₄- und C₁₅-Alkoholen, wobei die Säuregruppen mit hydriertem Tallamin neutralisiert sind. Geeignete Poly(meth)acrylsäureester sind beispielsweise in der WO 00/44857 beschrieben.

Dem Mitteldestillat-Kraftstoff bzw. Dieselkraftstoff wird der Kaltfließverbesserer bzw. das Gemisch verschiedener Kaltfließverbesserer in einer Gesamtmenge von vorzugsweise 10 bis 5000 Gew.-ppm, besonders bevorzugt von 20 bis 2000 Gew.-ppm, stärker bevorzugt von 50 bis 1000 Gew.-ppm und insbesondere von 100 bis 700 Gew.-ppm, z.B. von 200 bis 500 Gew.-ppm, zugegeben.

### B4) Schmierfähigkeitsverbesserer

Geeignete Schmierfähigkeitsverbesserer (Lubricity Improver bzw. Friction Modifier) basieren üblicherweise auf Fettsäuren oder Fettsäureestern. Typische Beispiele sind Tallölfettsäure, wie beispielsweise in der WO 98/004656 beschrieben, und Glycerinmonooleat. Auch die in der US 6 743 266 B2 beschriebenen Reaktionsprodukte aus natürlichen oder synthetischen Ölen, beispielsweise Triglyceriden, und Alkanolaminen sind als solche Schmierfähigkeitsverbesserer geeignet.

### B5) Korrosionsinhibitoren

Geeignete Korrosionsinhibitoren sind z.B. Bernsteinsäureester, vor allem mit Polyolen, Fettsäurederivate, z.B. Ölsäureester, oligomerisierte Fettsäuren, substituierte Ethanolamine und Produkte, die unter dem Handelsnamen RC 4801 (Rhein Chemie Mannheim, Deutschland) oder HiTEC 536 (Afton Corporation) vertrieben werden.

### B6) Demulgatoren

Geeignete Demulgatoren sind z.B. die Alkali- oder Erdalkalisalze von Alkyl-substituierten Phenol- und Naphthalinsulfonaten und die Alkali- oder Erdalkalisalze von Fettsäuren, außerdem neutrale Verbindungen wie Alkoholalkoxylate, z.B. Alkoholethoxylate, Phenolalkoxylate, z.B. tert-Butylphenolethoxylat oder tert-Pentylphenolethoxylat, Fettsäuren, Alkylphenole, Kondensationsprodunkte von Ethylenoxid (EO) und Propylenoxid (PO), z.B. auch in Form von EO/PO-Blockcopolymeren, Polyethylenimine oder auch Polysiloxane.

### B7) Dehazer

Geeignete Dehazer sind z.B. alkoxylierte Phenol-Formaldehyd-Kondensate, wie beispielsweise die unter dem Handelsnamen erhältlichen Produkte NALCO 7D07 (Nalco) und TOLAD 2683 (Petrolite).

### B8) Antischaummittel

Geeignete Antischaummittel sind z.B. Polyether-modifizierte Polysiloxane, wie beispielsweise die unter dem Handelsnamen erhältlichen Produkte TEGOPREN 5851 (Goldschmidt), Q 25907 (Dow Corning) und RHODOSIL (Rhone Poulenc).

### B9) Cetanzahlverbesserer

Geeignete Cetanzahlverbesserer sind z.B. aliphatische Nitrate wie 2-Ethylhexylnitrat und Cyclohexylnitrat sowie Peroxide wie Di-tert-butylperoxid.

### B10) Antioxidantien

Geeignete Antioxidantien sind z.B. substituierte Phenole, wie 2,6-Di-tert.-butylphenol und 6-Di-tert.-butyl-3-methylphenol sowie Phenylendiamine wie N,N'-Di-sec.-butyl-p-phenylendiamin.

### B11) Metalldeaktivatoren

Geeignete Metalldeaktivatoren sind z.B. Salicylsäurederivate wie N,N'-Disalicyliden-1,2-propandiamin.

### B12) Lösungsmittel

Geeignete sind z.B. unpolare organische Lösungsmittel wie aromatische und aliphatische Kohlenwasserstoffe, beispielsweise Toluol, Xylole, "white spirit" und Produkte, die unter dem Handelsnamen SHELLSOL (Royal Dutch/Shell Group) und EXXSOL (ExxonMobil) vertrieben werden, sowie polare organische Lösungsmittel, beispielsweise Alkohole wie 2-Ethylhexanol, Decanol und Isotridecanol. Derartige Lösungsmittel gelangen meist zusammen mit den vorgenannten Additiven und Co-Additiven, die sie zur besseren Handhabung lösen oder verdünnen sollen, in den Dieselkraftstoff.

### B13) Hilfsstoffe gegen Ablagerungen in Injektoren

Es stellt eine weitere bevorzugte Ausführungsform dar, die erfindungsgemäßen Verbindungen mit weiteren Hilfsstoffen gegen interne und externe Ablagerungen in Injektoren in Dieselmotoren zu kombinieren.

Bevorzugt kann es sich dabei um Olefin-polymerisierbare Carbonsäure-Copolymere handeln, wobei das Copolymer wenigstens eine freie Carbonsäureseitengruppe enthält, oder eine mit Epoxid in Gegenwart eines Olefin-polymerisierbares Carbonsäure-Copolymeren, wobei das Copolymer wenigstens eine freie Carbonsäureseitengruppe enthält, quaternisierte Stickstoffverbindung, wobei die polymerisierbare Carbonsäure eine polymerisierbare Mono- oder Polycarbonsäure ist.

Ferner kann es sich um Copolymere, Copolymer-haltige Reaktionsprodukte, oder einer Copolymer-haltigen Teilfraktion davon handeln, wobei das Copolymer erhältlich ist durch
(1) Copolymerisation von a) mindestens einem ethylenisch ungesättigten, polymerisierbaren Polycarbonsäureanhydrid mit b) mindestens einem polymerisierbaren Olefin;
(2) anschließende Derivatisierung des Copolymers aus Schritt (1) durch teilweise oder vollständige Umsetzung der Anhydridreste des Copolymers aus Schritt (1) mit Wasser, wenigstens einer Hydroxylverbindung, wenigstens einem primären oder sekundären Amin; oder Gemischen davon, unter Bildung eines carboxylgruppenhaltigen Copolymer-Derivats; und gegebenenfalls
(3) Quaternisierung einer quaternisierbaren (insbesondere tertiären) Stickstoffverbindung mit einem Epoxid und dem Copolymer-Derivat aus Schritt (2).

Ferner kann es sich um Copolymere, Copolymer-haltige Reaktionsprodukte, oder einer Copolymer-haltigen Teilfraktion davon handeln, wobei das Copolymer erhältlich ist durch
(1) Copolymerisation von
   a) mindestens einer ethylenisch ungesättigten, polymerisierbaren Mono-oder Polycarbonsäure mit
   b) mindestens einem polymerisierbaren Olefin;
(2) anschließende Derivatisierung des Copolymers aus Schritt (1) durch teilweise Umsetzung der Carboxylreste des Copolymers mit wenigstens einer Hydroxylverbindung, wenigstens einem primären oder sekundären Amin; oder Gemischen davon, unter Bildung eines Copolymerderivats mit verringertem Gehalt an freien Carboxylgruppen; und gegebenenfalls
(3) Quaternisierung einer quaternisierbaren Stickstoffverbindung mit einem Epoxid und dem Copolymerderivat aus Schritt (2).

Ferner kann es sich um Copolymere, Copolymer-haltige Reaktionsprodukte, oder einer Copolymer-haltigen Teilfraktion davon handeln, wobei das Copolymer erhältlich ist durch
(1) Copolymerisation von
   a) mindestens eine ethylenisch ungesättigten, polymerisierbaren Mono-oder Polycarbonsäure mit
   b) mindestens einem polymerisierbaren Olefin und gegebenenfalls
(2) Quaternisierung einer quaternisierbaren Stickstoffverbindung mit einem Epoxid und dem Hydrolyseprodukt aus Schritt (1);

Derartige Copolymer-Verbindungen sind beispielsweise beschrieben in der Europäischen Patentanmeldung EP Anmeldenummer 14152991.7 der vorliegenden Anmelderin.

### C) Kraftstoffe

Das erfindungsgemäße Additiv eignet sich in hervorragender Weise als Kraftstoffzusatz und kann im Prinzip in jeglichen Kraftstoffen eingesetzt werden. Es bewirkt eine ganze Reihe von vorteilhaften Effekten beim Betrieb von Verbrennungsmotoren mit Kraftstoffen. Bevorzugt wird das erfindungsgemäße quaternisierte Additiv in Mitteldestillat-Kraftstoffen, insbesondere Dieselkraftstoffen, eingesetzt.

Gegenstand der vorliegenden Erfindung sind daher auch Kraftstoffe, insbesondere Mitteldestillat-Kraftstoffe, mit einem als Zusatzstoff zur Erzielung von vorteilhaften Effekten beim Betrieb von Verbrennungsmotoren, beispielsweise von Dieselmotoren, insbesondere von direkteinspritzenden Dieselmotoren, vor allem von Dieselmotoren mit Common-Rail-Einspritzsystemen, wirksamen Gehalt an dem erfindungsgemäßen quaternisierten Additiv. Dieser wirksame Gehalt (Dosierrate) liegt in der Regel bei 10 bis 5000 Gew.-ppm, vorzugsweise bei 20 bis 1500 Gew.-ppm, insbesondere bei 25 bis 1000 Gew.-ppm, vor allem bei 30 bis 750 Gew.-ppm, jeweils bezogen auf die Gesamtmenge an Kraftstoff.

Bei Mitteldestillat-Kraftstoffen wie Dieselkraftstoffen oder Heizölen handelt es sich vorzugsweise um Erdölraffinate, die üblicherweise einen Siedebereich von 100 bis 400°C haben. Dies sind meist Destillate mit einem 95%-Punkt bis zu 360°C oder auch darüber hinaus. Dies können aber auch so genannte "Ultra Low Sulfur Diesel" oder "City Diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.-%. Neben den durch Raffination erhältlichen mineralischen Mitteldestillat-Kraftstoffen bzw. Dieselkraftstoffen sind auch solche, die durch Kohlevergasung oder Gasverflüssigung ["gas to liquid" (GTL)-Kraftstoffe] oder durch Biomasse-Verflüssigung ["biomass to liquid" (BTL)-Kraftstoffe] erhältlich sind, geeignet. Geeignet sind auch Mischungen der vorstehend genannten Mitteldestillat-Kraftstoffe bzw. Dieselkraftstoffe mit regenerativen Kraftstoffen, wie Biodiesel oder Bioethanol. Denkbar sind ferner hydrierte Pflanzenöle (HVO) oder used kitchen oil (UKO).

Die Qualitäten der Heizöle und Dieselkraftstoffe sind beispielsweise in DIN 51603 und EN 590 näher festgelegt (vgl. auch Ullmann's Encyclopedia of Industrial Chemistry, 5. Auflage, Band A12, S. 617 ff.).

Das erfindungsgemäße quaternisierte Additiv kann neben seiner Verwendung in den oben genannten Mitteldestillat-Kraftstoffen aus fossilem, pflanzlichem oder tierischem Ursprung, die im wesentlichen Kohlenwasserstoffmischungen darstellen, auch in Mischungen aus solchen Mitteldestillaten mit Biobrennstoffölen (Biodiesel) eingesetzt werden. Derartige Mischungen werden im Sinne der vorliegenden Erfindung auch von dem Begriff "Mitteldestillat-Kraftstoff" umfasst. Sie sind handelsüblich und enthalten meist die Biobrennstofföle in untergeordneten Mengen, typischerweise in Mengen von 1 bis 30 Gew.-% insbesondere von 3 bis 10 Gew.-%, bezogen auf die Gesamtmenge aus Mitteldestillat fossilen, pflanzlichem oder tierischen Ursprungs und Biobrennstofföl.

Biobrennstofföle basieren in der Regel auf Fettsäureestern, vorzugsweise im wesentlichen auf Alkylester von Fettsäuren, die sich von pflanzlichen und/oder tierischen Ölen und/oder Fetten ableiten. Unter Alkylestern werden üblicherweise Niedrigalkylester, insbesondere C₁- bis C₄-Alkylester, verstanden, die durch Umesterung der in pflanzlichen und/oder tierischen Ölen und/oder Fetten vorkommenden Glyceride, insbesondere Triglyceride, mittels Niedrigalkoholen, beispielsweise Ethanol oder vor allem Methanol ("FAME"), erhältlich sind. Typische Niedrigalkylester auf Basis von pflanzlichen und/oder tierischen Ölen und/oder Fetten, die als Biobrennstofföl oder Komponenten hierfür Verwendung finden, sind beispielsweise Sonnenblumenmethylester, Palmölmethylester ("PME"), Sojaölmethylester ("SME") und insbesondere Rapsölmethylester ("RME"). Denkbar sind ferner hydrierte Pflanzenöle (HVO) oder used kitchen oil (UKO).

Besonders bevorzugt handelt es sich bei den Mitteldestillat-Kraftstoffen bzw. Dieselkraftstoffen um solche mit niedrigem Schwefelgehalt, das heißt mit einem Schwefelgehalt von weniger als 0,05 Gew.-%, vorzugsweise von weniger als 0,02 Gew.-%, insbesondere von weniger als 0,005 Gew.-% und speziell von weniger als 0,001 Gew.-% Schwefel.

Als Ottokraftstoffe kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

Das erfindungsgemäße quaternisierte Additiv eignet sich insbesondere als Kraftstoffzusatz in Kraftstoffzusammensetzungen, insbesondere in Dieselkraftstoffen, zur Überwindung der eingangs geschilderten Probleme bei direkteinspritzenden Dieselmotoren, vor allem bei solchen mit Common-Rail-Einspritzsystemen.

Bevorzugte Zusatzstoffe in derartige Kraftstoffen sind die nachfolgend aufgeführten speziellen Verbindungsklassen (A)und (C):
Bevorzugte Zusatzstoffe (A) sind von Bernsteinsäureanhydrid abgeleitete Verbindungen mit langkettigen Kohlenwasserstoffresten mit in der Regel 15 bis 700, vor allem 30 bis 200 Kohlenstoffatomen. Diese Verbindungen können weitere funktionelle Gruppen aufweisen, die vorzugsweise ausgewählt sind unter Hydroxy-, Amino-, Amido- und/oder Imidogruppen. Bevorzugte Additive sind die entsprechenden Derivate von Polyalkenylbernsteinsäureanhydrid, welche z. B. durch Umsetzung von Polyalkenen mit Maleinsäureanhydrid auf thermischem Weg oder über die chlorierten Kohlenwasserstoffe erhältlich sind. Das zahlenmittlere Molekulargewicht der langkettigen Kohlenwasserstoffreste liegt vorzugsweise in einem Bereich von etwa 200 bis 10.000, besonders bevorzugt 400 bis 5000, insbesondere 600 bis 3000 und speziell 650 bis 2000. Vorzugsweise leiten sich diese langkettigen Kohlenwasserstoffreste von konventionellen und insbesondere von den zuvor genannten reaktiven Polyisobutenen ab. Von besonderem Interesse als Zusatzstoffe (A) sind die Derivate von Polyalkenylbernsteinsäureanhydriden mit Ammoniak, Monoaminen, Polyaminen, Monoalkoholen und Polyolen. Zur Derivatisierung bevorzugte Polyamine umfassen Ethylendiamin, Diethylentriamin, Triethylentetramin, Tetraethylenpentamin, Propylendiamin, etc. Geeignete Alkohole umfassen einwertige Alkohole, wie Ethanol, Allylalkohol, Dodecanol und Benzylalkohol, mehrwertige Alkohole, wie Ethylenglykol, Diethylenglykol, Propylenglykol, 1,2-Butandiol, Neopentylglykol, Glycerin, Trimethylolpropan, Erythrit, Pentaerythrit, Mannitol und Sorbitol.

Als Zusatzstoffe geeignete Bernsteinsäureanhydrid-Derivate (A) sind beispielsweise in der US 3 522 179, US 4 234 435, US 4 849 572, US 4 904 401, US 5 569 644 und US 6 165 235 beschrieben, worauf hier in vollem Umfang Bezug genommen wird.

Bevorzugte Zusatzstoffe (C) sind Mannich-Addukte. Derartige Addukte werden prinzipiell durch Mannich-Umsetzung von aromatischen Hydroxylverbindungen, insbesondere Phenol und Phenolderivaten, mit Aldehyden und Mono- oder Polyaminen erhalten. Vorzugsweise handelt es sich um die Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin, Dimethylaminopropylamin, etc. Geeignete Mannich-Addukte und Verfahren zu ihrer Herstellung sind z. B. in der US 5 876 468, EP-A 831 141, EP-A 1 233 990 und EP-A 1 226 188 beschrieben, worauf hier in vollem Umfang Bezug genommen wird.

Die Zusatzstoffe (A) und (C) sowie gegebenenfalls weitere der zuvor genannten Zusatzstoffe können üblicherweise jeweils in Mengen von jeweils 0,0001 bis 1 Gew.-%, vorzugsweise 0,001 bis 0,6 Gew.-% und insbesondere 0,0015 bis 0,4 Gew.-%, bezogen auf die Gesamtmenge der Kraftstoffzusammensetzung, eingesetzt werden.

Die Erfindung wird nun anhand der folgenden Ausführungsbeispiele näher beschrieben. Insbesondere die im Folgenden genannten Testmethoden sind Teil der allgemeine Offenbarung der Anmeldung und nicht auf die konkreten Ausführungsbeispiele beschränkt.

### Experimenteller Teil:

### A. Allgemeine Testmethoden

### Motorentest

### 1. XUD9 Test - Bestimmung der Flow Restriction

Die Durchführung erfolgt nach den Standardbestimmungen gemäß CEC F-23-1-01.

### 2. DW10 Test - Bestimmung des Leistungsverlusts durch Injektorablagerungen im Common Rail Dieselmotor

### 2.1. DW10- KC - Keep Clean Test

Der Keep Clean Test lehnt sich an die CEC Test Prozedur F-098-08 Issue 5 an. Dabei kommen der gleiche Testaufbau und Motorentyp (PEUGEOT DW10 ) wie in der CEC Prozedur zum Einsatz.

### Änderung und Besonderheiten:

Bei den Versuchen kamen gereinigte Injektoren zum Einsatz. Die Reinigungsdauer im Ultraschallbad in 60°C Wasser + 10% Superdecontamine (Intersciences, Brüssel) betrug 4h.

### Testlaufzeiten:

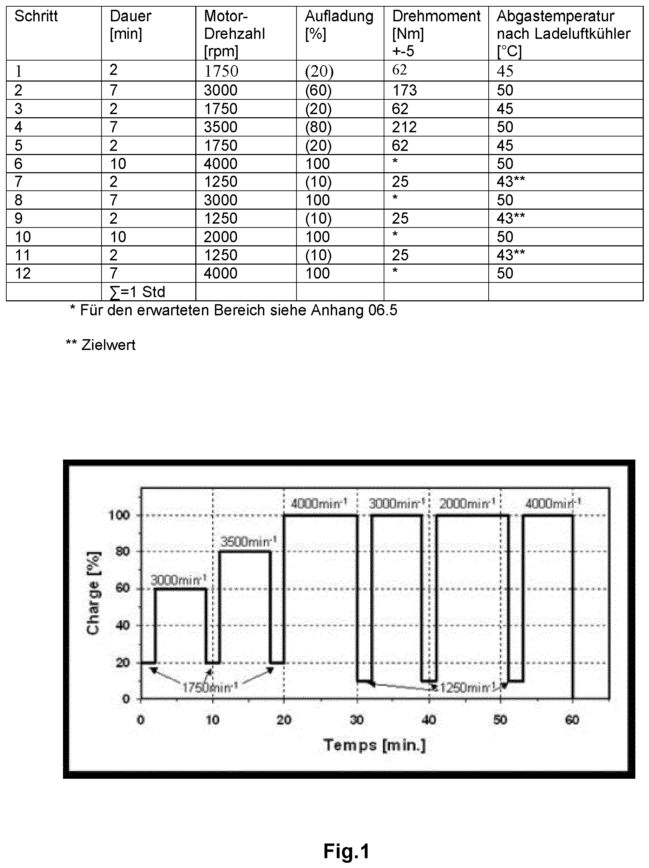

Der Testzeitraum betrug, wenn nicht anders angegeben, 12h ohne Abstellphasen. Der in Figur 1 dargestellte einstündige Testzyklus aus der CEC F-098-08 wurde dabei 12-mal durchfahren.

### Leistungsbestimmung (wenn nicht anders angegeben):

Die Anfangsleistung P0,KC [kW] wird aus dem gemessenen Drehmoment bei 4000/min Volllast direkt nach Teststart und Warmlauf des Motors berechnet. Die Vorgehensweise ist in der Issue 5 der Testprozedur (CEC F-98-08) beschrieben. Dabei wird der gleiche Testaufbau und der Motorentyp PEUGEOT DW10 verwendet.

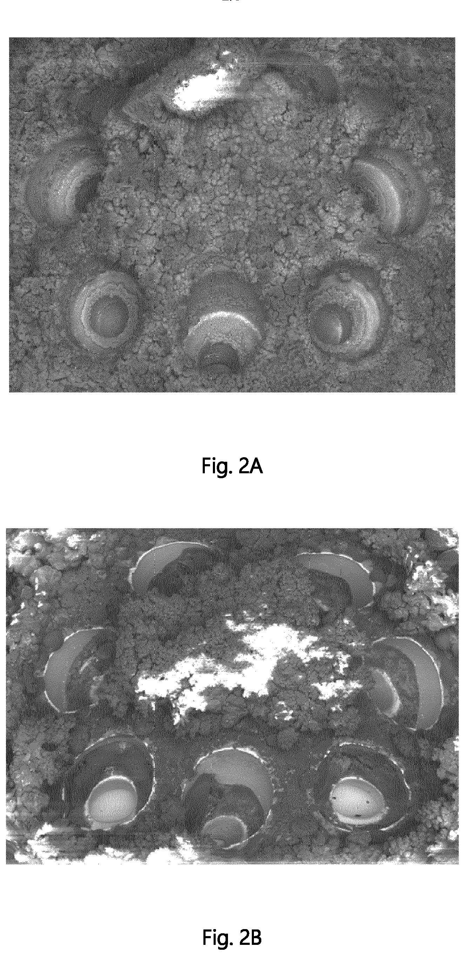

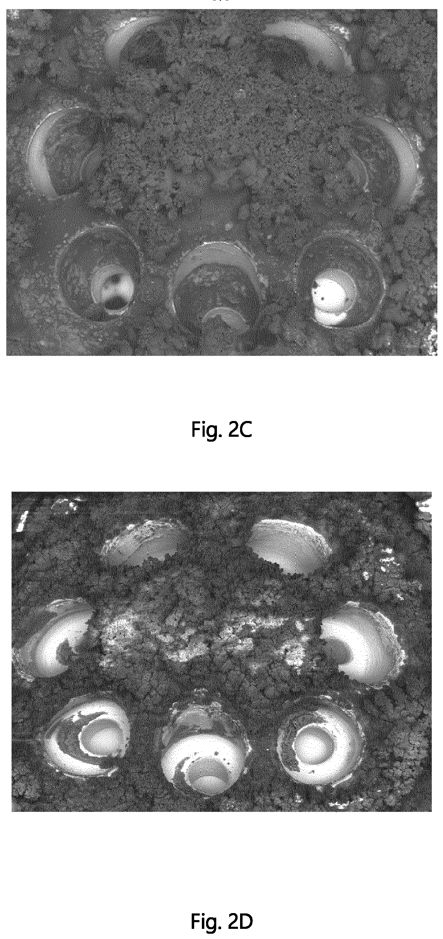

Die Endleistung (Pend,KC) wird im 12. Zyklus in Stufe 12, (siehe Tabelle, Figur 2) bestimmt. Auch hier ist der Betriebspunkt 4000/min Volllast. Pend,KC [kW] errechnet sich aus dem gemessenen Drehmoment.

Der Leistungsverlust im KC Test wird wie folgt berechnet: $Powerloss , KC \left[%\right] = \left(1-\frac{Pend , KC}{P 0 , KC}\right) ∗ 100$

### 2.2. DW10-Dirty Up - Clean Up-(DU-CU)

Der DU-CU Test lehnt sich an die CEC Test Prozedur F-098-08 Issue 5 an. Die Vorgehensweise ist in der Issue 5 der Testprozedur (CEC F-98-08) beschrieben. Dabei wird der gleiche Testaufbau und der Motorentyp PEUGEOT DW10 verwendet.

Der DU - CU Test besteht aus zwei einzelnen Tests, die hintereinander gefahren werden. Der erste Test dient zur Ablagerungsbildung (DU), der zweite zum Entfernen der Ablagerungen (CU). Nach dem DU wird der Leistungsverlust (Powerloss) bestimmt. Nach Ende des DU Laufs wird der Motor für mindestens 8 Stunden nicht betrieben und auf Umgebungstemperatur abgekühlt. Danach wird mit dem CU Kraftstoff der CU gestartet, ohne die Injektoren auszubauen und zu reinigen. Die Ablagerungen und der powerloss gehen im Idealfall im CU-Testverlauf zurück.

### Änderung und Besonderheiten:

Gereinigte Injektoren wurden vor jedem DU Test in den Motor eingebaut. Die Reinigungsdauer im Ultraschallbad bei 60°C ,Wasser + 10% Superdecontamine (Intersciences, Brüssel) betrug 4h.

### Testlaufzeiten (wenn nicht anders angegeben):

Der Testzeitraum betrug 4,28 Stunden für den DU und 8h oder 12h für den CU. Der Motor wurde im DU und CU Test ohne Abstellphasen betrieben.

Der in Figur 1 dargestellte einstündige Testzyklus aus der CEC F-098-08 wurde dabei jeweils 12-mal durchfahren.

Bei manchen DU Versuchen wurde eine beschleunigte Prozedur angewendet. Dafür wurde kein Testzyklus aus der CEC F-098-08 verwendet, sondern der Motor wurde bei 4000/min Volllast betrieben mit erhöhter Menge von Zn (3 ppm anstatt 1ppm in CEC F-098-08 Prozedur).

### Leistungsbestimmung:

Die Anfangsleistung P0,du [kW] wird aus dem gemessenen Drehmoment bei 4000/min Volllast direkt nach Teststart und Warmlauf des Motors berechnet. Die Vorgehensweise ist ebenfalls in der Issue 5 der Testprozedur beschrieben.

Die Endleistung (Pend,du) wird im 12. Zyklus in Stufe 12, (siehe Tabelle oben) bestimmt. Auch hier ist der Betriebspunkt 4000/min Volllast. Pend,du [kW] errechnet sich aus dem gemessenen Drehmoment.

Der Leistungsverlust im DU wird wie folgt berechnet $Powerloss , du \left[%\right] = \left(1-\frac{Pend , du}{P 0 , du}\right) ∗ 100$

### Clean up

Die Anfangsleistung **P0,cu** [kW] wird aus dem gemessenen Drehmoment bei 4000/min Volllast direkt nach Teststart und Warmlauf des Motors im CU berechnet. Die Vorgehensweise ist ebenfalls in der Issue 5 der Testprozedur beschrieben.

Die Endleistung (Pend,cu) wird im 12. Zyklus in Stufe 12, (siehe Tabelle Figur 2) bestimmt. Auch hier ist der Betriebspunkt 4000/min Volllast. Pend,cu [kW] errechnet sich aus dem gemessenen Drehmoment.

Der Leistungsverlust im CU-Test wird wie folgt berechnet (negative Zahl beim power-loss im cu-Test bedeutet Leistungszuwachs) $Powerloss \left(DU, CU\right) \left[%\right] = \left(\frac{Pend , du - pend , cu}{P 0 , du}\right) ∗ 100$

Als Kraftstoff wurde ein handelsüblicher Dieselkraftstoff der Fa. Haltermann (RF-06-03) eingesetzt. Diesem wurden zur künstlichen Anregung der Bildung von Ablagerungen an den Injektoren 1 Gew.-ppm Zink in Form einer Zink-Didodecanoat-Lösung zugesetzt.

### 3. IDID Test - Bestimmung der Additivwirkung gegen interne Injektorablagerungen

Die Bildung von Ablagerungen im Inneren des Injektors wurde anhand der Abweichungen der Abgastemperaturen der Zylinder am Zylinderausgang beim Kaltstart des DW10-Motors charakterisiert.

Zur Förderung der Bildung von Ablagerungen wurden dem Kraftstoff 1 mg/l Na in Form eines Salzes einer organischen Säure (Natriumnaphthenat), 20 mg/l Dodecenylbernsteinsäure und 10 mg/l Wasser zugegeben.

Der Test wird als dirty-up-clean-up Test (DU-CU) durchgeführt.

DU-CU lehnt sich an die an die CEC Test Prozedur F-098-08 Issue 5 an.

Der DU - CU Test besteht aus zwei einzelnen Tests, die hintereinander gefahren werden. Der erste Test dient zur Ablagerungsbildung (DU), der zweite zum Entfernen der Ablagerungen (CU).

Nach dem DU Lauf wird nach einer mindestens achtstündigen Stillstands-Phase ein Kaltstart des Motors mit anschließendem 10-minütigen Leerlauf durchgeführt.

Danach wird mit dem CU Kraftstoff der CU gestartet, ohne die Injektoren auszubauen und zu reinigen. Nach dem CU Lauf über 8h wird nach einer mindestens achtstündigen Stillstands-Phase ein Kaltstart des Motors mit anschließendem 10-minütigen Leerlauf durchgeführt. Die Auswertung erfolgt durch den Vergleich der Temperaturverläufe für die einzelnen Zylinder nach Kaltstart des du und des CU-Laufs.

Der IDID-Test zeigt die interne Ablagerungsbildung im Injektor an. Als Kenngröße dient bei diesem Test die Abgastemperatur der einzelnen Zylinder. Bei einem Injektorsystem ohne IDID erhöhen sich die Abgastemperaturen der Zylinder gleichmäßig. Bei vorhandenem IDID erhöhen sich die Abgastemperaturen der einzelnen Zylinder nicht gleichmäßig und weichen voneinander ab.

Die Temperatursensoren befinden sich hinter dem Zylinderkopfaustritt im Abgaskrümmer. Signifikante Abweichung der einzelnen Zylindertemperaturen (z.B. > 20°C) zeigen das Vorliegen von internen Injektorablagerungen (IDID) an.

Die Tests (DU und CU) werden mit jeweils 8h Laufzeit durchgeführt. Der einstündige Testzyklus aus der CEC F-098-08 (siehe Figur 3) wird dabei jeweils 8-mal durchfahren. Bei Abweichungen der einzelnen Zylindertemperaturen von größer 45°C zum Mittelwert aller 4 Zylinder wird der Test vorzeitig abgebrochen.

Änderung und Besonderheiten: Gereinigte Injektoren wurden vor jedem DU-Testbeginn eingebaut. Die Reinigungsdauer im Ultraschallbad bei 60°C Wasser + 10% Superdecontamine betrug 4h.

### B. Herstellungsbeispiele:

### Herstellungsbeispiele 1 bis 4: Quaternisierung von tertiären Fettaminen mit Propylenoxid in Gegenwart verschiedener Hydrocarbyl-substituierter Bernsteinsäuren

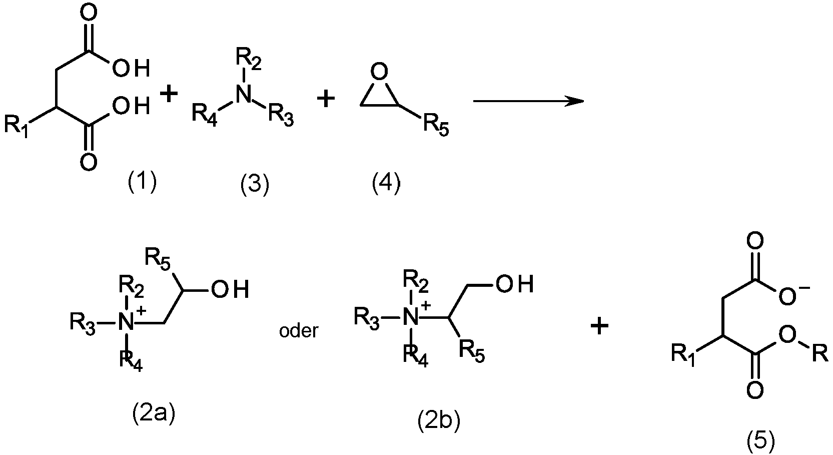

R₁ steht dabei für langkettiges Hydrocarbyl; R₂, R₃ und R₄ entsprechen Rₐ, R_{b} und R_{c} sind wie oben definiert; R₅ entspricht R_{d} wie oben definiert; und R steht für H oder ist ein durch Veresterung mit dem Epoxid erzeugter Rest wie z.B. -CH₂CH(R₅)OH

### a) Verwendete Reagenzien:

Polyisobutylenbernsteinsäureanhydrid (PIBSA, Glissopal^{®} SA, Fa. BASF):
Hergestellt aus Maleinsäureanhydrid und Polyisobuten 1000 in bekannter Weise. Soweit nicht anders angegeben wurden für die erfindungsgemäßen Herstellungsbeispiele Qualitäten mit einem Bismaleinierungsgrad von 10 bis 20% und Verseifungzahlen im Bereich von 84-95 mg KOH/g verwendet. Zur Herstellung von Polyisobutylenbernsteinsäure wurde Polyisobutylenbernsteinsäureanhydrid entsprechend der Verseifungszahl mit der äquimolaren Menge Wasser versetzt und bei einer Temperatur von 80°C hydrolysiert. Beispielsweise ergab die Umsetzung von Polyisobutylenbernsteinsäureanhydrid (Verseifungszahl 85,6 mg KOH/g) nach 4 h Reaktionszeit bei 80°C ein Reaktionsprodukt, das eine Säurezahl von 83,9 mg KOH/g aufwies. Die Bildung der Polyisobutylenbernsteinsäure wurde per IR-Spektroskopie bestätigt (1711 cm⁻¹).

In analoger Weise wurden Tetrapropenylbernsteinsäureanhydrid (CAS 26544-38-7) und ein gemischtes i-Hexadecenyl/i-Octadecenylbernsteinsäureanhydrid (CAS 32072-96-1 und 28777-98-2) der Firma Pentagon zu den entsprechenden Bernsteinsäurederivaten hydrolysiert.

Cocoyldimethylamin: (N,N-Dimethyl-N-C12/14-amin, CAS 68439-70-3 bzw. 112-18-5) mit einer Gesamtaminzahl von 246 mg KOH/g.

N-Methyl-N,N-Ditalgfettamin: Armeen^{®} M2HT von Akzo Nobel, CAS 61788-63-4, mit einer Gesamtaminzahl von 108 mg KOH/g.

### Weiter wurde verwendet:

N,N-Dimethylhexadecylamin (n-C₁₆H₃₃NMe₂, CAS 112-69-6, Fa. Aldrich). Tridecylamin (verzweigt; Isomerengemisch, CAS 86089-17-0) der Fa. BASF.

N,N-Dimethyl-1,3-diaminopropan (DMAPA, CAS 109-55-7) der Fa. BASF.

2-Ethylhexanol und 2-Propylheptanol der Fa. BASF.

Solvent Naphtha naphthalene depleted (ND): Solvesso^{™} 150 ND der Fa. Exxon Mobil.

### b) Allgemeine Synthesevorschrift

In einem 2 I Autoklaven wird eine Lösung des tert. Amins (1 Äq. entsprechend der Gesamtaminzahl) und des Alkylenbernsteinsäurederivats (1 Äq. entsprechend der Säurezahl) in dem angegebenen Lösungsmittel (2-Ethylhexanol, wenn nicht anders angegeben) vorgelegt. Die Menge an Lösungsmittel und die Ansatzgröße werden so gewählt, dass das Endprodukt einen Aktivgehalt von 50% und der Reaktor einen Füllgrad von ca. 70% aufweist. Anschließend wird dreimal mit N₂ gespült, ein Vordruck von ca. 2 bar N₂ eingestellt und die Temperatur auf 50°C erhöht. Das angegebene Alkylenoxid, wenn nicht anders angegeben Propylenoxid (2 Äq.) wird innerhalb von 1 h zudosiert. Anschließend wird 15 h bei 50°C nachgerührt, auf 25°C abgekühlt, mit N₂ gespült und der Reaktor entleert. Das Produkt wird in einen 2 l Doppelmantelreaktor überführt und überschüssiges Alkylenoxid wird durch Einleiten eines N₂-Stromes (10 l/h) unter Vakuum (70 mbar) bei 50°C für 6 h entfernt. ¹H-NMR (CDCl₃) bestätigt die Quaternierung (δ = 3.3 ppm, Singulett, R₂N(CH₃)₂ bzw. R₃NCH₃).

### c) Durchgeführte Versuche

Unter Befolgung der obigen Synthesevorschrift wurden die folgenden Quaternisierungen mit Propylenoxid durchgeführt

| **Herstellungsbeispiel** | **tert. Amin** | **Hydrocarbyl-substituierte Bernsteinsäure** |
|---|---|---|
| **1** | Cocoyldimethylamin | Polyisobutylenbernsteinsäure |
| **2** | Cocoyldimethylamin | Tetrapropenylbernsteinsäure |
| **3** | Cocoyldimethylamin | i-Hexadecenyl/i-Octadecenylbernsteinsäure |
| **4** | N-Methyl-N,N-Ditalgfettamin | Tetrapropenylbernsteinsäure |
| **6** | n-C₁₆H₃₃NMe₂ | Polyisobutylenbernsteinsäure |
| **7** | n-C₁₆H₃₃NMe₂ | Polyisobutylenbernsteinsäure |
| **8** | n-C₁₆H₃₃NMe₂ | Polyisobutylenbernsteinsäure |
| **9** | n-C₁₆H₃₃NMe₂ | Polyisobutylenbernsteinsäure |
| **10** | n-C₁₆H₃₃NMe₂ | Polyisobutylenbernsteinsäure |
| **11** | n-C₁₆H₃₃NMe₂ | Polyisobutylenbernsteinsäure |
| **12** | n-C₁₆H₃₃NMe₂ | Polyisobutylenbernsteinsäure |
| **13** | Cocoyldimethylamin | Polyisobutylenbernsteinsäure |
| **16** | N,N-Dimethylethanolamin*15 PO | Tetrapropenylbernsteinsäure |
| **17** | PIBSA-DMAPA Succinimid | Tetrapropenylbernsteinsäure |

Anmerkungen zu Herstellungsbeispiel:

| | |
|---|---|
| Nr. 7: | 2-Propylheptanol wurde anstelle von 2-Ethylhexanol als Lösungsmittel verwendet. |
| Nr. 8: | 2-Ethylhexanol/Solvent Naphtha ND 1:1 (w/w) wurde anstelle von 2-Ethylhexanol als Lösungsmittel verwendet. |
| Nr. 9: | Ethylenoxid (1,5 Äq.) anstelle von Propylenoxid wurde verwendet. 2-Propylheptanol wurde anstelle von 2-Ethylhexanol als Lösungsmittel verwendet. |
| Nr. 10: | Das verwendete PIBSA (aus Maleinsäureanhydrid und Polyisobuten 1000) hatte einen Bismaleinierungsgrad von 32% und eine Verseifungszahl von 112,5 mg KOH/g. |
| Nr. 11: | 1,5 Äq. Propylenoxid wurden verwendet. |
| Nr. 12: | 1,1 Äq. Propylenoxid wurden verwendet. |
| Nr. 13: | 2-Propylheptanol wurde anstelle von 2-Ethylhexanol als Lösungsmittel verwendet. Das verwendete PIBSA (aus Maleinsäureanhydrid und Polyisobuten 550) hatte eine Verseifungszahl von 142,5 mg KOH/g. |
| Nr. 16: | 2-Propylheptanol wurde als Lösungsmittel verwendet, als Amin wurde ein Polyetheramin erhalten durch 15-fache Propoxylierung von N,N-Dimethylethanolamin (Herstellung siehe Synthesebeispiel 1 der WO 2013/064689 A1) eingesetzt. |
| Nr. 17: | 2-Propylheptanol wurde als Lösungsmittel verwendet, als Amin wurde das Kondensationprodukt von Polyisobutylenbernsteinsäure (PIBSA) und DMAPA eingesetzt, siehe Herstellungsbeispiel 1 der WO 2013/000997 A1. |

### Herstellungsbeispiel 5: Quaternisierung von Trimethylamin mit Dodecenenoxid in Gegenwart von Tetrapropenylbernsteinsäuren

### Reagenzien: Dodecenoxid (CAS 2855-19-8) von Aldrich, Trimethylamin (wasserfrei, CAS 75-50-3) von BASF

In einem mit N₂ inertisierten 2 l Autoklaven wird eine Lösung von Trimethylamin (47,2 g, 0,8 mol) und Dodecenoxid (147,2 g, 0,8 mol) in 2-Ethylhexanol (194,4 g) vorgelegt. Anschließend wird die Temperatur auf 40°C erhöht. Eine Lösung von Tetrapropenylbernsteinsäure (252,8 g, 0,8 mol) in 2-Ethylhexanol (252,8 g) wird innerhalb von 1,5 h zudosiert. Anschließend wird 15 h bei 40°C nachgerührt. Flüchtige Bestandteile werden durch Einleiten eines N₂-Stromes bei 40°C entfernt, anschließend wird der Reaktor entleert. ¹H-NMR (CDCl₃) bestätigt die Quaternierung (δ = 3.3 ppm, Singulett, RN(CH₃)₃).

### Herstellungsbeispiel 14: Synthese von iC₁₃NMe₂

Tridecylamin (140,2 g) wird bei Raumtemperatur vorgelegt und unter Rühren innerhalb von 15 min mit Ameisensäure (166,7 g) versetzt. Das Reaktionsgemisch wird auf 45°C erwärmt und wässrige Formaldehyd-Lösung (37%; 132,7 g) wird unter CO₂-Entwicklung innerhalb von 25 min zugetropft. Anschließend wird 23 h bei 80°C nachgerührt. Nach Abkühlen auf Raumtemperatur wird Salzsäure (32%; 121,5 g) unter Rühren hinzugegeben. Es wird 3 h bei Raumtemperatur nachgerührt und das Wasser am Rotationsverdampfer im Vakuum entfernt. Das Produktgemisch wird mit 500 ml Wasser versetzt und mit 50%iger Natronlauge wird das Amin freigesetzt. Es wurde zweimal mit Methyl-tert-butylether extrahiert, die vereinigten organischen Phasen wurden über Natriumsulfat getrocknet und das Lösungsmittel wurde am Rotationsverdampfer entfernt. Das Produkt (143,5 g) zeigte eine Gesamtaminzahl von 228 mg KOH/g mit 94% tert. Amin.

### Herstellungsbeispiel 15: Quaternierung von iC₁₃NMe₂ mit Propylenoxid/ Tetrapropenylbernsteinsäure

Nach der allgemeinen Synthesevorschrift wurden iC₁₃NMe₂ (Herstellungsbeispiel 14), Tetrapropenylbernsteinsäure und Propylenoxid in 2-Propylheptanol anstelle von 2-Ethylhexanol umgesetzt.

### Vergleichsbeispiel 1: Inventive Example 3 der GB 2496514

Dimethyloctadecyl-(2-hydroxyhexyl)ammoniumacetat wird durch Quaternierung von n-C₁₈H₃₇NMe₂ mit Hexenoxid/Essigsäure erhalten. Im Gegensatz zu allen erfindungsgemäßen Beispielen trübt dieses Produkt als 50%ige Lösung in 2-Ethylhexanol bei Lagerung bei Raumtemperatur über einen Zeitraum von 1 Woche ein.

### Vergleichsbeispiel 2: Quaternierung von Cocoyldimethylamin mit Propylenoxid/Ölsäure

Analog zur allgemeinen Synthesevorschrift wird in einem 2 l Autoklaven eine Lösung von Cocoyldimethylamin (1 Äq.) und Ölsäure (1 Äq.) in 2-Ethylhexanol vorgelegt. Die Menge an 2-Ethylhexanol und die Ansatzgröße werden so gewählt, dass das Endprodukt einen Aktivgehalt von 50% und der Reaktor einen Füllgrad von ca. 70% aufweist. Anschließend wird dreimal mit N₂ gespült, ein Vordruck von ca. 2 bar N₂ eingestellt und die Temperatur auf 50°C erhöht. Propylenoxid (2 Äq.) wird innerhalb von 1 h zudosiert.

Anschließend wird 15 h bei 50°C nachgerührt, auf 25°C abgekühlt, mit N₂ gespült und der Reaktor entleert. Das Produkt wird in einen 2 l Doppelmantelreaktor überführt und überschüssiges Propylenoxid wird durch Einleiten eines N₂-Stromes (10 l/h) unter Vakuum (70 mbar) bei 50°C für 6 h entfernt.

### Vergleichsbeispiel 3: Quaternierung von Cocoyldimethylamin mit Propylenoxid/Ölsäure

Analog zur allgemeinen Synthesevorschrift wird in einem 2 l Autoklaven eine Lösung von Cocoyldimethylamin (1 Äq.) und Ölsäure (2 Äq.) in 2-Ethylhexanol vorgelegt. Die Menge an 2-Ethylhexanol und die Ansatzgröße werden so gewählt, dass das Endprodukt einen Aktivgehalt von 50% und der Reaktor einen Füllgrad von ca. 70% aufweist. Anschließend wird dreimal mit N₂ gespült, ein Vordruck von ca. 2 bar N₂ eingestellt und die Temperatur auf 50°C erhöht. Propylenoxid (2 Äq.) wird innerhalb von 1 h zudosiert. Anschließend wird 15 h bei 50°C nachgerührt, auf 25°C abgekühlt, mit N₂ gespült und der Reaktor entleert. Das Produkt wird in einen 2 l Doppelmantelreaktor überführt und überschüssiges Propylenoxid wird durch Einleiten eines N₂-Stromes (10 l/h) unter Vakuum (70 mbar) bei 50°C für 6 h entfernt.

### Analysenbeispiel 1

### a) Bestimmung des Quaternierungsgrades:

Quaternierungsgrade werden per ¹H-NMR-Spektroskopie bestimmt. Dazu wird das entsprechende Lösungsmittel mit einer Kugelrohrdestille (60°C, p = 10⁻³ mbar, 3 h) entfernt. Zur Bestimmung des Quaternierungsgrades wird der Alkyteil gegen die Signale des quaternierten Produktes RCH₂NMe₂CH₂CH(OH)R' integriert. Die Quotienten aus den Integralen der Signale des quaternierten Produktes und der entsprechenden theoretischen Werte multipliziert mit 100% ergeben den Quaternierungsgrad. Die Werte für die verschiedenen Signale werden dabei gemittelt. Reste an Lösungsmittel (Dublett bei δ = 3.55 ppm für HOCH₂CHRR') werden berücksichtigt.

| Nr. | Synthese nach | Quaternierungsgrad [%] |
|---|---|---|
| 1 | Vergleichsbeispiel 1 | 71 |
| 2 | Vergleichsbeispiel 2 | 59 |
| 3 | Herstellungsbeispiel 1 | 99 |
| 4 | Herstellungsbeispiel 3 | 92 |
| 5 | Herstellungsbeispiel 7 | 99 |
| 6 | Herstellungsbeispiel 8 | 90 |
| 7 | Herstellungsbeispiel 11 | 85 |
| 8 | Vergleichsbeispiel 3 | 79 |

Das ¹H-NMR-Spektrum von Vergleichsbeispiel 2 zeigt zudem ein Signal bei δ = 3.98 ppm (dd, J = 1.0, 6.0 Hz), das auf Esterbildung schließen lässt. Integration des Signales zeigt die Bildung von 29% des Veresterungsproduktes aus Ölsäure und Propylenoxid. Ein Signal bei δ = 2.21 ppm (s) lässt auf unreagiertes Cocoyldimethylamin schließen.

Das erfindungsgemäße Verfahren der Quaternierung von tertiären Fettaminen mit Alkylenoxiden in Gegenwart von Alkylidenbernsteinsäuren liefert überraschenderweise deutlich höhere Quaternierungsgrade als die Vergleichsbeispiele, in denen Monocarbonsäuren wie Essigsäure oder Ölsäure eingesetzt werden.

### b) Thermogravimetrie

Für die thermogravimetrische Analyse wurde das entsprechende Lösungsmittel mit einer Kugelrohrdestille (60-70°C, p = 10⁻³ mbar, 3 h) entfernt. Die Thermogravimetrie wurde von 30°C bis 900°C gemessen mit einer Temperatursteigerung von 20°C/min. unter Stickstoffstoffatmosphäre bei einer Flussrate von 60mL/min. Folgende Massenänderungen (TG) bei 350°C wurden bestimmt:

| Nr | Synthese nach | Massenänderung (TG) bei 350°C |
|---|---|---|
| 1 | Herstellungsbeispiel 1 | 17% |
| 2 | Herstellungsbeispiel 7 | 34% |

### C. Anwendungsbeispiele:

In den folgenden Anwendungsbeispielen werden die Additive entweder als Reinsubstanz (so wie in obigen Herstellungsbeispielen synthetisiert) oder in Form eines Additiv-Paketes eingesetzt.

### Anwendungsbeispiel 1: Bestimmung der Additivwirkung auf die Bildung von Ablagerungen in Dieselmotor-Einspritzdüsen

DW10 Test nach CEC F-098-08
Kraftstoff: Sommerdiesel, ohne Leistungsadditive gemäß EN 590 B7

### Teil 1: Dirty up (DU)

Zn Gehalt im Kraftstoff: 3mg/kg,
Dauer: 12 Stunden Non-Stop
Leistung (t=0 h) = 98.2kW (bei Testbeginn)
Leistung (t=12 h) = 89,9 kW ( bei Ende DU)
Leistungsverlust (t = 12h) = 8,5%

### Teil 2: Clean up (CU)

Zn Gehalt im Kraftstoff: 1 mg/kg,
Dauer: 6 Stunden Non-Stop
Additiv: 50ppm aktiver Bestandteil von Additiv gemäß Herstellungsbeispiel 2
Leistung (t=0 h) = 89.2kW (bei Testbeginn)
Leistung (t=6 h) = 97,5 kW (bei Ende CU)

Bezogen auf den Ausgangswert der Leistung (98,2 kW) beobachtet man eine nach 6 Stunden eine Anstieg der Leistung von 90.8% auf 99,3%.

Alle Angaben beziehen sich auf Gewichts-ppm (mg/kg), sofern nicht anders angegeben.

### Anwendungsbeispiel 2: DW10 Zn Motortest (clean up)

Der Test wurde mit einem Peugeot DW10 Motor durchgeführt, der nach Standard-CEC F-98-08 Prozedur verwendet wird, wobei in Abwandlung jedoch schärfere Bedingungen im Dirty-up Teil verwendet wurden sowie ein Lauf unter Volllast anstelle der CEC F-98-08 Prozedur. Der Test bestand aus zwei Teilen:

### I. Dirty up:

Die schärferen Bedingungen erlauben die deutlich schnellere Bildung von Injektorablagerungen und damit eine schnellere power-loss Bestimmung als unter Standard CEC F-98-08 Bedingungen: Der Motor wurde 4,28 h bei Volllast (4000 rpm) mit Grundkraftstoff nach EN590 B7, ohne Leistungsadditive, enthaltend 3 mg/kg Zn, betrieben. Die Ergebnisse sind in der nachfolgenden Tabelle zusammengefasst.

Der Leistungsverlust im DU wird wie folgt berechnet $Powerloss , du \left[%\right] = \left(1-\frac{Pend , du}{P 0 , du}\right) ∗ 100$

### II. Clean up:

Für clean up-Test, verkürzt auf 8 h nach CEC F-98-08 Prozedur mit 1 ppm Zn in Form einer Zink-Didodecanoat-Lösung und Grundkraftstoff nach EN590 B7 Kraftstoff, ohne Leistungsadditive, enthaltend erfindungsgemäße Zusätze wurden die in folgender Tabelle zusammengefassten Resultate erzielt.

Der Leistungsverlust im CU-Test wird wie folgt berechnet (negative Zahl beim power-loss im CU-Test bedeutet Leistungszuwachs) $Powerloss \left(DU, CU\right) \left[%\right] = \left(\frac{Pend , du - PEND , CU}{P 0 , du}\right) ∗ 100$

| Test | Zusatz | Leistung vor Test, kW | Leistung nach Test, kW | Leistungsverlust, % |
|---|---|---|---|---|
| Dirty up (beschleunigtes Verfahren), Volllast | 3 ppm Zn | 98,3 | 92,9 | 5,5 |
| Clean up, 8 Stunden, verkürztes Verfahren nach CEC F-98-08 | 1 ppm Zn und 33 ppm Probe gemäß Herstellungsbeispiel 1 | 93,0 | 96,4 | -3,6 |
| | | | | |
| Dirty up (be- | 3 ppm Zn | 94,8 | 90,5 | 4,5 |
| schleunigtes Verfahren), Volllast | | | | |
| Clean up, 8 Stunden, verkürztes Verfahren nach CEC F-98-08 | 1 ppm Zn und 48 ppm Probe gemäß Herstellungsbeispiel 7 | 90,0 | 93,3 | -3,0 |
| | | | | |
| Dirty up (beschleunigtes Verfahren), Volllast | 3 ppm Zn | 94,7 | 90,8 | 4,1 |
| Clean up, 8 Stunden, verkürztes Verfahren nach CEC F-98-08 | 1 ppm Zn und 60 ppm Probe gemäß Herstellungsbeispiel 7 | 90,4 | 94,5 | -3,9 |
| | | | | |
| Dirty up (beschleunigtes Verfahren), Volllast | 3 ppm Zn | 93,8 | 90,2 | 3,8 |
| Clean up, 8 Stunden, verkürztes Verfahren nach CEC F-98-08 | 1 ppm Zn und 60 ppm Probe gemäß Herstellungsbeispiel 13 | 91,8 | 94,6 | -4,7 |

Die erfindungsgemäß beschriebenen Verbindungen sind wirksam gegen die Bildung von Ablagerung in Direkteinspritzermotoren, wie Peugeot DW10, bei Prüfung nach CEC F-98-08, und sind in der Lage, die gebildeten Ablagerungen früher zu entfernen.

### Anwendungsbeispiel 3: XUD9 Motortest (keep clean)

Der Test wurde durchgeführt gemäß der Standardprozedur CEC F-023-01 mit einem Peugeot Motor XUD9 mit Diesel-Grundkraftstoff nach EN590 B7, ohne Leistungsadditive.

| Zusatz | Flussverminderung bei 0,1 mm Nadelhub, % |
|---|---|
| Kein Zusatz | 76,8 |
| 20 ppm Probe gemäß Herstellungsbeispiel 1 | 67,3 |
| 100 ppm Probe gemäß Herstellungsbeispiel 1 | 19,5 |
| 24 ppm Probe gemäß Herstellungsbeispiel 3 | 46,7 |
| 36 ppm Probe gemäß Herstellungsbeispiel 3 | 24,0 |
| 36 ppm Probe gemäß Herstellungsbeispiel 7 | 46,8 |
| 24 ppm Probe gemäß Herstellungsbeispiel 6 | 52,0 |

Die erfindungsgemäß beschriebenen Verbindungen sind wirksam gegen die Bildung von Ablagerung in indirekten Einspritzermotoren, wie Peugeot XUD9, bei Prüfung nach CEC F-023-01, und sind in der Lage, die gebildeten Ablagerungen früher zu entfernen.

### Anwendungsbeispiel 4: CFPP EN 116 Test

Der Test wurde gemäß dem DIN EN 116 Standardverfahren zur Bestimmung des Kaltfließverhaltens (cold flow filter plugging point, CFPP) mit Winterdiesel Grundkraftstoff nach EN590 B7, ohne Leistungsadditive durchgeführt.

| Zusatz | CFPP Temperatur gemäß EN 116, °C |
|---|---|
| Kein Zusatz | -27 |
| 40 ppm Probe gemäß Herstellungsbeispiel 1 | -28 |
| 80 ppm Probe gemäß Herstellungsbeispiel 1 | -26 |
| 40 ppm Probe gemäß Herstellungsbeispiel 3 | -27 |
| 120 ppm Probe gemäß Herstellungsbeispiel 3 | -26 |
| 70 ppm Probe gemäß Herstellungsbeispiel 7 | -29 |
| 80 ppm von Probe gemäß Vergleichsbeispiel 1 | -22 |

Die in dieser Erfindung beschriebenen Verbindungen verursachen keine Verschlechterung der Kaltfließeigenschaften und keine Verschlechterung des CFPP-Wertes gemessen nach der EN116 Norm.

### Anwendungsbeispiel 5: Motorölverträglichkeit

Der Test wurde durchgeführt nach der Norm DGMK 531 1-A mit Grundkraftstoff nach EN590 B7 ohne Leistungsadditive und Wintershall Multi-Rekord Top 15W-40 Motoröl. Das zu testende Produkt wurde mit Motoröl vermischt und für 3 Tage auf 90 °C erhitzt. Anschließend wurde abgekühlt und mit Dieselkraftstoff auf ein Volumen von 500 ml verdünnt. Daraufhin wurde das Gemisch durch einen im Verfahren beschriebenen Filter filtriert. Eine Filtrationszeit über 120 Sekunden wurde als durchgefallen bewertet.

| Zusatz im Test | Filtrationszeit, s | Bestanden/durchgefallen |
|---|---|---|
| 50% Lösung von Probe gemäß Herstellungsbeispiel 1 in Ethylhexanol | 105 | Bestanden |
| 50% Lösung von Probe gemäß Herstellungsbeispiel 3 in Propylheptanol | 120 | Bestanden |
| 50% Lösung von Muster gemäß Vergleichsbeispiel 1 | >300 | Nicht bestanden, Filter verstopft |

Die erfindungsgemäß beschriebenen Verbindungen verursachen keine Verschlechterung der Motorölverträglichkeit gemessen nach der DGMK 531 1-A Norm und führen nicht zu einer Verschlechterung der Motoröleigenschaften.

### Anwendungsbeispiel 6: Korrosionsschutztest nach ASTM D665B (modifiziert)

Der Test wurde durchgeführt gemäß Norm ASTM D665 B (modifiziert) mit Wasser (künstlichem Meerwasser) im Gemisch mit Diesel-Grundkraftstoff nach EN590 B7, ohne Leistungsadditive.

Die Modifizierungen bestanden darin, dass die Temperatur 60 °C und die Dauer des Tests 4 Stunden betrug.

Die Auswertung des Tests erfolgte nach der NACE-Bewertung. Sowohl Kraftstoffe mit als auch ohne Zusätze wurden untersucht. Die Ergebnisse sin in folgender Tabelle aufgeführt.

| | |
|---|---|
| A | 100% rostfrei |
| B++ | 0,1% oder weniger der gesamten Oberfläche verrostet |
| B+ | 0,1% - 5% der gesamten Oberfläche verrostet |
| B | 5% - 25% der gesamten Oberfläche verrostet |
| C | 25% - 50% der gesamten Oberfläche verrostet |
| D | 50% - 75% der gesamten Oberfläche verrostet |
| E | 75% - 100% der gesamten Oberfläche verrostet |

| Zusatz | Bewertung im ASTM D665B Test (mit künstlichem Meerwasser) |
|---|---|
| Keine Zusätze | E |
| 70 ppm Probe gemäß Herstellungsbeispiel 3 | A |
| 70 ppm Probe gemäß Herstellungsbeispiel 1 | A |
| 70 ppm Probe gemäß Herstellungsbeispiel 6 | A |
| 70 ppm Probe gemäß Herstellungsbeispiel 7 | A |

Die erfindungsgemäß beschriebenen Verbindungen zeigen eine sehr starke Korrosionsschutzwirkung, wie gezeigt durch die ASTM D 665 B (unter Verwendung von künstlichem Meerwasser).

### Anwendungsbeispiel 7: DW10 Zn Motortest (keep clean)

Der Test wurde mit einem Peugeot DW10 Motor durchgeführt, gemäß der standardmäßigen 44-stündigen CEC F-98-08 Prozedur. In den Tests eingesetzt wurde ein Grundkraftstoff nach EN590 B7, ohne Leistungsadditive. Sowohl Kraftstoffe mit als auch ohne Zusätze wurden untersucht. Die Ergebnisse sind in folgender Tabelle zusammengefasst.

| Zusätze | Leistungsverlust nach dem Test, % |
|---|---|
| 1 ppm Zn | 4,3 |
| 1 ppm Zn und 36 ppm Probe gemäß Herstellungsbeispiel 7 | 0 |

Die in dieser Erfindung beschriebenen Verbindungen wirken effektiv gegen die Bildung von Ablagerungen in direkt einspritzenden Motoren wie dem Peugeot DW10 wie verwendet in der Testprozedur gemäß CEC F-98-08 und sind in der Lage zuvor gebildete Ablagerungen zu entfernen.

### Anwendungsbeispiel 8: HFRR DIN ISO 12156-1 Schmierfähigkeitstest

Der Test wurde durchgeführt dem Standard DIN ISO 12156-1 Test zur Bestimmung der Schmierfähigkeit von Dieselkraftstoffen. Der Kraftstoff wurde mit und ohne Zusätze getestet. In der Messung wurde der Abrieb bestimmt. Je höher der Abrieb ist, desto schlechter sind die Schmierfähigkeitseigenschaften des Kraftstoffes. Es wurde Coryton B0 Kraftstoff mit geringer Schmierfähigkeit in diesem Test eingesetzt.

| Zusatz | HFRR Abrieb gemäß DIN ISO 12156-1 Test, µm |
|---|---|
| Kein Zusatz | 518 |
| 70 ppm Probe gemäß Herstellungsbeispiel 1 | 365 |

Die erfindungsgemäß beschriebenen Verbindungen können die Schmierfähigkeit von Dieselkraftstoffen verbessern und einer Fehlfunktion von Kraftstoffpumpen wie gemessen im DIN ISO 12156-1 Test vorbeugen.

### Anwendungsbeispiel 9: DW10 Na Seifen IDID test (clean up)

Zur Untersuchung des Einflusses der Additive auf die Performance von direkteinspritzenden Dieselmotoren wurde als weitere Testmethode der IDID-Motorentest, bei dem die Abgastemperaturen der Zylinder am Zylinderausgang beim Kaltstart des DW10-Motors bestimmt wurden. Verwendet wurde ein direkteinspritzender Dieselmotor mit Common-Rail-System des Herstellers Peugeot gemäß Testmethoden CEC F-098-08. Als Kraftstoff wurde ein handelsüblicher B7 Dieselkraftstoff gemäß EN 590 eingesetzt. Diesem wurden zur künstlichen Anregung der Bildung von Ablagerungen jeweils 1 mg/L Na in Form von Natriumnaphthenat sowie 20 mg/L Dodecenylbernsteinsäure zugesetzt.

Ähnlich wie das Verfahren CEC F-98 -08 wird die Motorleistung während des Testsgemessen.

Der Test bestand aus zwei Teilen:

### I. Dirty up:

Der Test wurde ohne Zusatz von Verbindungen gemäß dieser Erfindung durchgeführt. Der Test wurde auf 8 Stunden verkürzt, das CEC F-98 -08 Verfahren wurde ohne Zusatz von Zn wurde durchgeführt, aber mit Zusatz von Natriumnaphthenat und Dodecylbernsteinsäure (DDS). Wenn signifikante Abweichungen von Abgastemperaturen beobachtet wurden, wurde die Prüfung vor Erreichen der 8 Stunden-Marke angehalten, um Motorschäden zu vermeiden. Nach dem dirty up- Lauf, ließ man den Motor abkühlen und danach wurde erneut gestartet und im Leerlauf 5 Minuten betrieben. Während dieser 5 Minuten wurde der Motor aufgewärmt. Die Abgastemperatur von jedem Zylinder wurde aufgezeichnet. Je geringer die Unterschiede zwischen den ermittelten Abgas-Temperaturen sind, um so niedriger ist die Menge an gebildeten IDID.

Es wurden jeweils die Abgastemperaturen der 4 Zylinder ("Z1" bis "Z4") an den Zylinder-ausgängen nach 0 Minuten ("ϑ0") und nach 5 Minuten ("ϑ5")gemessen. Die Ergebnisse der Abgastemperatur-Messungen mit Durchschnittswerten ("Δ") und den größten Abweichungen von Δ nach unten ("-") und oben ("+") für die beiden Testläufe sind in der folgenden Übersicht zusammengefasst.

### II. Clean up:

Der Test wurde auf 8 Stunden verkürzt, das CEC F-98 -08 Verfahren wurde ohne Zusatz von Zn wurde durchgeführt. Es wurden jedoch jeweils 1 mg/L Na in Form von Natriumnaphthenat sowie 20 mg/L Dodecenylbernsteinsäure sowie eine erfindungsgemäße Verbindung zugesetzt, und die Motorenleistung bestimmt.

Nach dem clean up wurde der Motor abgekühlt und erneut gestartet. Die Abgastemperatur von jedem Zylinder wurde aufgezeichnet. Je geringer die Unterschiede zwischen den ermittelten Abgas-Temperaturen sind, um so niedriger ist die Menge an gebildeten IDID.

Es wurden jeweils die Abgastemperaturen der 4 Zylinder ("Z1" bis "Z4") an den Zylinder-ausgängen nach 0 Minuten ("ϑ0") und nach 5 Minuten ("ϑ5")gemessen. Die Ergebnisse der Abgastemperatur-Messungen mit Durchschnittswerten ("Δ") und den größten Abweichungen von Δ nach unten ("-") und oben ("+") sind in der folgenden Übersicht zusammengefasst.

### Dirty up - Clean up - Sequenz 1:

### Dirty up

Signifikante Abweichungen in Abgastemperaturen wurden während des Tests beobachtet, so dass dieser nach 3 Stunden angehalten wurde, um Motorschäden zu vermeiden.
Nach dirty up:

| | | | | | |
|---|---|---|---|---|---|
| ϑ0 | Z1: 40°C | Z2: 35°C | Z3: 32°C | Z4: 48°C | |
| ϑ5 | Z1: 117°C | Z2: 45°C | Z3: 47°C | Z4: 109°C | Δ: 79,5°C |
| (+37,5°C / -32,5°C) | | | | | |

Wesentliche Abweichungen von dem Mittelwert und signifikante Unterschiede zwischen den einzelnen Zylindern belegen die Präsenz von IDID.
Clean up

Nach clean up mit 150 ppm Probe gemäß Herstellungseispiel 1 in Gegenwart von 1 mg/L Na + 20 mg/L Dodecenylbernsteinsäure:

| | | | | |
|---|---|---|---|---|
| ϑ0 Z1: 42°C | Z2: 42°C | Z3: 29°C | Z4: 34°C | |
| ϑ5 Z1: 85°C | Z2: 86°C | Z3: 57°C | Z4: 53°C | Δ: 70,3°C (-17,3°C / +15,7°C) |

Die Abweichung vom Mittelwert der Temperatur der Abgase ist niedrig, was für die Entfernung von IDID spricht.

Die erfindungsgemäß beschriebenen Verbindungen sind sehr effektiv gegen die Bildung von IDID in direkteinspritzenden Motoren, wie man am Beispiel des Peugeot DW10 sehen kann, der in dem Test ähnlich der CEC F-98-08 Prozedur eingesetzt wird, jedoch in Gegenwart von 1 mg/L Na in Form von Natriumnaphthenat sowie 20 mg/L Dodecenylbernsteinsäure.

### Anwendungsbeispiel 10: DW10 Na power loss Test (clean up)

Zur Untersuchung der Wirksamkeit der erfindungsgemäßen Verbindungen gegen power loss, verursacht durch Metalle, wie Na, K, Ca und andere (und nicht durch Zn wie oben beschrieben), wurde ein IDID-Motorentest wie oben beschrieben eingesetzt. Während des dirty up- und clean up-Laufs wird die Leistung nach CEC F-098-08 gemessen, mit einem verkürzten clean up-Zeitraum, wie oben beschrieben.

Der Leistungsverlust im DU wird wie folgt berechnet $Powerloss , du \left[%\right] = \left(1-\frac{Pend , du}{P 0 , du}\right) ∗ 100$

Der Leistungsverlust im CU-Test wird wie folgt berechnet (negative Zahl beim power-loss im CU-Test bedeutet Leistungszuwachs) $Powerloss \left(DU, CU\right) \left[%\right] = \left(\frac{Pend , du - pend , cu}{P 0 , du}\right) ∗ 100$

| Test | Zusatz | Leistung vor Test, kW | Leistung nach Test, kW | Leistungsverlust, % |
|---|---|---|---|---|
| Dirty up, 8 Stunden, Verfahren wie oben beschrieben | 1 mg/L Na + 20 mg/L Dodecenylbernsteinsäure | 97,6 | 92,3 | 5,4 |
| Clean up, 8 Stunden, Verfahren wie oben beschrieben | 1 mg/L Na + 20 mg/L Dodecenylbernsteinsäure and 150 ppm Probe gemäß Herstellungsbeispiel 1 | 91,6 | 93,0 | -0,7 |

Die in dieser Erfindung beschriebenen Verbindungen sind effektiv gegen die Bildung von Ablagerungen, die von anderen Metallen als Zn, wie Na, K, Ca verursacht werden, wie am obigen Na power loss Test gezeigt wurde.

### Anwendungsbeispiel 11: Injektorsauberkeit (Direkteinspritzender Otto-Motor; DISI)

a) In den folgenden Anwendungstests eingesetzte Produkte:

| | |
|---|---|
| Test 1: | kein Additiv (Basisdurchlauf) |
| Test 2: | C16-Dimethylamin+PIB-Bernsteinsäure+PO (Herstellungseispiel 7), 25 mg/kg aktiver Gehalt |
| Test 3: | Tridecyldimethylamin+Dodecenylbernsteinsäure+PO (Herstellungseispiel 15), 25 mg/kg aktiver Gehalt |
| Test 4: | Dimethylethanolamin/15PO+Dodecenylbernsteinsäure (Herstellungseispiel 16), 25 mg/kg aktiver Gehalt |

In allen Tests wurde europäischer RON 92 E0 Ottokraftstoff verwendet.
b) Die Tests wurden gemäß folgendem Verfahren, ursprünglich in der US2013225463 beschrieben, durchgeführt.
Methode: BASF Hausmethode
Motor: Aufgeladener Vierzylinder mit 1,6 Liter Hubraum
Testdauer: 60 Stunden

### Testergebnisse:

| Test | Änderung²⁾ des FR-Wertes¹⁾ | Aussehen des Injektors |
|---|---|---|
| Test 1 (Basisdurchlauf) | + 4,54% | Fig. 2A |
| Test 2 | - 2,66% | Fig. 2B |
| Test 3 | - 1,90% | Fig. 2C |
| Test 4 | - 1,99% | Fig. 2D |

| | | |
|---|---|---|
| ¹⁾: Der FR-Wert ist ein durch die Motorsteuerung erfasster Parameter, der mit der Dauer des Einspritzvorgangs des Kraftstoffes in den Brennraum korreliert. Je ausgeprägter die Bildung von Ablagerungen in den Injektordüsen, desto länger ist die Einspritzdauer bzw. höher ist der FR-Wert. Umgekehrt bleibt der FR-Wert konstant bzw. nimmt tendenziell leicht ab, wenn die Injektordüsen frei von Ablagerungen bleiben. ²⁾: Änderung des FR-Wertes in % im Vergleich zum FR-Wert zu Beginn des Tests (je größer die positiven Werte, desto mehr Ablagerungen werden im Injektor gebildet und desto größer ist die Kontamination des Injektors) | | |

Die gezeigten Ergebnisse legen dar, dass die Produkte, die oben in den erfindungsgemäßen Beispielen beschrieben sind, dafür geeignet sind, die Bildung von Ablagerungen in Injektoren direkteinspritzender Otto-Motoren zu verhindern und zuvor gebildete Ablagerungen zu entferne

## Patentansprüche

1. Verwendung einer quaternisierten Stickstoffverbindung, erhältlich durch Quaternisierung einer quaternisierbaren Stickstoffverbindung, enthaltend wenigstens eine quaternisierbare, insbesondere tertiäre Aminogruppe mit einem Quaternierungsmittel, das die wenigstens eine quaternisierbare, insbesondere tertiäre Aminogruppe in eine quaternäre Ammoniumgruppe überführt, wobei das Quaternierungsmittel das ein Hydrocarbylepoxid in Kombination mit einer freien hydrocarbyl-substituierten Polycarbonsäure ist;
als Detergensadditiv in Kraftstoffen; oder
als Dieselkraftstoffadditiv zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen; oder
als Ottokraftstoffadditiv zur Verringerung von Ablagerungen im Einlasssystem eines Ottomotors, wie insbesondere DISI und PFI (Port Fuel Injector) -Motoren; oder
als Dieselkraftstoffadditiv zur Verringerung und/oder Vermeidung von Ablagerungen in den Einspritzsystemen, bevorzugt bestimmt in einem XUD 9 Test, nach CEC-F-23-1-01 oder in einem DW10 Test in Anlehnung an CEC F-98-08, wie insbesondere der Internal Diesel Injector Deposits (IDID) und / oder von Ventilkleben in direkteinspritzenden Dieselmotoren, insbesondere in Common-Rail-Einspritzsystemen.

2. Verwendung nach Anspruch 1, wobei die quaternisierbare Stickstoffverbindung ausgewählt ist unter:
a) wenigstens einem Alkylamin der folgenden allgemeinen Formel 3
RₐR_{b}R_{c}N (3)
worin
wenigstens einer der Reste Rₐ, R_{b} und R_{c} für einen geradkettigen oder verzweigten, gesättigten oder ungesättigten C₈-C₄₀-Hydrocarbylrest, insbesondere geradkettigen oder verzweigten C₈ - C₄₀-Alkylrest, steht und die übrigen Reste für gleiche oder verschiedene, geradkettige oder verzweigte, gesättigte oder ungesättigten C₁-C₆-Hydrocarbylreste, insbesondere C₁-C₆-Alkylreste, stehen; oder
worin alle Reste Rₐ, R_{b} und R_{c} für gleiche oder verschiedene geradkettige oder verzweigte, gesättigte oder ungesättigte C₈-C₄₀-Hydrocarbylreste, insbesondere geradkettige oder verzweigte C₈ - C₄₀-Alkyl-Reste stehen; oder
worin die Reste Rₐ, R_{b} und R_{c} gleiche oder verschiedene kurzkettige Alkylreste, insbesondere geradkettige oder verzweigte Alkylgruppen mit 1 bis 7 oder insbesondere 1 bis 4 Kohlenstoffatomen aufweisen;
b) wenigstens einem Polyalken-substituierten Amin, enthaltend wenigstens eine quaternisierbare, insbesondere tertiäre, Aminogruppe;
c) wenigstens einem Polyether-substituierten Amin, enthaltend wenigstens eine quaternisierbare, insbesondere tertiäre, Aminogruppe; und
d) wenigstens einem Reaktionsprodukt eines hydrocarbylsubstituierten Acylierungsmittels und einer Verbindung, enthaltend ein Stickstoff- oder Sauerstoffatom und zusätzlich enthaltend wenigstens eine quaternisierbare Aminogruppe; und
e) Mischungen davon.

3. Verwendung nach einem der Ansprüche 1 und 2, wobei das Quaternierungsmittel ein Epoxid der allgemeinen Formel 4 umfasst 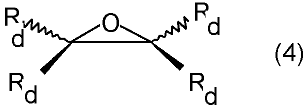 wobei
die darin enthaltenen Reste R_{d} gleich oder verschieden sind und für H oder für einen Hydrocarbylrest stehen, wobei der Hydrocarbylrest für einen aliphatischen oder aromatischen Rest mit 1 bis 10 Kohlenstoffatomen steht

4. Verwendung nach einem der vorhergehenden Ansprüche, wobei die freie Säure des Quaternierungsmittels eine hydrocarbyl-substituierte C₃-C₂₈- Dicarbonsäure ist.

5. Verwendung nach einem der vorhergehenden Ansprüche, wobei der Hydrocarbyl-Substituent der Carbonsäure ein zahlengemitteltes Molekulargewicht (Mₙ) von 85 bis 20.000 aufweist; oder ein Polyalkylenrest mit einem Polymerisationsgrad von 2 bis 100 oder 3 bis 50 oder 4 bis 25 ist.

6. Verwendung nach einem der vorhergehenden Ansprüche, wobei das quaternisierbare tertiäre Amin eine Verbindung der Formel 3 ist, worin wenigstens zwei der Reste Rₐ, R_{b} und R_{c} gleich oder verschieden sind und für einen geradkettigen oder verzweigten C₁₀-C₂₀-Alkylrest stehen und der übrige Rest für C₁-C₄-Alkyl steht.

7. Verwendung nach einem der vorhergehenden Ansprüche, wobei das Quaternierungsmittel ausgewählt ist unter Hydrocarbylepoxiden der obigen allgemeinen Formel 4 in Kombination mit einer Hydrocarbyl-substituierten C₃-C₂₈-Dicarbonsäure.

8. Verwendung nach einem der vorhergehenden Ansprüche, wobei die Carbonsäure ausgewählt ist unter Hydrocarbyl-substituierter Malon-, Bernstein-, Glutar-, Adipin- und Pimelinsäure.

9. Verwendung nach einem der Ansprüche 1 bis 8, wobei das quaternisierbare Amin ausgewählt ist aus der Gruppe bestehend aus Hydroxyalkyl-substituierten primären, sekundären, und insbesondere tertiären Monoaminen und Hydroxyalkyl-substituierten primären, sekundären und insbesondere tertiären Diaminen.

10. Verwendung nach einem der Ansprüche 1 bis 8, wobei es sich bei dem quaternisierbaren Amin um ein hydroxyalkyl-substituiertes Monoamin handelt, ausgewählt ist aus der Gruppe bestehend aus N-Hydroxyalkyl-monoaminen, N,N-Dihydroxyalkyl-monoaminen und N,N,N-Trihydroxyalkyl-monoaminen.

11. Verwendung nach einem der Ansprüche 1 bis 8, wobei das quaternisierbare Amin ausgewählt ist aus der Gruppe bestehend aus Ethanolamin, 3-Hydroxy-1-propylamin, Diethanolamin, Diisopropanolamin, N-(2-Hydroxyethyl)ethylendiamin, Triethanolamin, 1-(3-Hydroxypropyl)imidazol, Tris(hydroxymethyl)amin, 3-Dimethylamino-1-propanol, 3-Diethylamino-1-propanol, 2-Dimethylamino-1-ethanol und 4-Diethylamino-1-butanol.

12. Verwendung nach einem der vorhergehenden Ansprüche, wobei der Kraftstoff ausgewählt unter Dieselkraftstoffen, Biodieselkraftstoffen, Ottokraftstoffen, und Alkanol-haltigen Ottokraftstoffen.

13. Verwendung nach einem der vorhergehenden Ansprüche, wobei es sich bei dem Kraftstoff um Diesel- oder eine Biodiesel-Kraftstoff-Zusammensetzung handelt, umfassend Biobrennstofföl, in Mengen von 1 bis 30 Gew.-%, insbesondere von 3 bis 10 Gew.-%, bezogen auf die Gesamtmenge aus Mitteldestillat fossilen, pflanzlichem oder tierischen Ursprungs und Biobrennstofföl; insbesondere basierend auf Alkylestern von Fettsäuren; vor allem Niedrigalkylestern, insbesondere C₁- bis C₄-Alkylestern, die durch Umesterung der in pflanzlichen und/oder tierischen Ölen und/oder Fetten vorkommenden Glyceride, insbesondere Triglyceride, mittels Niedrigalkoholen, erhältlich sind.

14. Verwendung nach Anspruch 13, wobei der Alkylester von Fettsäure ausgewählt ist unter Sonnenblumenmethylester, Palmölmethylester ("PME"), Sojaölmethylester ("SME"), hydrierte Pflanzenöle ("HVO"), used kitchen oil ("UKO") und insbesondere Rapsölmethylester ("RME").

## Claims

1. Use of a quaternized nitrogen compound, obtainable by quaternization of a quaternizable nitrogen compound containing at least one quaternizable, in particular tertiary, amino group with a quaternizing agent that converts the at least one quaternizable, in particular tertiary, amino group into a quaternary ammonium group, wherein the quaternizing agent is a hydrocarbyl epoxide in combination with a free hydrocarbyl-substituted polycarboxylic acid;
as a detergent additive in fuels; or
as a diesel fuel additive for reducing fuel consumption of direct-injection diesel engines, in particular diesel engines with common rail injection systems, and/or for minimizing power loss in direct-injection diesel engines, in particular in diesel engines with common rail injection systems; or
as a gasoline fuel additive for reducing deposits in the intake system of a gasoline engine, such as in particular DISI and PFI (Port Fuel Injector) engines; or
as a diesel fuel additive for reducing and/or preventing deposits in the injection systems, preferably determined in an XUD 9 test according to CEC-F-23-1-01 or in a DW10 test based on CEC F-98-08,
such as in particular Internal Diesel Injector Deposits (IDID) and/or valve sticking in direct-injection diesel engines, in particular in common rail injection systems.

2. Use according to claim 1, wherein the quaternizable nitrogen compound is selected from:
a) at least one alkylamine of the following general formula 3
RₐR_{b}R_{c}N (3)
wherein
at least one of the radicals Rₐ₁, R_{b} and R_{c} represents a straight-chain or branched, saturated or unsaturated C₈-C₄₀ hydrocarbyl radical, in particular a straight-chain or branched C₈-C₄₀ alkyl radical, and the remaining radicals represent identical or different, straight-chain or branched, saturated or unsaturated C₁-C₆ hydrocarbyl radicals, in particular C₁-C₆ alkyl radicals; or
wherein all radicals Rₐ₁, R_{b} and R_{c} represent identical or different straight-chain or branched, saturated or unsaturated C₈-C₄₀ hydrocarbyl radicals, in particular straight-chain or branched C₈-C₄₀ alkyl radicals; or
wherein the radicals Rₐ₁, R_{b} and R_{c} have identical or different short-chain alkyl radicals, in particular straight-chain or branched alkyl groups having 1 to 7 or in particular 1 to 4 carbon atoms;
b) at least one polyalkene-substituted amine containing at least one quaternizable, in particular tertiary, amino group;
c) at least one polyether-substituted amine containing at least one quaternizable, in particular tertiary, amino group; and
d) at least one reaction product of a hydrocarbyl-substituted acylating agent and a compound containing a nitrogen or oxygen atom and additionally containing at least one quaternizable amino group; and
e) mixtures thereof.

3. Use according to any one of claims 1 and 2, wherein the quaternizing agent comprises an epoxide of general formula 4 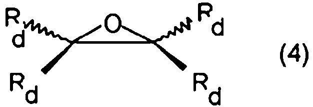 wherein
the radicals R_{d} contained therein are identical or different and represent H or a hydrocarbyl radical, wherein the hydrocarbyl radical represents an aliphatic or aromatic radical having 1 to 10 carbon atoms

4. Use according to any one of the preceding claims, wherein the free acid of the quaternizing agent is a hydrocarbyl-substituted C₃-C₂₈- dicarboxylic acid.

5. Use according to any one of the preceding claims, wherein the hydrocarbyl substituent of the carboxylic acid has a number-average molecular weight (Mₙ) of 85 to 20,000; or is a polyalkylene radical having a degree of polymerization of 2 to 100 or 3 to 50 or 4 to 25.

6. Use according to any one of the preceding claims, wherein the quaternizable tertiary amine is a compound of formula 3, wherein at least two of the radicals Rₐ₁, R_{b} and R_{c} are identical or different and represent a straight-chain or branched C₁₀-C₂₀ alkyl radical and the remaining radical represents C₁-C₄ alkyl.

7. Use according to any one of the preceding claims, wherein the quaternizing agent is selected from hydrocarbyl epoxides of the above general formula 4 in combination with a hydrocarbyl-substituted C₃-C₂₈- dicarboxylic acid.

8. Use according to any one of the preceding claims, wherein the carboxylic acid is selected from hydrocarbyl-substituted maleic, succinic, glutaric, adipic and pimelic acid.

9. Use according to any one of claims 1 to 8, wherein the quaternizable amine is selected from the group consisting of hydroxyalkyl-substituted primary, secondary, and in particular tertiary monoamines and hydroxyalkyl-substituted primary, secondary and in particular tertiary diamines.

10. Use according to any one of claims 1 to 8, wherein the quaternizable amine is a hydroxyalkyl-substituted monoamine selected from the group consisting of N-hydroxyalkyl monoamines, N,N-dihydroxyalkyl monoamines and N,N,N-trihydroxyalkyl monoamines.

11. Use according to any one of claims 1 to 8, wherein the quaternizable amine is selected from the group consisting of ethanolamine, 3-hydroxy-1-propylamine, diethanolamine, diisopropanolamine, N-(2-hydroxyethyl)ethylenediamine, triethanolamine, 1-(3-hydroxypropyl)imidazole, tris(hydroxymethyl)amine, 3-dimethylamino-1-propanol, 3-diethylamino-1-propanol, 2-dimethylamino-1-ethanol and 4-diethylamino-1-butanol.

12. Use according to any one of the preceding claims, wherein the fuel is selected from diesel fuels, biodiesel fuels, gasoline fuels, and alkanol-containing gasoline fuels.

13. Use according to any one of the preceding claims, wherein the fuel is a diesel or biodiesel fuel composition comprising biofuel oil in amounts of 1 to 30% by weight, in particular 3 to 10% by weight, based on the total amount of middle distillate of fossil, vegetable or animal origin and biofuel oil; in particular based on alkyl esters of fatty acids; especially low alkyl esters, in particular C₁- to C₄-alkyl esters, obtainable by transesterification of glycerides, in particular triglycerides, present in vegetable and/or animal oils and/or fats by means of low alcohols.

14. Use according to claim 13, wherein the alkyl ester of fatty acid is selected from sunflower methyl ester, palm oil methyl ester ("PME"), soybean oil methyl ester ("SME"), hydrogenated vegetable oils ("HVO"), used kitchen oil ("UKO") and in particular rapeseed oil methyl ester ("RME").

## Revendications

1. Utilisation d'un composé azoté quaternisé, pouvant être obtenu par quaternisation d'un composé azoté quaternisable, contenant au moins un groupe amino quaternisable, en particulier tertiaire, avec un agent de quaternisation qui convertit le ou les groupes amino quaternisables, en particulier tertiaires, en un groupe ammonium quaternaire, l'agent de quaternisation étant un hydrocarbylepoxyde en combinaison avec un acide polycarboxylique libre substitué par un hydrocarbyle ;
en tant qu'additif détergent dans les carburants ; ou
en tant qu'additif pour carburant diesel afin de réduire la consommation de carburant des moteurs diesel à injection directe, en particulier des moteurs diesel équipés de systèmes d'injection Common Rail, et/ou afin de minimiser la perte de puissance (powerloss) dans les moteurs diesel à injection directe, en particulier dans les moteurs diesel équipés de systèmes d'injection Common Rail ; ou
en tant qu'additif pour carburant essence afin de réduire les dépôts dans le système d'admission d'un moteur à essence, tels que notamment les moteurs DISI et PFI (Port Fuel Injector) ; ou
en tant qu'additif pour carburant diesel afin de réduire et/ou d'éviter les dépôts dans les systèmes d'injection, de préférence déterminés dans un test XUD 9, selon CEC-F-23-1-01 ou dans un test DW10 basé sur CEC F-98-08,
tels que notamment les dépôts internes d'injecteurs diesel (Internal Diesel Injector Deposits, IDID) et/ou le collage de soupapes dans les moteurs diesel à injection directe, en particulier dans les systèmes d'injection Common Rail,
systèmes d'injection.

2. Utilisation selon la revendication 1, dans laquelle le composé azoté quaternisable est choisi parmi :
a) au moins une alkylamine de la formule générale suivante 3
RₐR_{b}R_{c}N (3)
dans laquelle
au moins l'un des radicaux Rₐ₁, R_{b} et R_{c} représente un radical hydrocarbyle en C₈-C₄₀ à chaîne droite ou ramifiée, saturé ou insaturé, en particulier un radical alkyle en C₈-C₄₀ à chaîne droite ou ramifiée, et les autres radicaux représentent des radicaux hydrocarbyle en C₁-C₆ identiques ou différents, à chaîne droite ou ramifiée, saturés ou insaturés, en particulier des radicaux alkyle en C₁-C₆ ; ou
dans laquelle tous les radicaux Rₐ₁, R_{b} et R_{c} représentent des radicaux hydrocarbyle en C₈-C₄₀ identiques ou différents, à chaîne droite ou ramifiée, saturés ou insaturés, en particulier des radicaux alkyle en C₈-C₄₀ à chaîne droite ou ramifiée ; ou
dans laquelle les radicaux Rₐ₁, R_{b} et R_{c} présentent des radicaux alkyle à chaîne courte identiques ou différents, en particulier des groupes alkyle à chaîne droite ou ramifiée ayant 1 à 7 ou en particulier 1 à 4 atomes de carbone ;
b) au moins une amine substituée par un polyalcène, contenant au moins un groupe amino quaternisable, en particulier tertiaire ;
c) au moins une amine substituée par un polyéther, contenant au moins un groupe amino quaternisable, en particulier tertiaire ; et
d) au moins un produit de réaction d'un agent d'acylation substitué par un hydrocarbyle et d'un composé contenant un atome d'azote ou d'oxygène et contenant en outre au moins un groupe amino quaternisable ; et
e) des mélanges de ceux-ci.

3. Utilisation selon l'une des revendications 1 et 2, dans laquelle l'agent de quaternisation comprend un époxyde de la formule générale 4 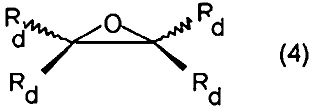 dans laquelle
les radicaux R_{d} qu'il contient sont identiques ou différents et représentent H ou un radical hydrocarbyle, le radical hydrocarbyle représentant un radical aliphatique ou aromatique ayant 1 à 10 atomes de carbone

4. Utilisation selon l'une des revendications précédentes, dans laquelle l'acide libre de l'agent de quaternisation est un acide dicarboxylique en C₃-C₂₈- substitué par un hydrocarbyle.

5. Utilisation selon l'une des revendications précédentes, dans laquelle le substituant hydrocarbyle de l'acide carboxylique présente une masse moléculaire moyenne en nombre (Mₙ) de 85 à 20 000 ; ou est un radical polyalkylène ayant un degré de polymérisation de 2 à 100 ou de 3 à 50 ou de 4 à 25.

6. Utilisation selon l'une des revendications précédentes, dans laquelle l'amine tertiaire quaternisable est un composé de la formule 3, dans laquelle au moins deux des radicaux Rₐ₁, R_{b} et R_{c} sont identiques ou différents et représentent un radical alkyle en C₁₀-C₂₀ à chaîne droite ou ramifiée, et le radical restant représente un alkyle en C₁-C₄.

7. Utilisation selon l'une des revendications précédentes, dans laquelle l'agent de quaternisation est choisi parmi les hydrocarbylépoxydes de la formule générale 4 ci-dessus en combinaison avec un acide dicarboxylique en C₃-C₂₈- substitué par un hydrocarbyle.

8. Utilisation selon l'une des revendications précédentes, dans laquelle l'acide carboxylique est choisi parmi les acides maléique, succinique, glutarique, adipique et pimélique substitués par un hydrocarbyle.

9. Utilisation selon l'une des revendications 1 à 8, dans laquelle l'amine quaternisable est choisie dans le groupe constitué par les monoamines primaires, secondaires et en particulier tertiaires substituées par un hydroxyalkyle et les diamines primaires, secondaires et en particulier tertiaires substituées par un hydroxyalkyle.

10. Utilisation selon l'une des revendications 1 à 8, dans laquelle l'amine quaternisable est une monoamine substituée par un hydroxyalkyle, choisie dans le groupe constitué par les N-hydroxyalkyl-monoamines, les N,N-dihydroxyalkyl-monoamines et les N,N,N-trihydroxyalkyl-monoamines.

11. Utilisation selon l'une des revendications 1 à 8, dans laquelle l'amine quaternisable est choisie dans le groupe constitué par l'éthanolamine, la 3-hydroxy-1-propylamine, la diéthanolamine, la diisopropanolamine, la N-(2-hydroxyéthyl)éthylènediamine, la triéthanolamine, le 1-(3-hydroxypropyl)imidazole, le tris(hydroxyméthyl)amine, la 3-diméthylamino-1-propanol, la 3-diéthylamino-1-propanol, la 2-diméthylamino-1-éthanol et la 4-diéthylamino-1-butanol.

12. Utilisation selon l'une des revendications précédentes, dans laquelle le carburant est choisi parmi les carburants diesel, les biocarburants diesel, les carburants essence et les carburants essence contenant de l'alcanol.

13. Utilisation selon l'une des revendications précédentes, dans laquelle le carburant est une composition de carburant diesel ou biodiesel comprenant une huile de biocarburant, en quantités de 1 à 30 % en poids, en particulier de 3 à 10 % en poids, par rapport à la quantité totale de distillat moyen d'origine fossile, végétale ou animale et d'huile de biocarburant ; basée en particulier sur des alkylesters d'acides gras ; notamment des alkylesters à chaîne courte, en particulier des alkylesters en C₁ à C₄, pouvant être obtenus par transestérification des glycérides, en particulier des triglycérides, présents dans les huiles et/ou graisses végétales et/ou animales, au moyen d'alcools à chaîne courte.

14. Utilisation selon la revendication 13, dans laquelle l'alkylester d'acide gras est choisi parmi l'ester méthylique de tournesol, l'ester méthylique d'huile de palme (« PME »), l'ester méthylique d'huile de soja (« SME »), les huiles végétales hydrogénées (« HVO »), l'huile de cuisine usagée (« UKO ») et en particulier l'ester méthylique d'huile de colza (« RME »).
